(19) **Europäisches Patentamt / European Patent Office / Office européen des brevets**

(11) **EP 3 638 283 B1**

(12) # EUROPEAN PATENT SPECIFICATION

(45) Date of publication and mention
of the grant of the patent:
**17.07.2024 Bulletin 2024/29**

(21) Application number: **17737488.1**

(22) Date of filing: **14.06.2017**

(51) International Patent Classification (IPC):
**A61K 38/16** $^{(2006.01)}$ **A61K 45/06** $^{(2006.01)}$
**A61P 25/28** $^{(2006.01)}$ **A61K 31/137** $^{(2006.01)}$

(52) Cooperative Patent Classification (CPC):
(C-Sets available)
**A61K 31/137; A61P 25/28; G01N 33/6872;**
G01N 2500/00; G01N 2800/2814; G01N 2800/2835;
G01N 2800/285 (Cont.)

(86) International application number:
**PCT/EP2017/064644**

(87) International publication number:
**WO 2018/228692 (20.12.2018 Gazette 2018/51)**

(54) **ANTAGONISTS OF CAV2.3 R-TYPE CALCIUM CHANNELS FOR THE TREATMENT OF NEURODEGENERATIVE DISEASES**

ANTAGONISTEN VON CALCIUMKANÄLEN VOM TYP CAV2.3 R ZUR BEHANDLUNG VON NEURODEGENERATIVEN ERKRANKUNGEN

ANTAGONISTES DE CANAUX CALCIQUES DE TYPE CAV2.3 R POUR LE TRAITEMENT DE MALADIES NEURODÉGÉNÉRATIVES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**22.04.2020 Bulletin 2020/17**

(73) Proprietor: **Lario Therapeutics Ltd.**
**Edinburgh EH2 4JY (GB)**

(72) Inventor: **LISS, Birgit**
**89081 Ulm (DE)**

(74) Representative: **HGF**
**HGF Limited**
**1 City Walk**
**Leeds LS11 9DX (GB)**

(56) References cited:
**WO-A1-94/15607        US-A1- 2012 321 594**
**US-A1- 2014 038 927**

- **KOMEILI GHOLAMREZA ET AL: "Effects of Isradipine on Induced-Parkinson's Disease in Rats", ZAHEDAN JOURNAL OF RESEARCH IN MEDICAL SCIENCES, vol. In Press, no. In Press, 26 April 2016 (2016-04-26), XP055818555, DOI: 10.17795/zjrms-6444**
- **ARIAS R L ET AL: "Amiloride is neuroprotective in an MPTP model of Parkinson's disease", NEUROBIOLOGY OF DISEASE, ELSEVIER, AMSTERDAM, NL, vol. 31, no. 3, 1 September 2008 (2008-09-01), pages 334 - 341, XP023905928, ISSN: 0969-9961, [retrieved on 20080707], DOI: 10.1016/J.NBD.2008.05.008**
- **BENKERT JULIA ET AL: "Abstract", NATURE COMMUNICATIONS, vol. 10, no. 1, 1 December 2019 (2019-12-01), XP055819358, Retrieved from the Internet <URL:https://www.nature.com/articles/s41467-019-12834-x.pdf> DOI: 10.1038/s41467-019-12834-x**

**(Cont. next page)**

- GOMEZ-MANCILLA BALTAZAR ET AL: "Effect of ethosuximide on rest tremor in the MPTP monkey model : ETHOSUXIMIDE AND REST TREMOR", MOVEMENT DISORDERS, vol. 7, no. 2, 1 January 1992 (1992-01-01), US, pages 137 - 141, XP055818897, ISSN: 0885-3185, DOI: 10.1002/mds.870070207
- SCHNEIDER TONI ET AL: "How "Pharmacoresistant" is Cav2.3, the Major Component of Voltage-Gated R-type Ca2+ Channels?", PHARMACEUTICALS, vol. 6, no. 6, 1 January 2013 (2013-01-01), pages 759 - 776, XP055805750, Retrieved from the Internet <URL:https://www.ncbi.nlm.nih.gov/pmc/articles/PMC3816731/pdf/pharmaceuticals-06-00759.pdf> DOI: 10.3390/ph6060759
- HAINSWORTH ATTICUS H. ET AL: "Actions of sipatrigine, 202W92 and lamotrigine on R-type and T-type Ca2+ channel currents", EUROPEAN JOURNAL OF PHARMACOLOGY, vol. 467, no. 1-3, 1 April 2003 (2003-04-01), NL, pages 77 - 80, XP055818901, ISSN: 0014-2999, DOI: 10.1016/S0014-2999(03)01625-X
- JONES-HUMBLE S A ET AL: "The novel anticonvulsant lamotrigine prevents dopamine depletion in C57 black mice in the MPTP animal model of Parkinson's disease", LIFE SCIENCE, PERGAMON PRESS, OXFORD, GB, vol. 54, no. 4, 1 January 1994 (1994-01-01), pages 245 - 252, XP023748453, ISSN: 0024-3205, [retrieved on 19940101], DOI: 10.1016/0024-3205(94)00813-2
- RODOLPHE HAJJ ET AL: "Combination of acamprosate and baclofen as a promising therapeutic approach for Parkinson's disease", SCIENTIFIC REPORTS, vol. 5, no. 1, 6 November 2015 (2015-11-06), XP055445739, DOI: 10.1038/srep16084
- GÉZA BERECKI ET AL: "Differential Ca v 2.1 and Ca v 2.3 channel inhibition by baclofen and [alpha]-conotoxin Vc1.1 via GABA B receptor activation", JOURNAL OF GENERAL PHYSIOLOGY, vol. 143, no. 4, 31 March 2014 (2014-03-31), NEW YORK, US, pages 465 - 479, XP055445926, ISSN: 0022-1295, DOI: 10.1085/jgp.201311104
- M. J. CANO-CEBRIÁN ET AL: "Local acamprosate modulates dopamine release in the rat nucleus accumbens through NMDA receptors: an in vivo microdialysis study", NAUNYN-SCHMIEDEBERG'S ARCHIVES OF PHARMACOLOGY, vol. 367, no. 2, 1 February 2003 (2003-02-01), DE, pages 119 - 125, XP055445779, ISSN: 0028-1298, DOI: 10.1007/s00210-002-0674-3

(52) Cooperative Patent Classification (CPC): (Cont.)

C-Sets
A61K 31/137, A61K 2300/00

## Description

### Field of the Invention

The invention relates to Cav2.3 R-type calcium channel antagonists for use in the treatment of Parkinson's disease (PD) as defined in present claim 1.

### Background of the Invention

Parkinson's disease (PD) is the second most common neurodegenerative disease after Alzheimer's disease. In Germany alone, approximately 350,000 people are affected. The main motor-symptoms are disturbed voluntary movement control, accompanied by muscle stiffness and a resting tremor. In the western world, the number of PD patients is increasing for years. This increase is not only due to an increased life expectancy. For unexplained reasons the age when motor symptoms manifest is decreasing and therefore, more and more younger people are suffering from PD. The cause for this increase in PD cases is still as incompletely understood as is the cause of the disease. However, while the cause for most PD cases is still unclear, a variety of genetic (PARK genes) and environmental factors (like mitochondrial dysfunction), leading to metabolic stress have been identified as PD stressors.

It should be emphasized that metabolic stress seems to constitute an essential common converging downstream factor for most - if not all - neurodegenerative diseases. However increasing age is the major risk factor for PD - as well as for most other neurodegenerative diseases (e.g. Alzheimer's disease, the most common neurodegenerative disease).

PD motor-symptoms are caused by a progressive loss of dopamine in the dorsal striatum, due to a respective progressive death of the dopaminergic (DA) midbrain neurons, particularly in the *Substantia nigra* (SN). If this death could be stopped or at least reduced after initial diagnosis, the disease process and thus progression of motor-symptoms can be stopped or slowed down. The ideal cell type-specific PD therapy should intervene early in a neuroprotective fashion, preferably before the classical PD motor symptoms develop, since usually 70-80% of the SN DA neurons are already lost when the typical PD motor-symptoms manifest. It is noteworthy that besides SN DA neurons a variety of other neurons are affected by degeneration in course of PD, while other neighboring DA midbrain neurons (like those in the ventral tegmental area, VTA) for unclear reason or much more resistant to PD stressor and neurodegenerative processes.

This so-called differential vulnerability of neuronal subpopulations is not only present in PD but in all neurodegenerative diseases. While its underlying causes are still unclear, intrinsic high levels of metabolic stress, in particular calcium ion related metabolic stress, has been identified as crucial for defining the vulnerability of neurons to neurodegenerative stressors.

However, targeted neuroprotective and restorative therapeutical interventions, would only be possible if the underlying molecular pathomechanisms of the disease understood. Since this is presently not the case, there is still no therapy with which the PD patients can be cured and treated symptomatically and with increasing disease duration. A curative PD therapy would slow down or stop disease progression, or even prevent manifestation of motor-symptoms. While theses therapies are the aim of intensive ongoing research and clinical trials, current established PD therapies however, are only symptomatic, aiming in particular to substitute the loss of dopamine in the brain or delay its metabolism (e.g. by dopamine-mimetics like L-DOPA the blood-brain-barrier permissive precursor of dopamine, dopamine receptor agonists, or monoamine oxidase inhibitors, like MAO-B). However, these therapies have considerable side effects (e.g., dyskinesia, hallucinations, and impulsive / compulsive syndrome ICB) that become more and more severe over time of treatment.

A novel potentially neuroprotective PD therapy is currently in a phase III clinical trial: it aims at voltage gated calcium channels called L-type (more precisely Cav1.3 or Cav1.2), and its pharmacological inhibition by drugs, like the dihydropiridin DHP isradipine (*Parkinson Study Group, 2013, PMID: 24123224, phase III clinical trial ClinicalTrials.gov Identifier: NCT02168842).* However inconclusive findings have been reported for SN DA neuroprotection in neurotoxin PD-mouse models, for pharmacological L-type calcium channel inhibition (by isradipine) or for genetic Cav1.3 ablation (*Ilijic etal., 2011 PMID: 21515375; Ortner et al., 2017, PMID: 28592699).*

In summary, besides a very limited patient collective for neurosurgical options (deep brain stimulation), at present only symptomatic pharmacological dopamine-mimetic PD-therapies, are established that provide decreasing efficiency with increasing side effects over time.

Cav2.3 R-type calcium channel antagonists for treating neurodegenerative diseases such as Parkinson's disease are known from:

US 2012/321594 A1 (HONG KYONSOO [US] ET AL) 20 December 2012 (2012-12-20)
RODOLPHE HAJJ ET AL: "Combination of acamprosate and baclofen as a promising therapeutic approach for Parkinson's disease",

SCIENTIFIC REPORTS,

vol. 5, no. 1, 6 November 2015 (2015-11-06), XP055445739, DOI: 10.1038/srep16084

GEZA BERECKI ET AL: "Differential Ca v 2.1 and Ca v 2.3 channel inhibition by baclofen and [alpha]-conotoxin Vc1.1 via GABA B receptor activation",

JOURNAL OF GENERAL PHYSIOLOGY,

vol. 143, no. 4, 31 March 2014 (2014-03-31), pages 465-479, XP55445926, NEW YORK, US

ISSN: 0022-1295, DOI: 10.1085/jgp.201311104

US 2014/038927 A1 (COHEN DANIEL [FR] ET AL) 6 February 2014 (2014-02-06)

M. J. CANO-CEBRIAN ET AL: "Local acamprosate modulates dopamine release in the rat nucleus accumbens through NMDA receptors: an in vivo microdialysis study",

NAUNYN-SCHMIEDEBERG'S ARCHIVES OF PHARMACOLOI vol. 367, no. 2, 1 February 2003 (2003-02-01), pages 119-125, XP055445779,

DE

ISSN: 0028-1298, DOI: 10.1007/s00210-002-0674-3

Komeili Gholamreza ET AL: "Effects of Isradipine on Induced-Parkinson's Disease in Rats",

Zahedan Journal of Research in Medical Sciences,

vol. In Press, no. In Press, 26 April 2016 (2016-04-26), XP55818555, DOI: 10.17795/zjrms-6444

ARIAS R L ET AL: "Amiloride is neuroprotective in an MPTP model of Parkinson's disease",

NEUROBIOLOGY OF DISEASE, ELSEVIER, AMSTERDAM, NL, vol. 31, no. 3, 1 September 2008 (2008-09-01), pages 334-341, XP023905928,

ISSN: 0969-9961 , DOI: 10.1016/J.NBD.2008.05.008

[retrieved on 2008-07-07]

Gomez-Mancilla Baltazar ET AL: "Effect of ethosuximide on rest tremor in the MPTP monkey model: ETHOSUXIMIDE AND REST TREMOR",

MOVEMENT DISORDERS,

vol. 7, no. 2, 1 January 1992 (1992-01-01), pages 137-141, XP55818897, US

ISSN: 0885-3185, DOI: 10.1002/mds.870070207

Schneider Toni ET AL: "How "Pharmacoresistant" is Cav2.3, the Major Component of Voltage-Gated R-type Ca2+ Channels?",

Pharmaceuticals,

vol. 6, no. 6, 1 January 2013 (2013-01-01), pages 759-776, XP055805750, DOI: 10.3390/ph6060759

Retrieved from the Internet:

URL:https://www.ncbi.nlm.nih.gov/pmc/articles/PMC3816731/pdf/ pharmaceuticals-06-00759.pdf

Hainsworth Atticus H. ET AL: "Actions of sipatrigine, 202W92 and lamotrigine on R-type and T-type Ca2+ channel currents",

European Journal of Pharmacology,

vol. 467, no. 1-3, 1 April 2003 (2003-04-01), pages 77-80, XP55818901, NL

ISSN: 0014-2999, DOI: 10.1016/S0014-2999(03)01625-X

JONES-HUMBLE 5 A ET AL: "The novel anticonvulsant lamotrigine prevents dopamine depletion in C57 black mice in the MPTP animal model of Parkinson's disease",

LIFE SCIENCE, PERGAMON PRESS, OXFORD, GB,

vol. 54, no. 4, 1 January 1994 (1994-01-01), pages 245-252, XP023748453,

ISSN: 0024-3205, DOI: 10.1016/0024-3205(94)00813-2 [retrieved on 1994-01-01]

WO9415607 A (Rhone Poulenc Rorer SA, published on 21.07.1994

It is therefore an object of the present invention to provide an improved and more effective treatment and therapy for Parkinson's disease.

**Summary of the Invention**

The invention relates to a Cav2.3 R-type calcium channel antagonist for use in the treatment of Parkinson's disease, wherein the antagonist is a CRISPR/Cas9 construct, and wherein said antagonist partially or fully inhibits the functional expression of the calcium voltage-gated channel subunit alpha 1E (CACNA1E) gene .

Differential vulnerability of different neuronal population to neurodegenerative stressor is a hallmark of all neurodegenerative diseases. Elevated metabolic stress and altered calcium homeostasis are not only interdependent factors, but seem also provide a crucial converging general downstream event in neurodegenerative disease and age-dependent neuron loss *(Duda et al., 2016, PMID: 26865375),* and thus offer attractive targets for therapeutical interventions.

In Parkinson's disease (PD) dopamine-releasing neurons within the *Substantia nigra* (SN DA) are particularly vulnerable to PD-stressors and to degeneration compared to other neighboring dopaminergic neurons. The age-dependent, progressive loss of SN DA neurons is a pathological hallmark of PD, as the resulting loss of striatal dopamine causes its major movement-related symptoms (such as problems with voluntary movement control, as well as rigidity, tremor, akinesia, and postural instability). SN DA neurons release dopamine from their axonal terminals within the dorsal striatum, and also from their cell bodies, and dendrites within the midbrain, in a calcium- and activity-dependent manner. Their intrinsically generated and metabolically challenging activity is created and modulated by the orchestrated function of different ion channels and dopamine D2-autoreceptors. While the cause of most PD cases is unclear and no curative therapies are available, neuron-specific calcium ($Ca^{2+}$) homeostasis and metabolic stress are considered important factors *(Schapira and Patel, 2014, PMID: 24664140; Duda et al., 2016, PMID: 26865375; Oertel and Schulz, 2016, PMID: 27577098; Singleton and Hardy, 2016, PMID: 27311081).*

Voltage-gated $Ca^{2+}$ channels (VGCCs), especially the Cav1.3 L-type, received particular attention, as they not only modulate activity-pattern and dopamine release of SN DA neurons, but also generate an activity-related, stressful dendritic $Ca^{2+}$ load that could trigger neurodegeneration and PD *(Guzman et al., 2010, PMID: 21068725; Surmeier et al., 2017, PMID: 28104909).*

The inventor had previously shown that Cav1.3 channels - besides stabilizing metabolic demanding intrinsically generated pacemaker activity of SN DA neurons - can also transiently sensitize inhibitory dopamine-autoreceptors of the D2-type (D2-AR) in SN DA neurons involving the neuronal $Ca^{2+}$ sensor NCS-1, in a supposed protective feedback-loop. This feedback mechanism is mediated by Cav1.3 LTCCs, serving as a $Ca^{2+}$ source for NCS-1 in SN DA neurons, supporting its $Ca^{2+}$ dependent binding to D2-ARs, and preventing their desensitization *(Dragicevic et al., PMID: 24934288)* and likely counteracts activity-dependent oscillatory elevated metabolic stress levels and cell death *(Guzman et al., 2010, PMID: 21068725).* But it was observed *only transiently* in mouse SN DA neurons in response to elevated dopamine levels, e.g. due to *in vivo* administration of L-DOPA (still the gold-standard in PD-therapy). In summary, the physiological roles of Cav1.3 LTCCs in SN DA neurons seem to be complex and are still not clear, and a causal neuroprotective effect of LTCC blockers (like isradipine) remains to be demonstrated in humans, while its effect in mouse models of PD are inconclusive / ambiguous. In summary, until today, no protective effect of DHPs, in particular isradipine, has been shown in prospective studies.

In contrast, the inventors have surprisingly found that another type of voltage gated calcium channel, the R-type or Cav2.3 channel (or the CACNALE gene, coding for all Cav2.3 variants), is significantly contributing to the high vulnerability of SN DA neurons to PD-stressors and to degeneration. These findings therefore challenge the current view on the role of L-Type VGCCs (Cav1.2/Cav1.3) as a main contributing factor for the high vulnerability of SN DA neurons to PD-stressors, and as drug targets for neuroprotective PD-therapy, as well as the ongoing search for novel Cav1.3 specific L-Type VGCC inhibitors as new PD-drugs *(Kang et al., 2012, PMID: 23093183; Orner et al., 2014, PMID: 24941892).*

The inventor shows that (i) Cav2.3 voltage gated channel activity in the major source for somatic activity-related calcium oscillations in the cell-bodies of mouse SN DA neurons, and not Cav1.3 or Cav1.2 L-Type VGCCs *(Ortner et al., 2017, PMID: 28592699)*, that (ii) Cav2.3 VGCC activity constitutively stimulates NCS-1/D2-AR signalling in SN DA neurons (as a presumed compensatory response to stressful Cav2.3 mediated somatic calcium-oscillations), and that (iii) NCS-1 activity does protect SN DA neurons from degeneration, and that (iv) highly vulnerable SN DA neurons (and their axonal projections) are fully protected in a chronic neurotoxin *in vivo* PD-mouse model (MPTP/probenecid) in Cav2.3 KO mice, with genetic Cav2.3 ablation in all cells. While SN DA neurons in a NCS-1 KO mouse display a higher vulnerability to degenerative stressors. Human genetic and cell-specific expression data complement these comprehensive mouse model data (see Examples).

The inventors discovery of Cav2.3 as the "stressful" calcium channel in highly vulnerable SN DA neurons, that is however not downregulated in human SN DA neurons in PD, is supported by a Cav2.3 downregulation in response to a PD-stressor (PARK1) in mouse dorsal vagal motor-neurons, as these neurons display a similar stressful calcium based pacemaker, but for unknown reason are much more resistant than SN DA neurons to degeneration in PD *(Goldberg et al., 2012 PMID: 22941107; Lasser-Katz et al., 2017, PMID: 28053029).*

These findings of the inventor lead to the conclusion that negative modulation of Cav2.3 channels offers a novel therapeutic avenue for PD, and that this intervention could have neuroprotective effects not only for SN DA neurons but also for other highly vulnerable neurons in PD.

Specifically, preventive administration or administration following early PD diagnosis, of suitable substances (i.e. antagonists) which inhibit functional activity Cav2.3 calcium channels or their functional expression, effects a slow down or an hold of the progressive loss of SN DA neurons. Such suitable substances according to the present invention relates to antagonists of Cav2.3 R-type calcium channels.

The term "modulate" or "modulation" according to the invention means a negative modulation and has to be understood as partially or fully inhibiting or impeding, e.g. downregulatiing, the functional expression of the calcium voltage-gated channel subunit alpha 1E (CACNA1E) gene.

The term "reduce" the activity of a Cav2.3 R-type calcium channel means that the functional activity is less than under normal conditions, i.e. without any modulation of the Cav2.3 R-type calcium channel. The activity can be fully or partly inhibited or blocked or interfered.

Any extent by which the functional activity or the functional expression of the Cav2.3 R-type calcium channel is reduced means a modulation according to the invention.

Preferably, the functional activity is reduced up to 10%, preferably up to 30%, more preferably up to 50%, even more preferably up to 70% and most preferably up to 100% compared to the normal activity of the Cav2.3 R-type calcium channel.

The term "downregulate / downregulation" means that the functional expression occurs to a lesser extent than under normal conditions, i.e. without any modulation of the Cav2.3 R-type calcium channel. The expression can be fully or partly inhibited or blocked or interfered.

Any extent by which the expression of the Cav2.3 R-type calcium channel is downregulated means a modulation according to the invention. Preferably, the expression is downregulated up to 10%, preferably up to 30%, more preferably up to 50%, even more preferably up to 70% and most preferably up to 100% compared to the normal activity of the Cav2.3 R-type calcium channel.

Crispr/Cas9 constructs according to the invention effect down-regulation, modulation or prevention of expression.

The term "treatment" relates to a therapeutic treatment, prophylactic or preventative measures, protection of neurons, and deferment of the disease onset, wherein the object is to delay, prevent or slow down the targeted pathologic condition or disorder. Subjects in need of a treatment include those already with disorder indications or disorders, as well as those prone to develop these disorders.

Subjects of the present invention are preferably mammals, more preferably humans.

The term "neurodegenerative diseases" according to the disclosure relates to diseases with progressive loss of structure and/or function of neurons, including the pathological death (i.e. degeneration) of neurons, in particular but not limited to the preferential degeneration of SN DA neurons in PD and during aging.

In another embodiment, the invention relates to the Cav2.3 R-type calcium channel antagonist, wherein the antagonist is used in combination with dopamine mimetics (which have to be understood as all substances which regulate/modulate the dopamine metabolism), such as L-DOPA and dopamine receptor agonists and/or monoamine oxidase inhibitors (MAO inhibitors). The invention also provides a pharmaceutical composition comprising a Cav2.3 R-type calcium channel antagonist as defined in present claim 1 and at least one pharmaceutically acceptable excipient and/or at least one carrier.

The term "pharmaceutically acceptable excipients" refers to excipients that do not produce an adverse, allergic or other untoward reaction when administered to an animal, preferably a human. It includes any and all solvents, dispersion media, coatings, antibacterial and antifungal agents, isotonic and absorption delaying agents and the like. For human administration, preparations should meet sterility, pyrogenicity, and general safety and purity standards.

Such pharmaceutically acceptable excipients are inherently nontoxic and nontherapeutic. Examples of such excipients include ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts, or electrolytes such as protamine sulfate, disodium hydrogen phosphate, potassium hydrogen phosphate, sodium chloride, zinc salts, colloidal silica, magnesium trisilicate, polyvinyl pyrrolidone, cellulose-based substances, and polyethylene glycol. Excipients for topical or gel-based forms of antagonist include polysaccharides such as sodium carboxymethylcellulose or methylcellulose, polyvinylpyrrolidone, polyacrylates, polyoxyethylene-polyoxypropylene-block polymers, polyethylene glycol, and wood wax alcohols. For all administrations, conventional depot forms are suitably used. Such forms include, for example, microcapsules, nano-capsules, liposomes, sublingual tablets, and sustained-release preparations.

The pharmaceutical composition may be administered by several routes of administration. Examples of adapted routes of administration include, but are not limited to intramuscular (i.m.), subcutaneous, intravenous, intraocular, transdermal, topical, parenteral, intranasal and oral administration. Parenteral administration may be by intravenous (IV) injection, subcutaneous (s.c.) injection, intramuscular (i.m) injection, intra-arterial injection, intrathecal (i.t.) injection, intra-peritoneal (i.p.) injection, or direct injection or other administration to the subject. Preferred routes of administration are oral and parenteral.

The pharmaceutical composition may be designed to be short-acting, fast-releasing, long-acting, or sustained-releasing as described herein. Thus, the pharmaceutical formulations may also be formulated for controlled release or for slow release.

The pharmaceutical composition preferably comprises a delivery system that controls the release of the composition. Examples of suitable carriers for sustained or delayed release include, but are not limited to, gelatin, gum Arabic, xanthane polymers, thermoplastic resins such as, for example polyvinyl halides, polyvinyl esters, polyvinylidene halides and halogenated polyolefins, elastomers such as, for example, brasiliensis, polydienes, and halogenated natural and synthetic rubbers, and flexible thermoset resins such as polyurethanes, epoxy resins, biodegradable polymers and the like.

The blood-brain barrier (BBB) is a physical barrier and system of cellular transport mechanisms between the blood vessels in the central nervous system (CNS) and most areas of the CNS itself. The BBB maintains homeostasis by restricting the entry of potentially harmful chemicals from the blood, and by allowing the entry of essential nutrients. However, the BBB can pose a barrier to delivery of pharmacological agents to the CNS for treatment of disorders. The pharmaceutical composition may preferably be used in conjunction with delivery systems that facilitate delivery of the agents to the central nervous system. For example, various blood brain barrier (BBB) permeability enhancers may be used to transiently and reversibly increase the permeability of the blood brain barrier to a treatment agent. Such BBB permeability enhancers include but are not limited to leukotrienes, bradykinin agonists, histamine, tight junction disruptors (e.g., zonulin, zot), hyperosmotic solutions (e.g., mannitol), cytoskeletal contracting agents, and short chain alkylglycerols (e.g., 1-O-pentylglycerol). Oral, sublingual, parenteral, implantation, nasal and inhalational routes can provide delivery of the active agent to the central nervous system. In some embodiments, the compounds of the present invention may be administered to the central nervous system with minimal effects on the peripheral nervous system.

For the prevention or treatment of Parkinson's disease, the appropriate dosage of antagonist will depend on the severity and course of the disease, whether the antagonists are administered for preventive or therapeutic purposes, previous therapy, the patient's clinical history and response to the antagonist, and the discretion of the attending physician. The antagonist is suitably administered to the patient at one time or over a series of treatments.

In one embodiment of the pharmaceutical composition, the antagonist is used in combination with dopamine mimetics (which have to be understood as all substances which regulate/modulate the dopamine metabolism), like L-DOPA and dopamine receptor agonists and/or monaminoxidase inhibitors (MAO inhibitors).

A "biological sample" according to the present invention relates to tissue, primary cells, cell-lines that are endogenously expressing the Cav2.3 ion channel or cell lines that are transiently or stably transfected or stimulated to express the Cav2.3 ion channel or any fractions of it. In these biological samples the activity of the Cav2.3 channels can be investigated by biophysical techniques (for example but not limited to electrophysiological, conductance, impedance or spectral methods), by radiometric or fluorescence displacement, or by signals that are related to an activity of the Cav2.3 channel as for example, but not limited to membrane potential, conductance, or changes of calcium or other charge carriers detected by optical, biophysical, electrical or analytical methods.

The methods according to the invention can be performed as described in McManus OB (2014) HTS assays for developing the molecular pharmacology of ion channels. Curr Opin Pharmacol. 15:91-96. and Hai-bo YU, Min LI, Wei-ping WANG, Xiao-liang WANG (2016) High throughput screening technologies for ion Channels, Acta Pharmacologica Sinica 37: 34-43

The measuring of the inhibition of the Cav2.3 R-type calcium channel can further be performed by using standard electrophysiological, analytical, biophysical, binding, fluorescence or luminescence methods as described in the Assay Guidance Manual as updated on March 31, 2017 (https://www.ncbi.nlm.nih.gov/books/NBK100915/). This includes methods to:

- evaluate currents (e.g. electrophysiological, patch-clamp or impedance methods);
- the change of the ion concentrations or charge differences between two compartments (including but not limited to intra- and extracellular solutions) using calcium or another suitable charge carrier (for example but not limited to barium) either labelled or un-labelled. The read-out can be fluorescence, luminescence, membrane-bound voltage sensors or analytical methods;
- the displacement of labelled agents (for example radio- or fluorescence labelled agents that have affinity to the channel or any of its modulatory sites.

**Brief Description of the Figures**

**FIGURES**

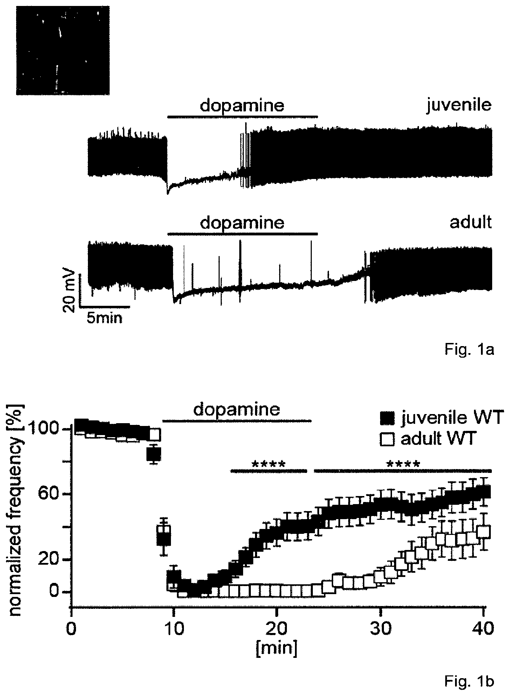

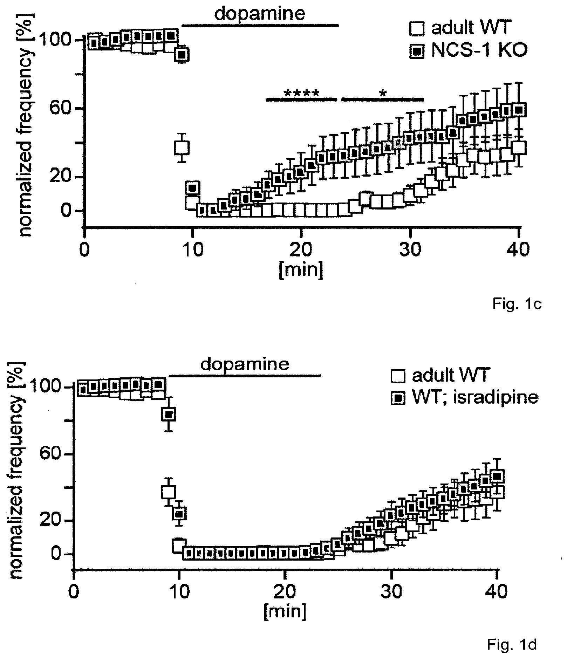

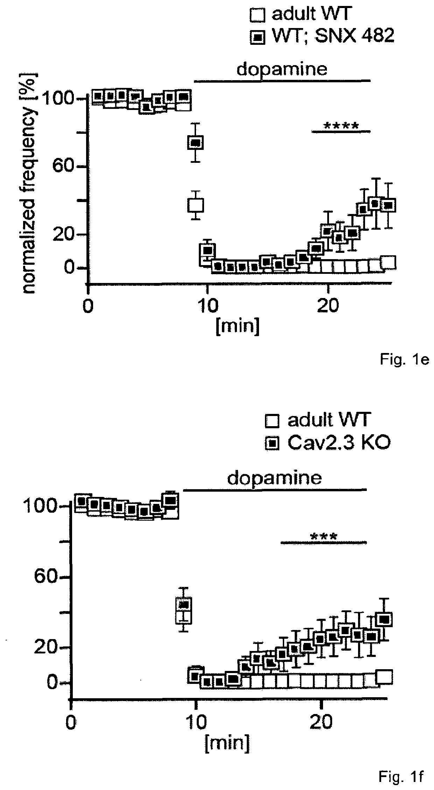

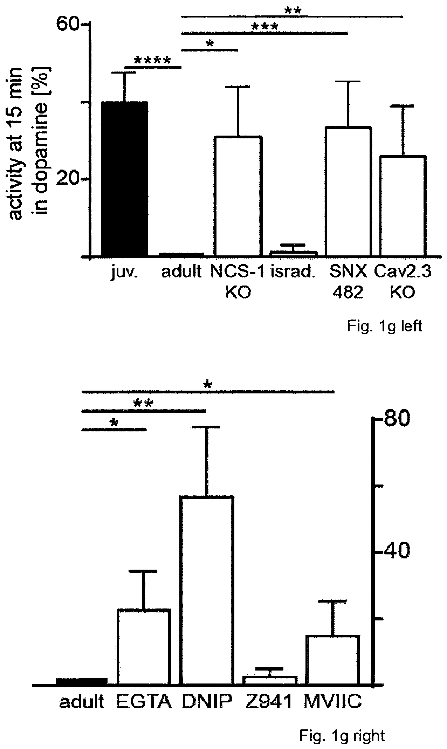

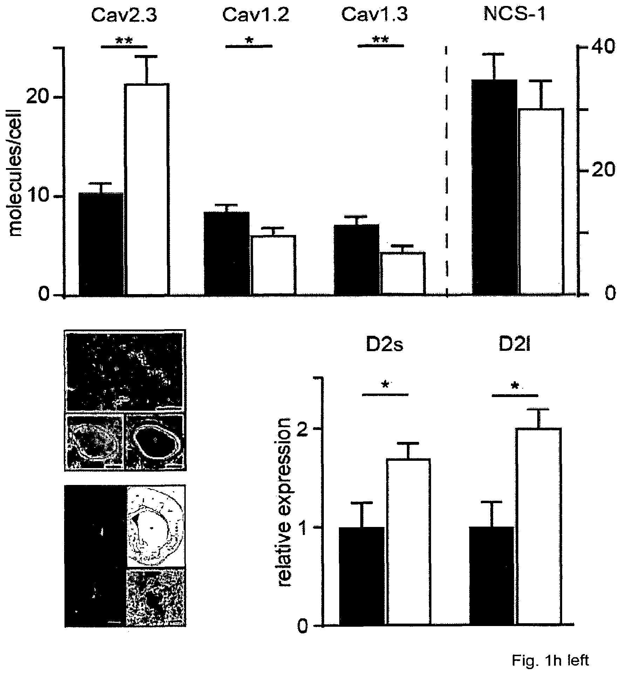

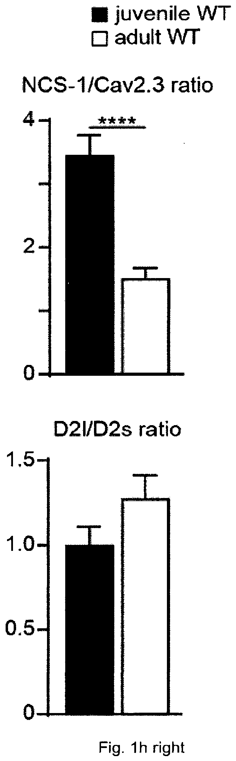

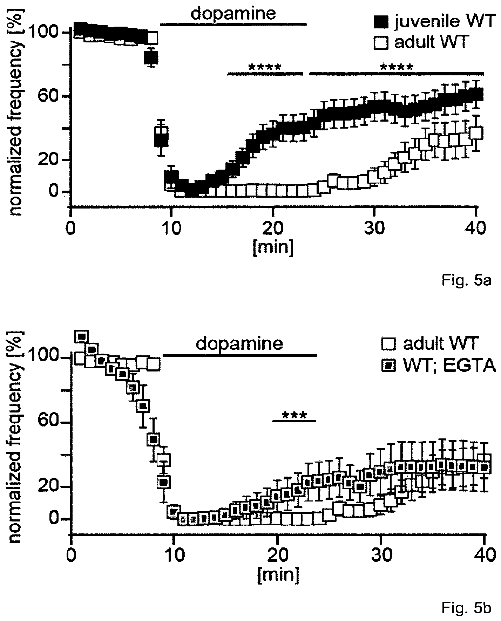

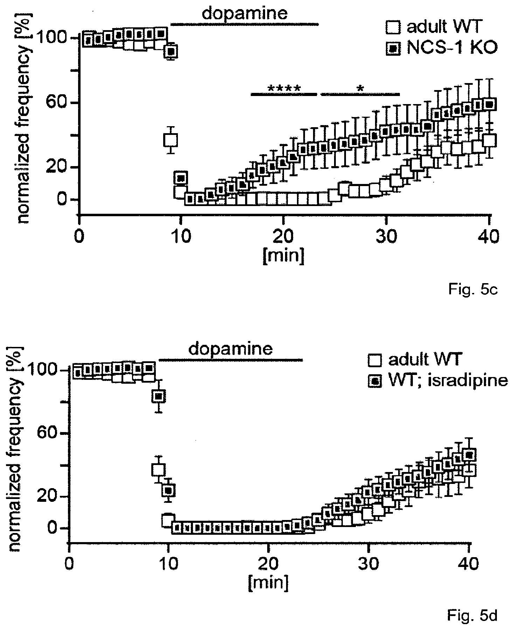

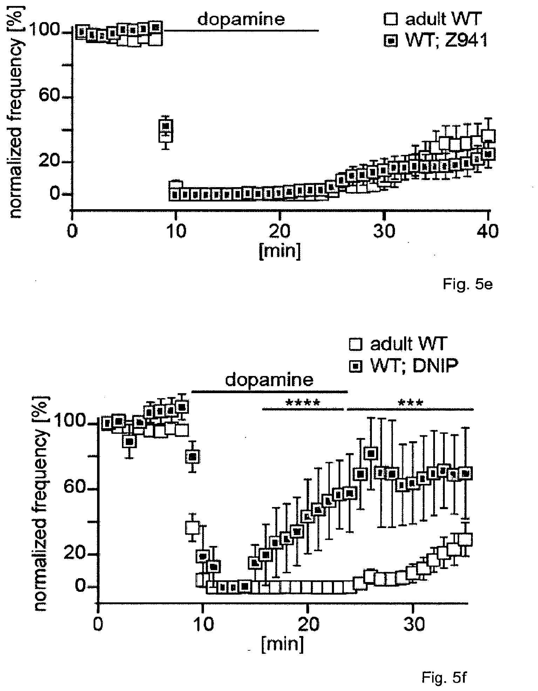

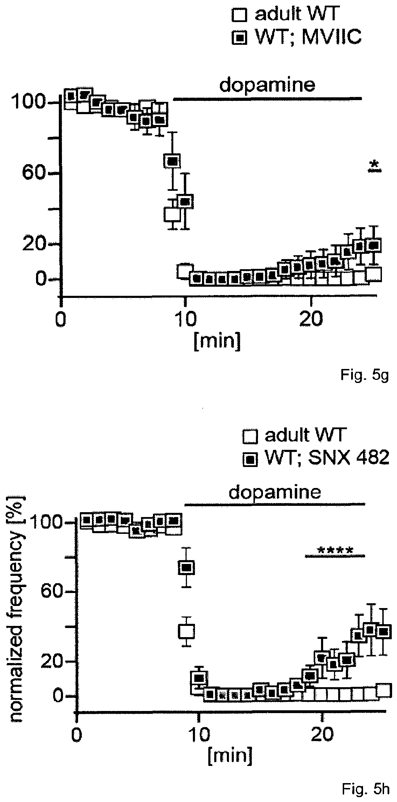

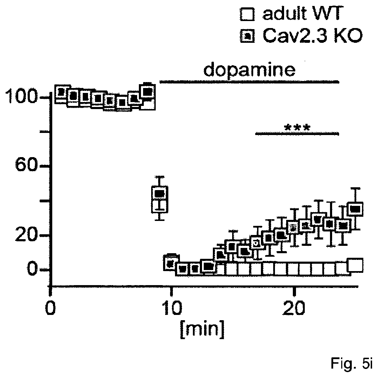

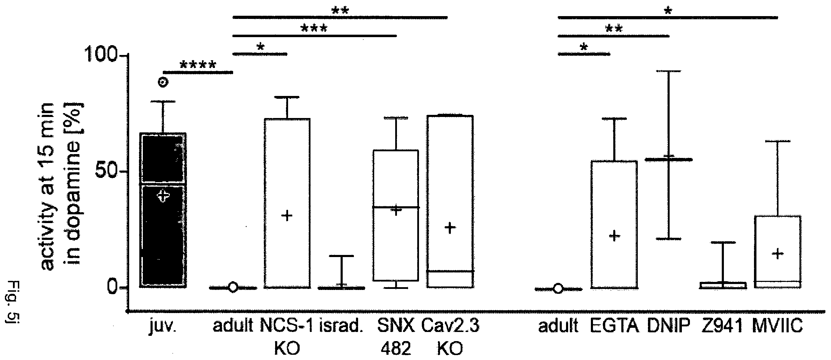

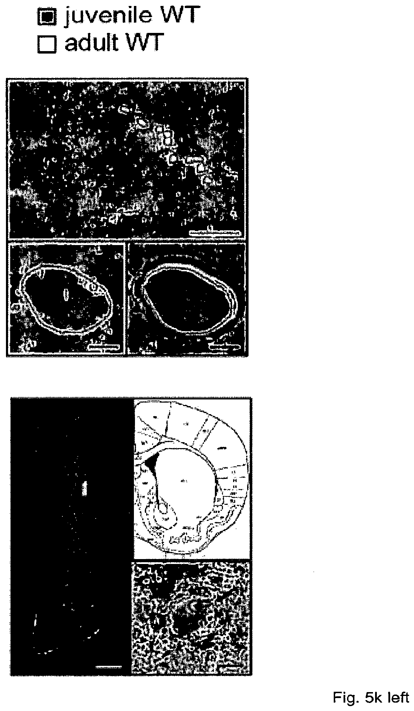

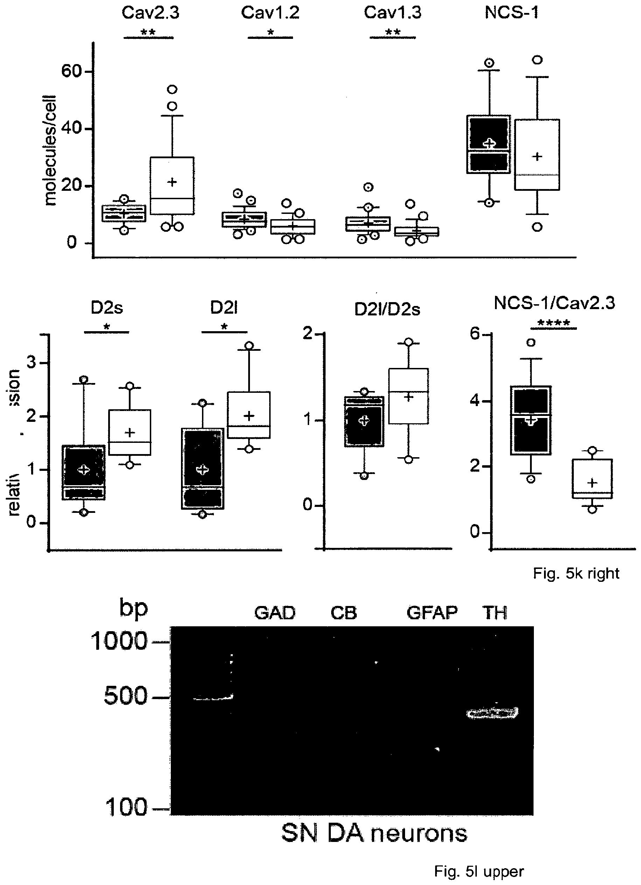

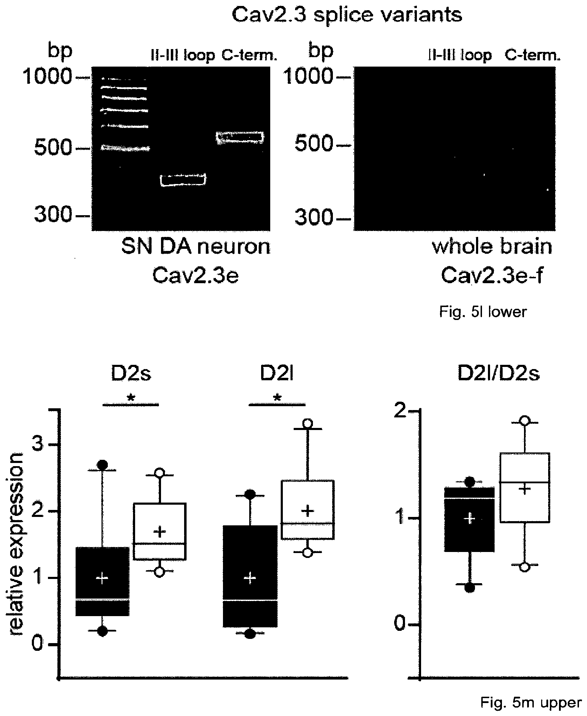

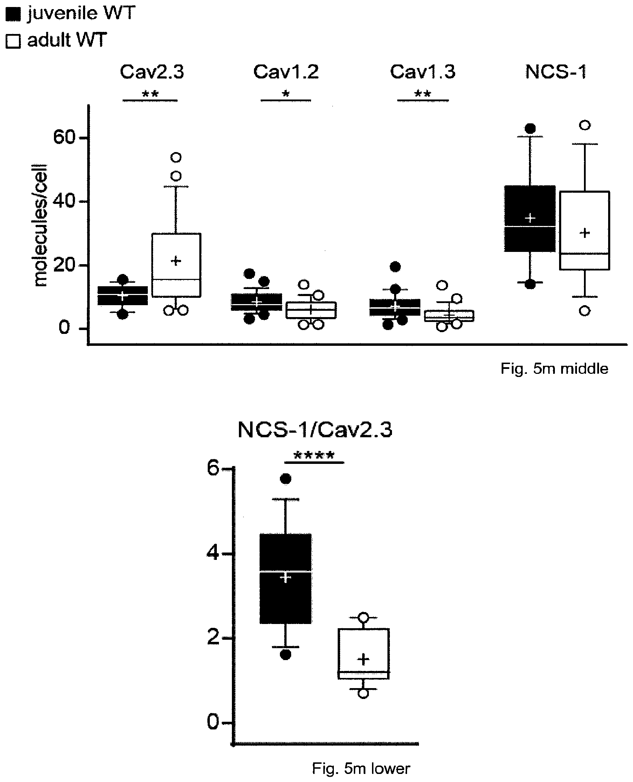

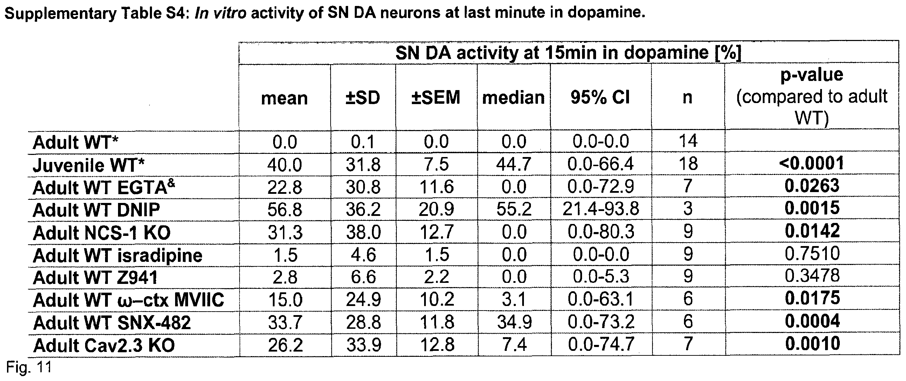

**Figure 1** demonstrates that sensitized dopamine-autoinhibition in mature SN DA neurons is mediated by Cav2.3, $Ca^{2+}$ and NCS-1/D2-AR interaction. **a,** SN DA neuron activity recorded from *in vitro* brain slices in perforated patch (insert). Dopamine (100 µM) bath-application as indicated. **b-f,** Normalized frequencies plotted over time for all analyzed SN DA neurons (data set in b partly from Dragicevic et al., 2014, PMID: 24934288Application of isradipine (300 nM), SNX-482 (100 nM) and mouse strain as indicated (WT, NCS-1 KO, Cav2.3 KO). **g,** Relative activity of SN DA neurons (from **Fig. 1b-f,** and Fig. 5 a-i) at the last minute in dopamine. **h,** UV-LMD & RT-qPCR results for Cav2.3, Cav1.2, Cav1.3, neuronal $Ca^{2+}$ sensor NCS-1, and for dopamine-autoreceptors (short and long splice variants D2s/D2l) in juvenile and adult mouse SN DA neurons. **Upper pictures:** juvenile mouse-brain-section before & after UV-LMD. Scale bars: 250 $\mu$m / 10 $\mu$m. **Lower pictures:** Striatal injection site of a retrogradely traced adult mouse-brain, aligned with mouse-brain atlas & labelled SN DA neuron. Scale bars: 10 $\mu$m. Data detailed in **Supplementary Tables S4, S5, S7A (Figs. 11,12,14).** Error bars: SEM.

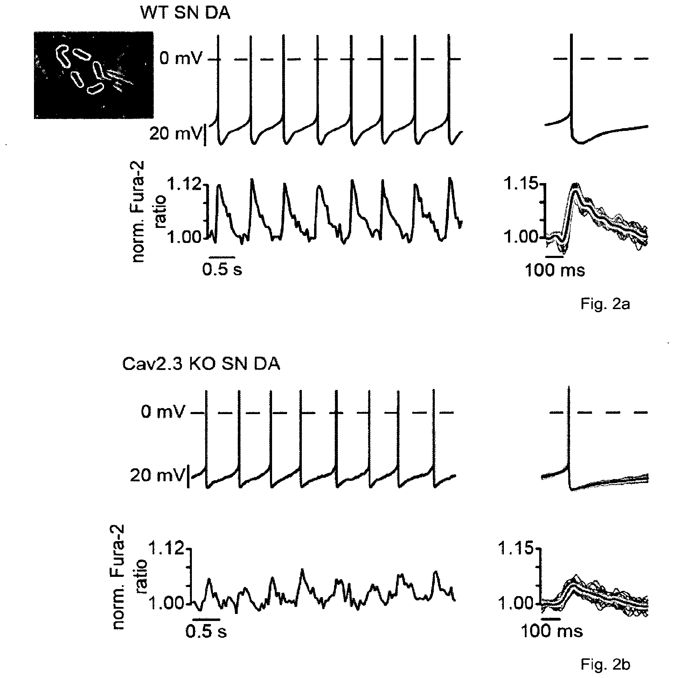

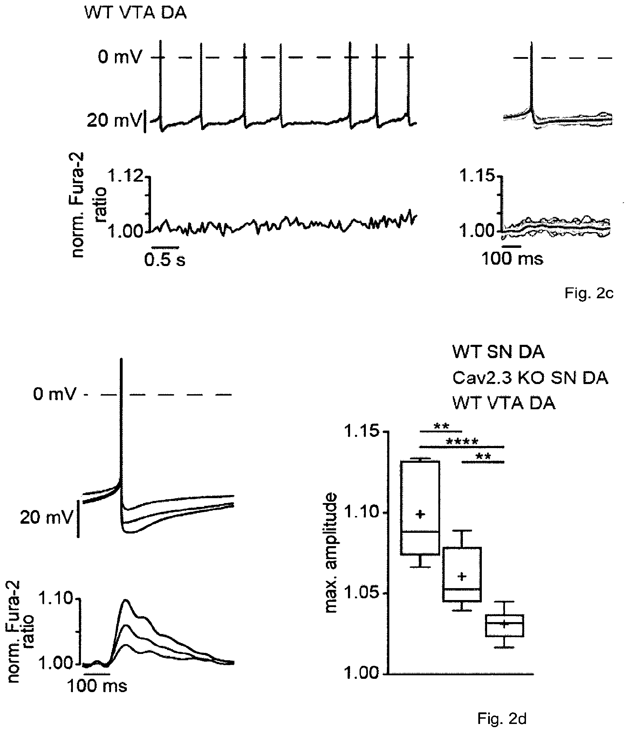

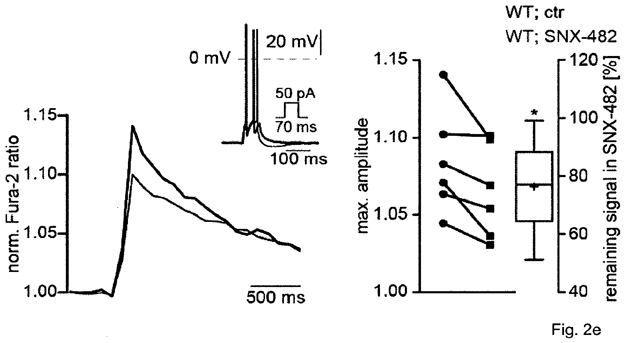

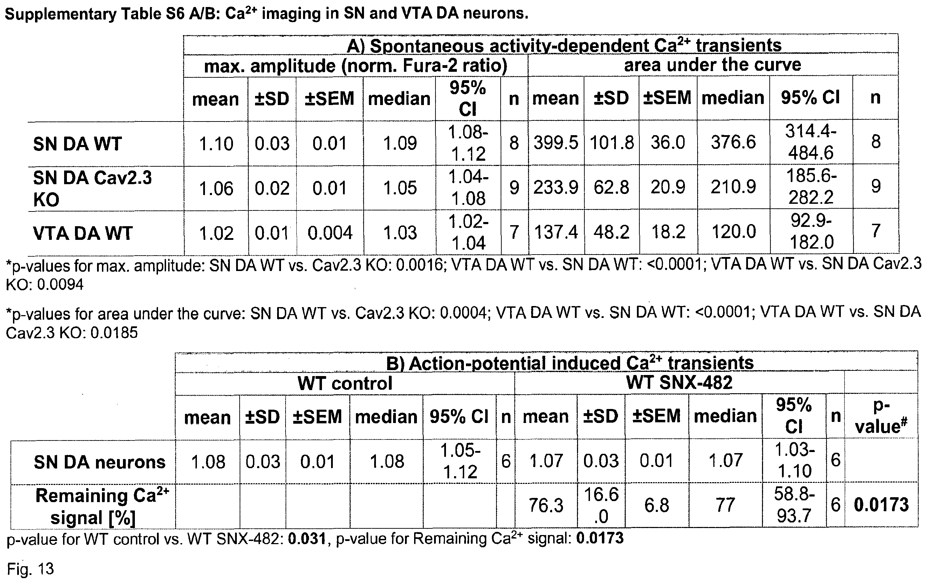

**Figure 2** shows that somatic activity-related $Ca^{2+}$ oscillations in SN DA neurons are to a large extent mediated by Cav2.3 channels. Neurons were recorded in the perforated-patch clamp configuration while somatic $Ca^{2+}$ dynamics were simultaneously imaged (insert a). **a-c, Left:** Recordings of a WT SN DA neuron (a), a Cav2.3 KO SN DA neuron (b), and a WT VTA DA neuron (c). Continuous recordings illustrate the action potential (AP) firing and the associated $Ca^{2+}$ oscillations. **Right:** Mean of 20 APs and associated mean $Ca^{2+}$ oscillations for the neurons from left. Individual traces are superimposed in grey. **d, Left:** Average spike and $Ca^{2+}$ transient of all analyzed WT SN DA (n=8; black trace) and Cav2.3 KO SN DA (n=9; blue trace), and mesolimbic WT VTA DA (n=7; red trace) neurons. **Right:** Summary boxplots showing the peak amplitudes of spike-related $Ca^{2+}$ oscillations of all analyzed neurons. **e, Left:** Voltage induced (insert) activity-related $Ca^{2+}$ transients of a WT SN DA neuron in the absence (black trace) and presence (red trace) of 100 nM SNX-482. **Right:** Relative $Ca^{2+}$ signals of all analyzed SN DA neurons in the absence and presence of SNX-482 and relative remaining $Ca^{2+}$ signal in SNX-482. Boxes: $25^{th}$ and $75^{th}$ percentiles, Whiskers: $10^{th}$ and $90^{th}$ percentiles. All data detailed in **Supplementary Table S6A/B (Fig. 13)..**

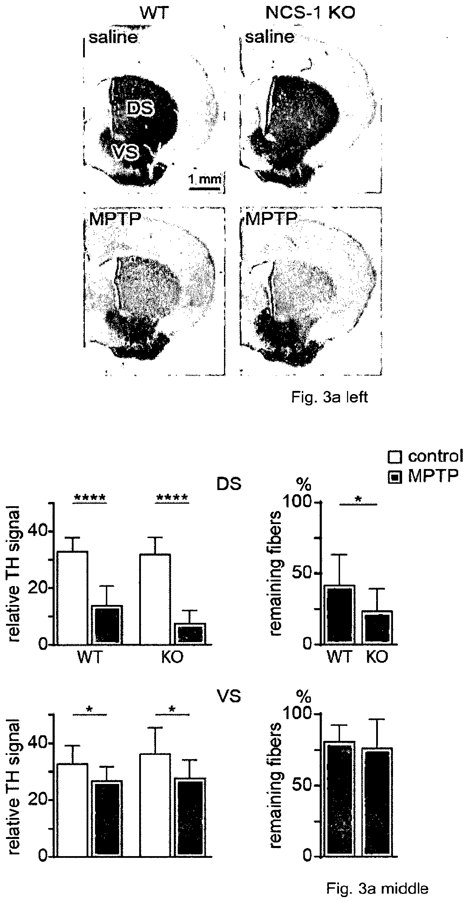

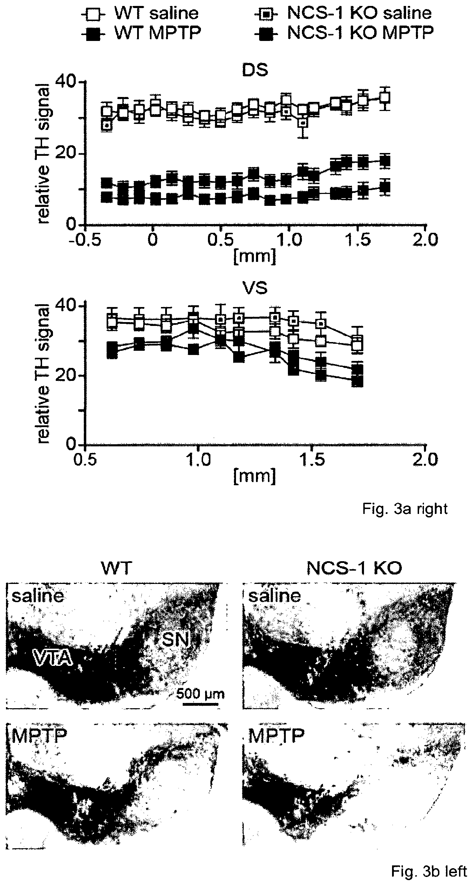

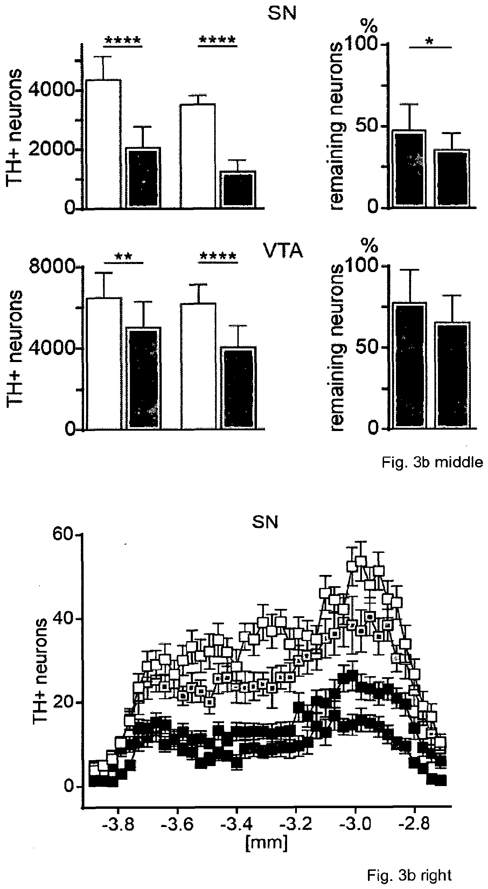

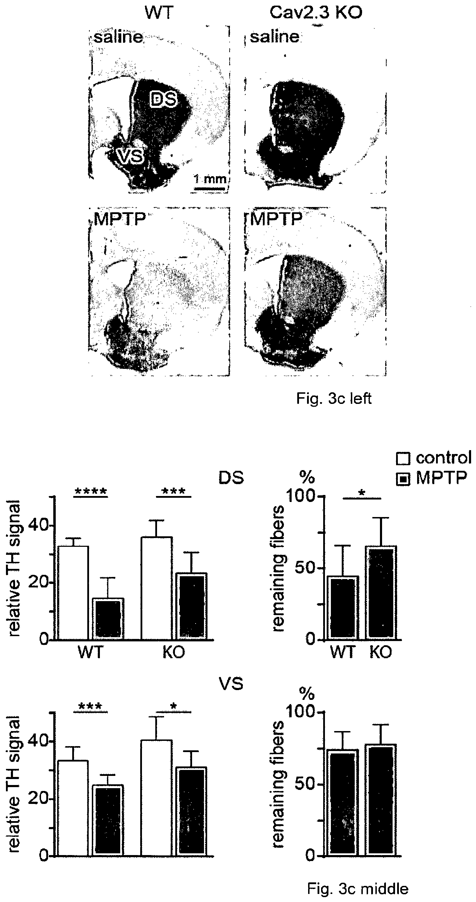

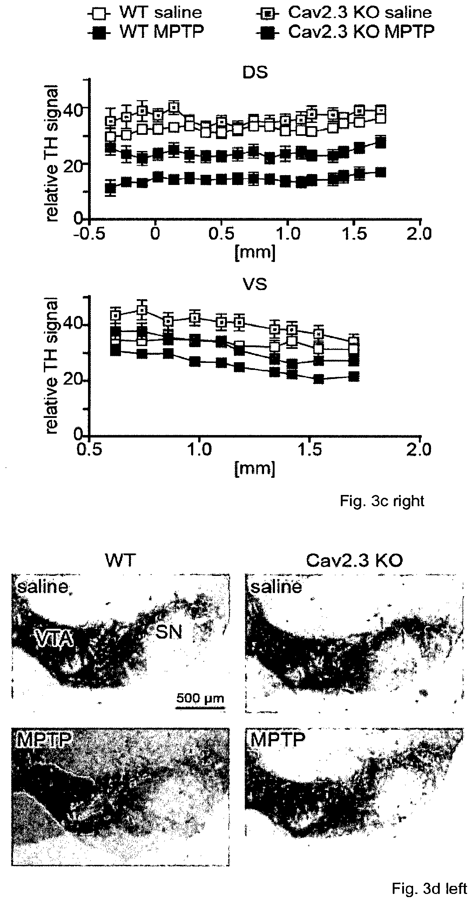

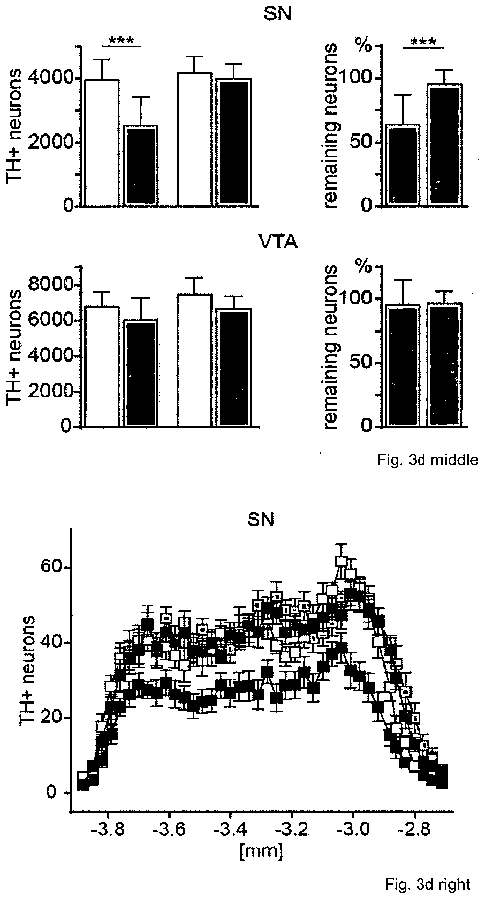

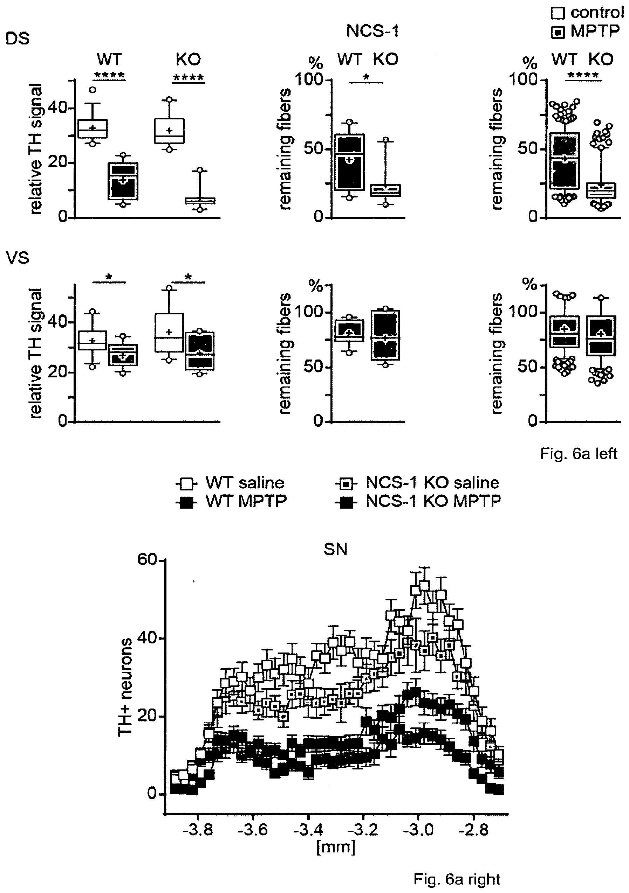

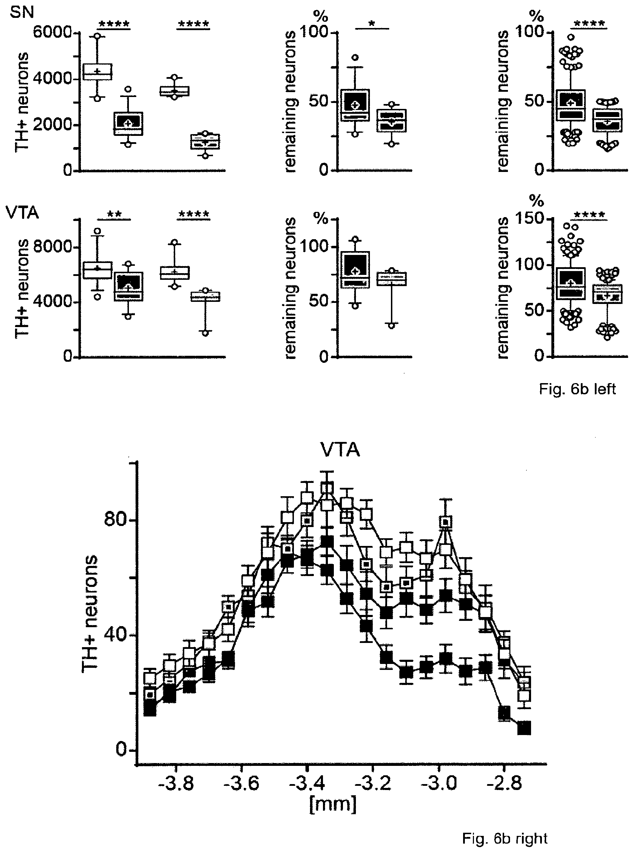

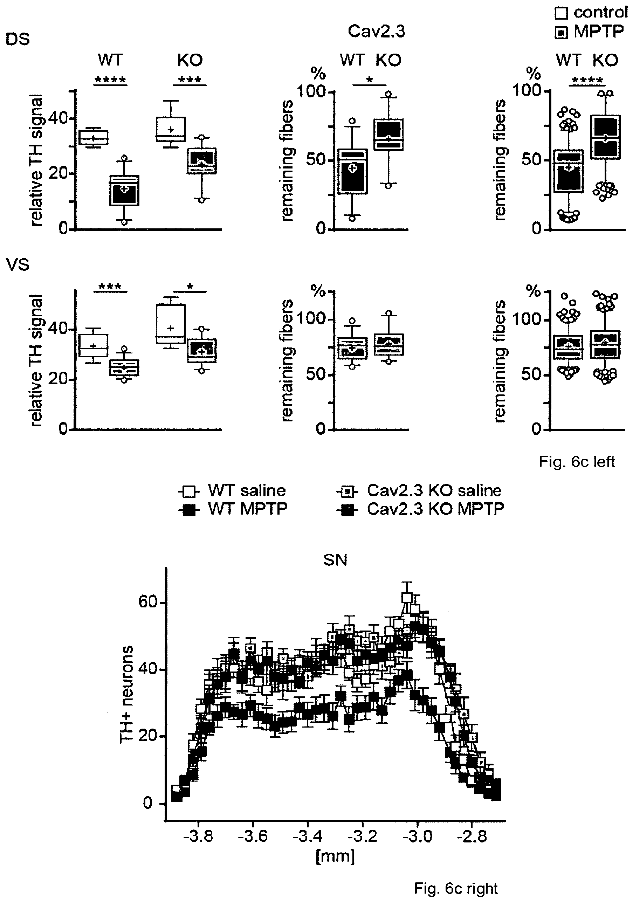

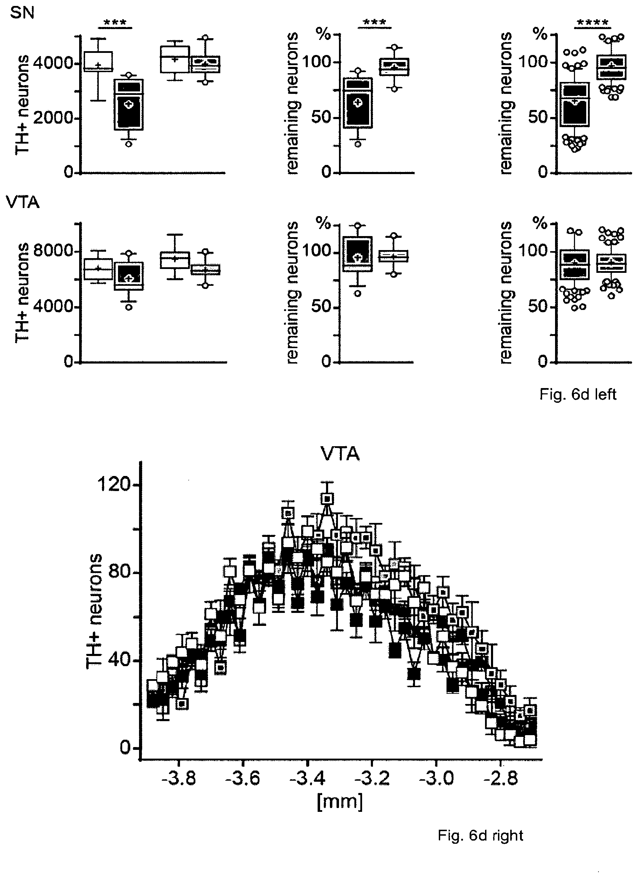

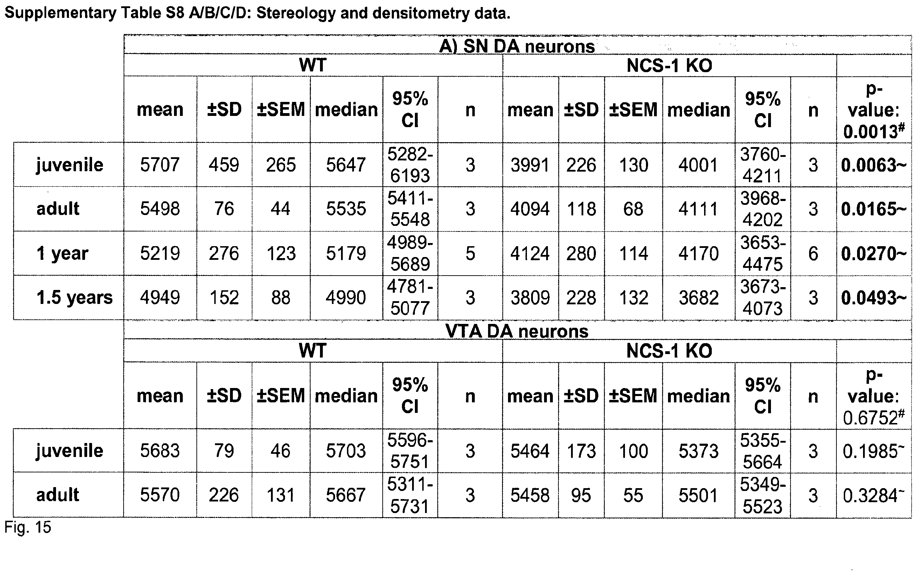

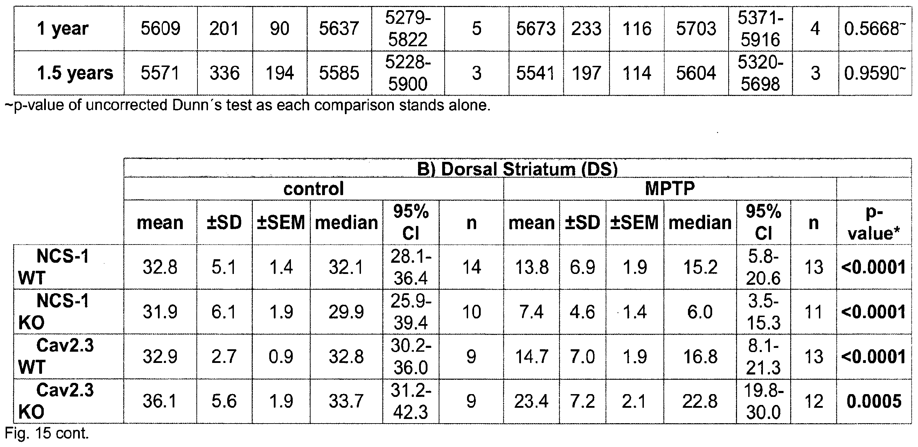

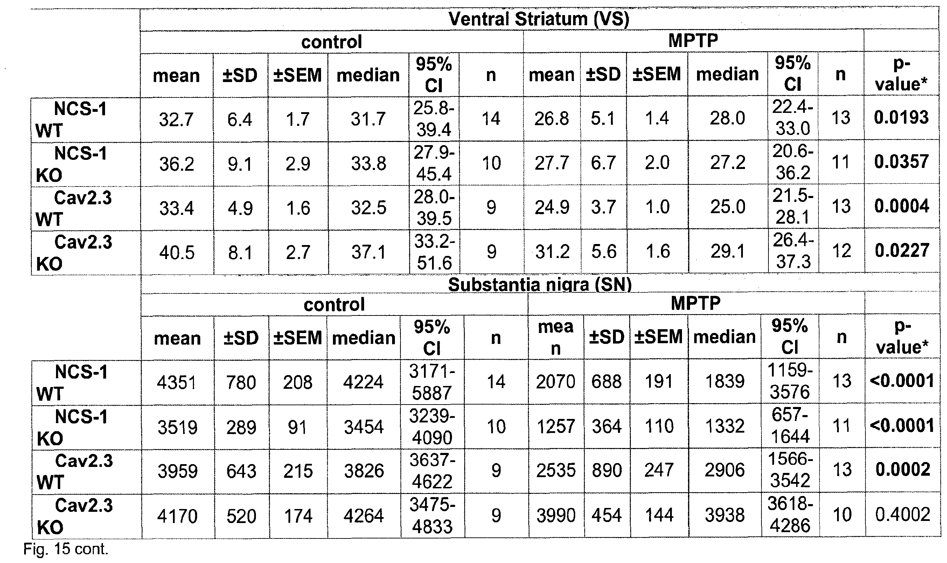

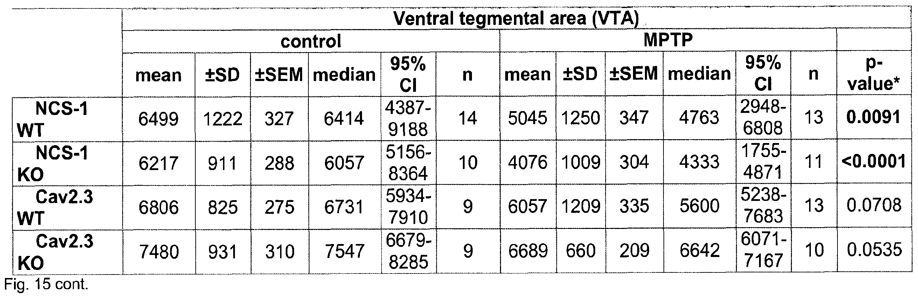

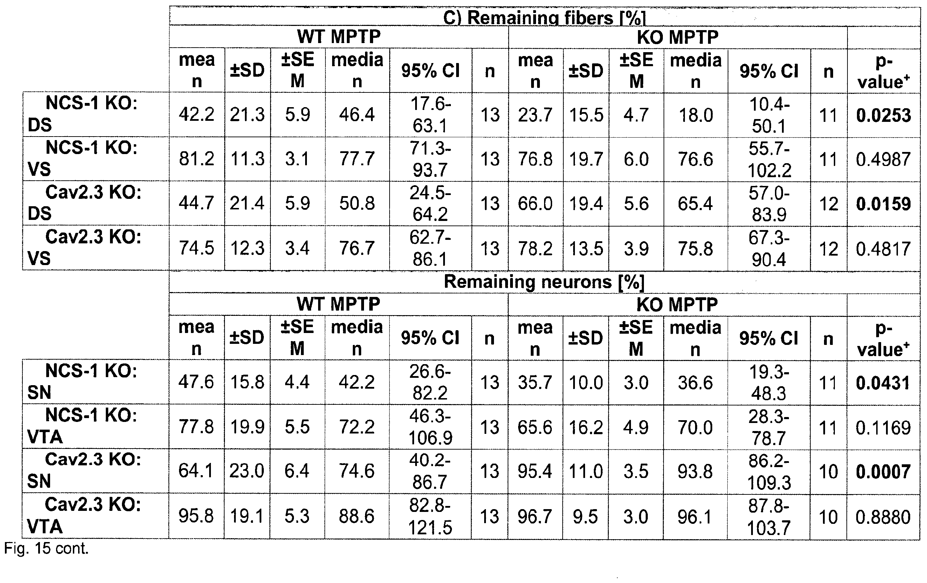

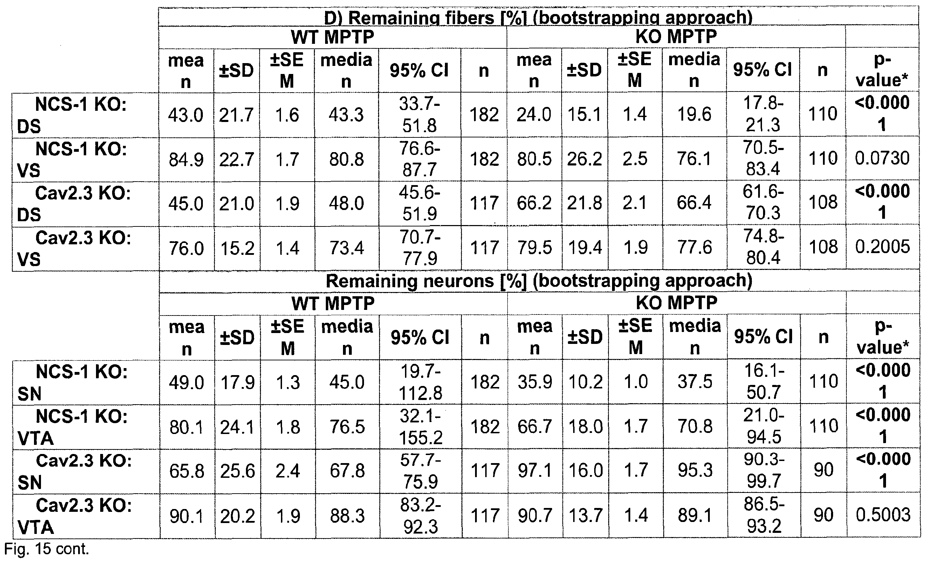

**Figure 3** demonstrates higher vulnerability of SN DA neurons in NCS-1 KO, and full and selective protection of SN DA neurons in Cav2.3 KO mice in a PD mouse-model (MPTP). **a/c, Left:** TH-immunostaining of striatal sections of WT, NCS-1 KO and Cav2.3 KO mice repeatedly treated with MPTP/probenecid or saline as controls, as indicated. **Middle:** Optical density quantification of TH-signal in dorsal striatum (DS) and ventral sitriatum (VS), and relative remaining TH-signal in MPTP mice. Error bars: SD. **Right:** Mean intensities for all analyzed sections for all animals. Error bars: SEM. **b/d, Left:** TH-immunostaining of coronal midbrain sections of WT, NCS-1 KO and Cav2.3 KO mice repeatedly treated with MPTP/probenecid or saline, as indicated. **Middle:** Stereological quantification of SN DA and VTA DA neurons, and relative remaining neurons in MPTP mice. Error bars: SD. **Right:** Mean absolute counted numbers of SN DA neurons for all analyzed sections for all animals. Error bars: SEM. Data values and additional bootstrapping analysis detailed in **Supplementary Table S8B/C/D (Fig. 15)** and **Fig. 6.**

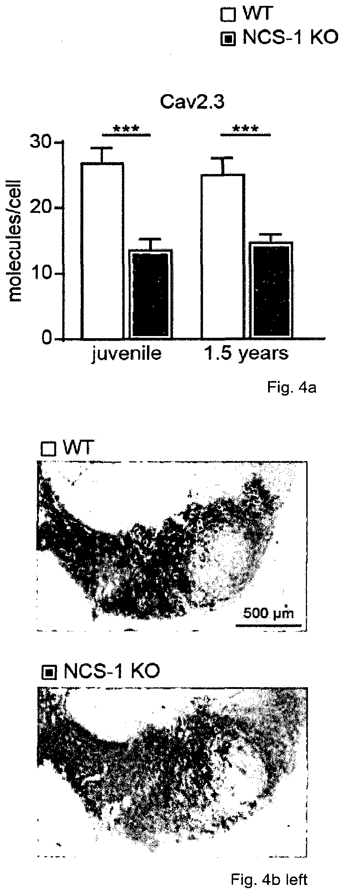

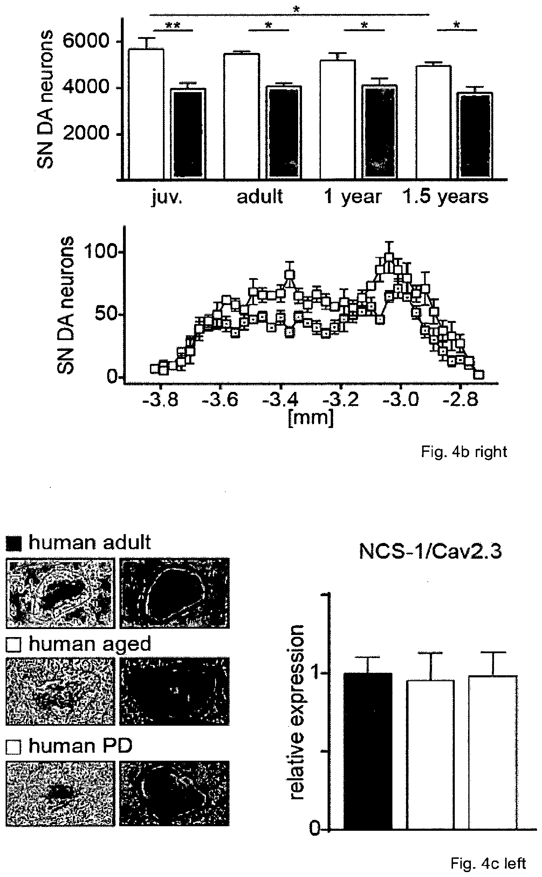

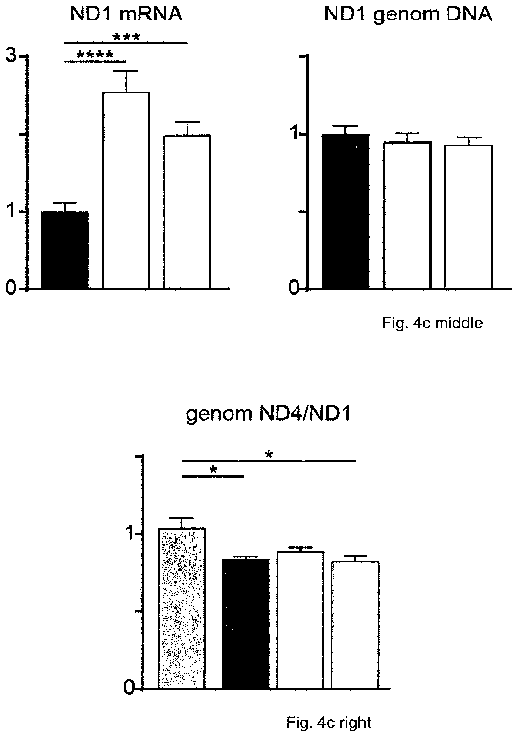

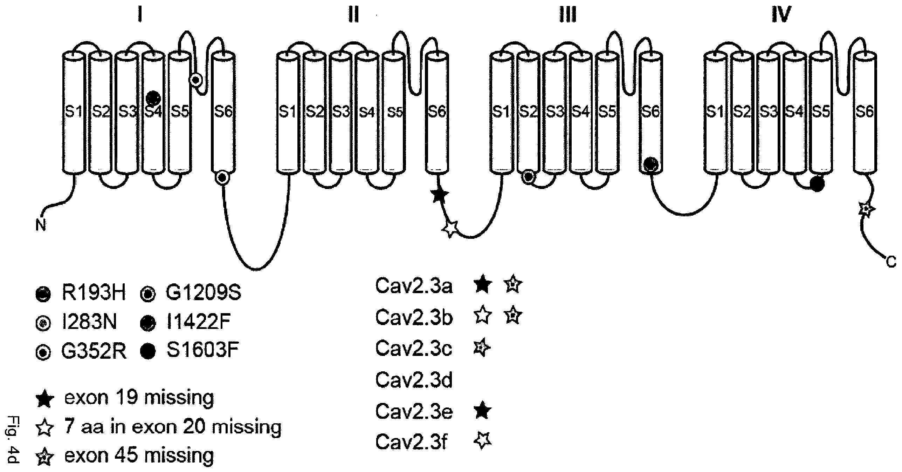

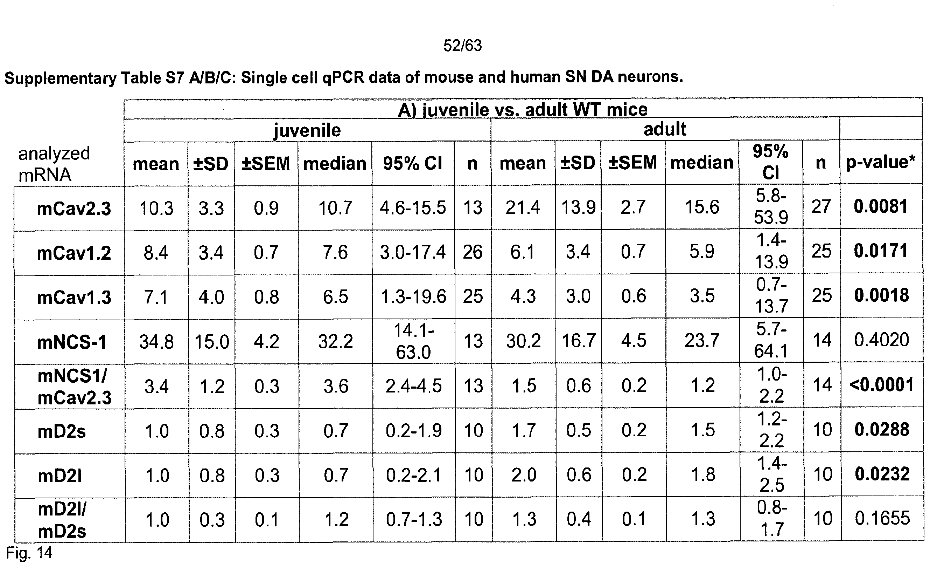

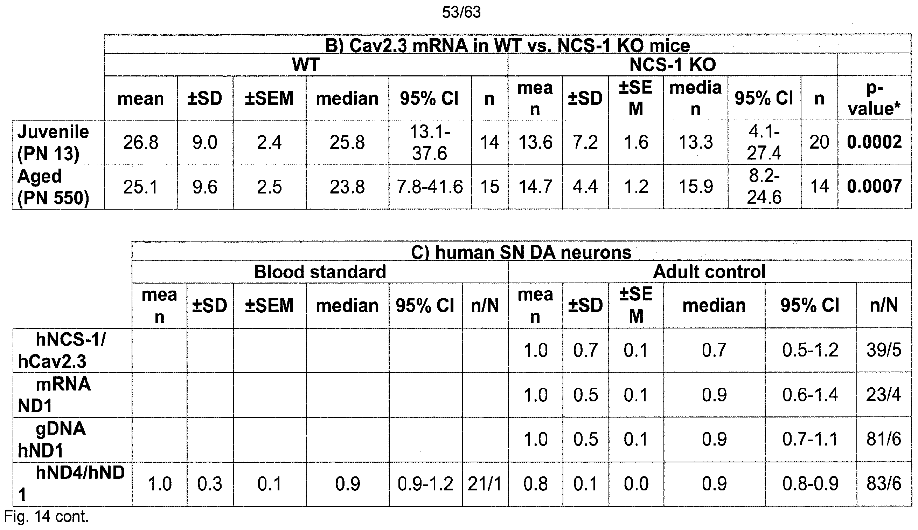

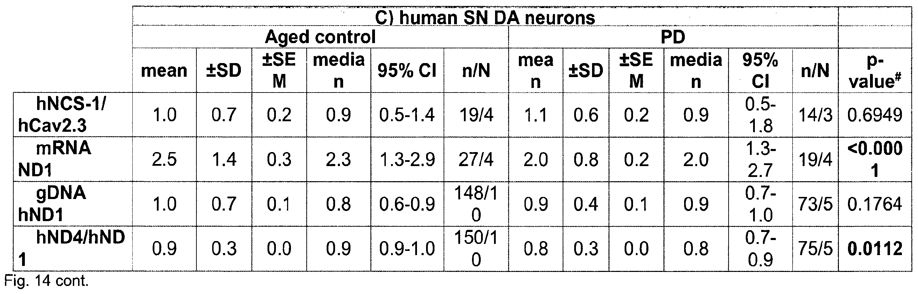

**Figure 4** shows cell-specific analysis of SN DA neurons in NCS-1 KO mice and human *post mortem* brains. **a,** UV-LMD & RT-qPCR of Cav2.3 in SN DA neurons from juvenile and aged (1.5 years) WT and NCS-1 KO mice. Error bars: SEM. **b, Left:** Tyrosine-hydroxylase stained midbrain sections from juvenile (PN13) WT and NCS-1 KO mice. **Right upper:** Stereological quantification of SN DA neuron numbers in WT and NCS-1 KO mice at four different ages. Error bars: SD. **Right lower:** Mean absolute numbers of counted SN DA neurons for all analyzed sections for all animals (at PN13). Error bars: SEM. **c, Pictures:** Neuromelanin-positive human SN DA neurons from *post mortem* brain-sections before and after UV-LMD. **From left to right:** RT-qPCR quantification of NCS-1/Cav2.3 mRNA ratios and NADH dehydrogenase ND1 mRNA (coded by mitochondrial DNA), as well as qPCR quantification of genomic DNA ND1-levles, and of genomic ND4/ND1 ratios as measure for mitochondrial DNA integrity (relative to fully intact mitochondrial DNA from fresh human blood samples) for human SN DA neurons from adult and aged healthy controls and PD patients brains. **d, Left:** Topological representation of *de novo* missense mutations (colored dots) identified in patients with NDD/ID in respect to location of missing exons (colored stars) in CACNA1E (coding for all Cav2.3 splicevariants a-e). **Right:** Combination of the exon use in the six known Cav2.3 splice variants, in bold detected variant in single SN DA neurons. All error bars: SEM. All data detailed in **Supplementary Tables S7B/C, S8A (Figs. 14**, 15).

**Figure 5** shows sensitized dopamine-autoinhibition in mature SN DA neurons is mediated by Cav2.3, $Ca^{2+}$ and NCS-1/D2-AR interaction. **a-i,** Experiments as in **Fig. 1.** Normalized frequencies plotted over time for all analyzed SN DA neurons. Dopamine (100 $\mu$M) bath-application as indicated. Application of EGTA (0.1 mM, whole cell), isradipine (300 nM), Z941 (10 $\mu$M), SNX-482 (100 nM), MVIIC (1$\mu$M) and mouse strain as indicated (WT, NCS-1 KO, Cav2.3 KO). **j,** Relative activity of SN DA neurons (from **Fig. E1a-i**) at the last minute in dopamine. **h,** UV-LMD & RT-qPCR results for Cav2.3, Cav1.2, Cav1.3, neuronal $Ca^{2+}$ sensor NCS-1, and for dopamine-autoreceptors (short and long splice variants D2s/D2l) in juvenile and adult mouse SN DA neurons. **Upper pictures:** juvenile mouse-brain-section before & after UV-LMD. Scale bars: 250 $\mu$m / 10 $\mu$m. **Lower pictures:** Striatal injection site of a retrogradely traced adult mouse-brain, aligned with mouse-brain atlas & labelled SN DA neuron. Scale bars: 10 $\mu$m. Data detailed in **Supplementary Tables S4, S5, S7A (Figs. 11, 12, 14).** Boxes: $25^{th}$ and $75^{th}$ percentiles, Whiskers: $10^{th}$ and $90^{th}$ percentiles.

**Figure 6** shows higher vulnerability of SN DA neurons in NCS-1 KO, and full and selective protection of SN DA neurons in Cav2.3 KO mice in a PD mouse-model (MPTP). **a/c, Left:** Optical density quantification of TH-signal in dorsal striatum (DS) and ventral sitriatum (VS) in saline and MPTP treated WT, NCS-1 KO and Cav2.3 KO mice. Error bars: SD. **Middle:** relative mean remaining TH-signals in DS and VS in MPTP treated animals, in respect to mean signals of respective saline treated animal groups, without and with bootstrapping analysis approach. Error bars: SD. **Right:** Mean absolute counted numbers of SN DA neurons for all analyzed sections for all animals. Error bars: SEM. **b/d, Left:** Stereological quantification of SN DA and VTA DA neurons in saline and MPTP treated WT, NCS-1 KO and Cav2.3 KO mice. Error bars: SD. **Middle:** relative mean remaining numbers of SN DA and VTA DA neurons in MPTP treated animals, in respect to mean signals of respective saline treated animal groups, without and with bootstrapping analysis approach. Error bars: SD. **Right:** Mean absolute counted numbers of VTA DA neurons for all analyzed sections for all animals. Error bars: SEM. All data detailed in **Supplementary Table S8B/C/D (Fig. 15).**

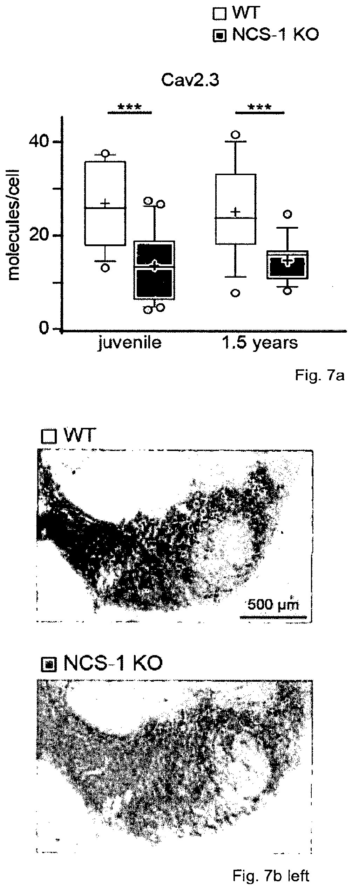

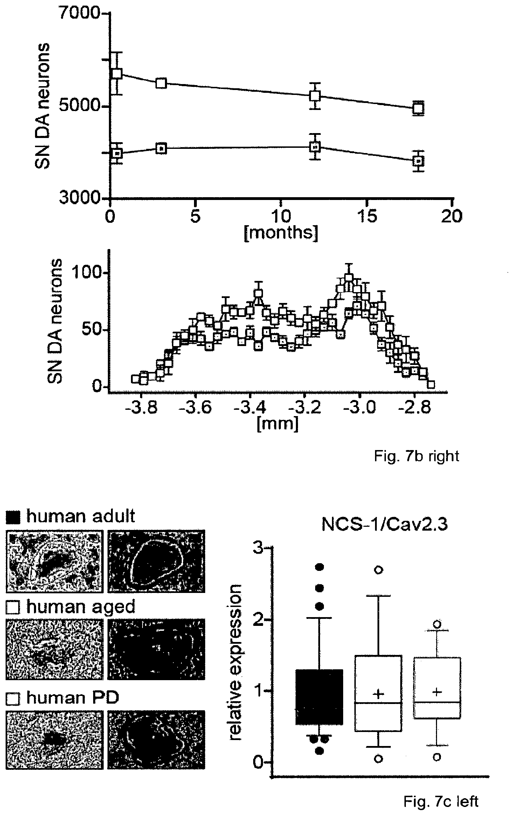

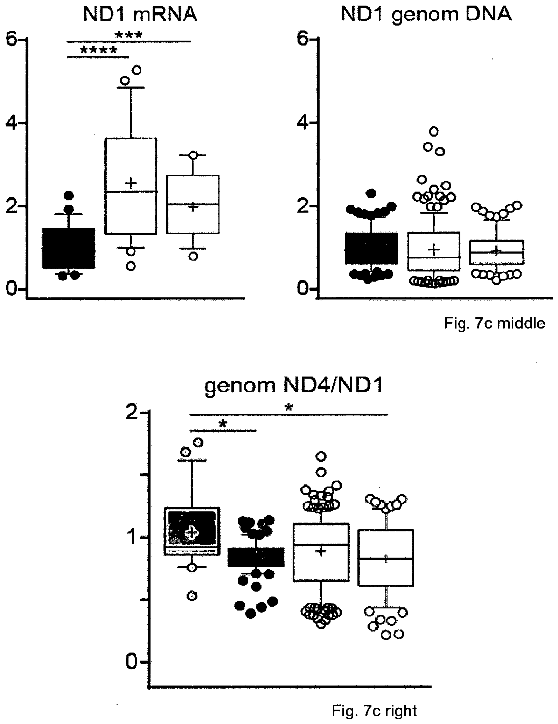

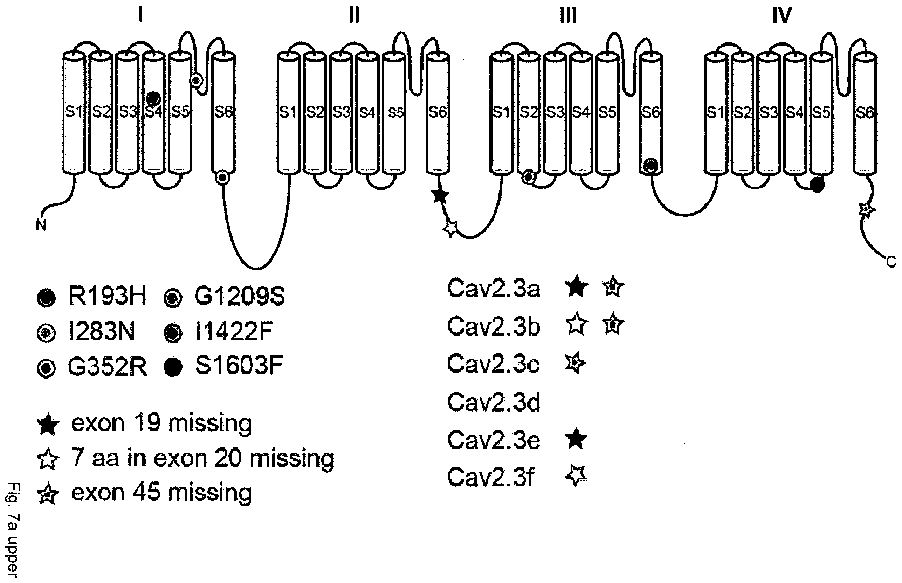

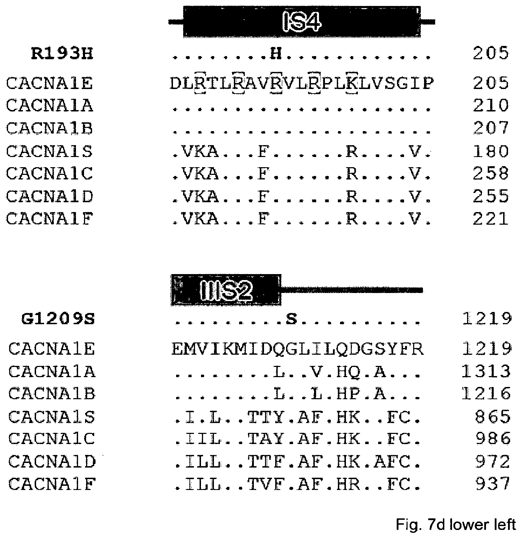

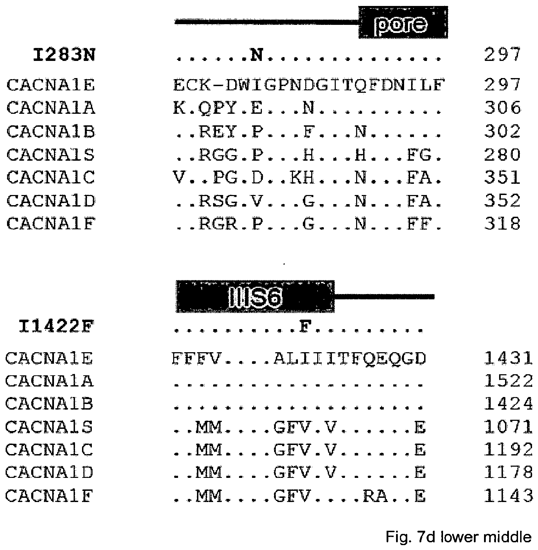

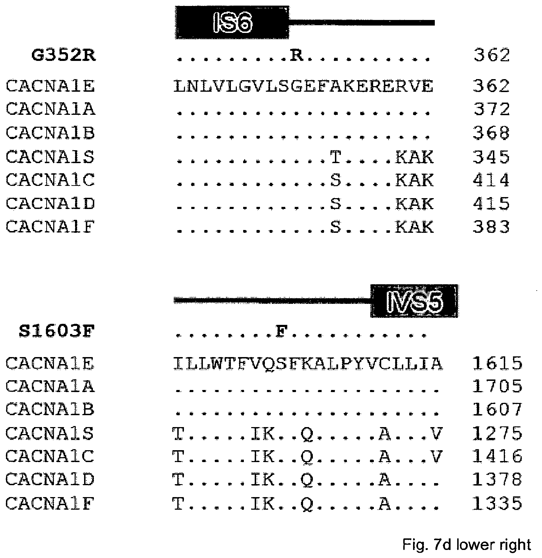

**Figure 7** shows cell-specific analysis of SN DA neurons in NCS-1 KO mice and human *post mortem* brains. **a,** UV-LMD & RT-qPCR of Cav2.3 in SN DA neurons from juvenile and aged (1.5 years) WT and NCS-1 KO mice. **b, Left:** Tyrosine-hydroxylase stained midbrain sections from juvenile (PN13) WT and NCS-1 KO mice. **Right upper:** Stereological quantification of SN DA neuron numbers in WT and NCS-1 KO mice at four different ages. **Right lower:** Mean absolute numbers of counted SN DA neurons for all analyzed sections for all animals (at PN13). **c, Pictures:** Neuromelanin-positive human SN DA neurons from *post mortem* brain-sections before and after UV-LMD. **From left to right:** RT-qPCR quantification of NCS-1/Cav2.3 mRNA ratios and NADH dehydrogenase ND1 mRNA (coded by mitochondrial DNA), as well as qPCR quantification of genomic DNA ND1-levles, and of genomic ND4/ND1 ratios as measure for mitochondrial DNA integrity (relative to fully intact mitochondrial DNA from fresh human blood samples) for human SN DA neurons from adult and aged healthy controls and PD patients brains. **d, Upper Left:** Topological representation of *de novo* missense mutations (colored dots) identified in patients with NDD/ID in respect to location of missing exons (colored stars) in CACNA1E (coding for all Cav2.3 splicevariants a-e). **Upper Right:** Combination of the exon use in the six known Cav2.3 splice variants, in bold detected variant in single SN DA neurons. **Lower:** Multiple protein sequence alignment shows the high conservation of all but one (I283N) mutated residues in Cav2.3 compared to other human Cav1/Cav2 proteins (identical amino acids shown as dots). Relative location of transmembrane domains or the pore region is depicted by blue boxes. The highly conserved positive residues of one of the S4 domains of CACNA1E involved in voltage sensing are shown with grey background (upper left alignment). Boxes: $25^{th}$ and $75^{th}$ percentiles, Whiskers: $10^{th}$ and $90^{th}$ percentile. All data detailed in **Supplementary Tables S7B/C, S8A (Figs. 14**, 15).

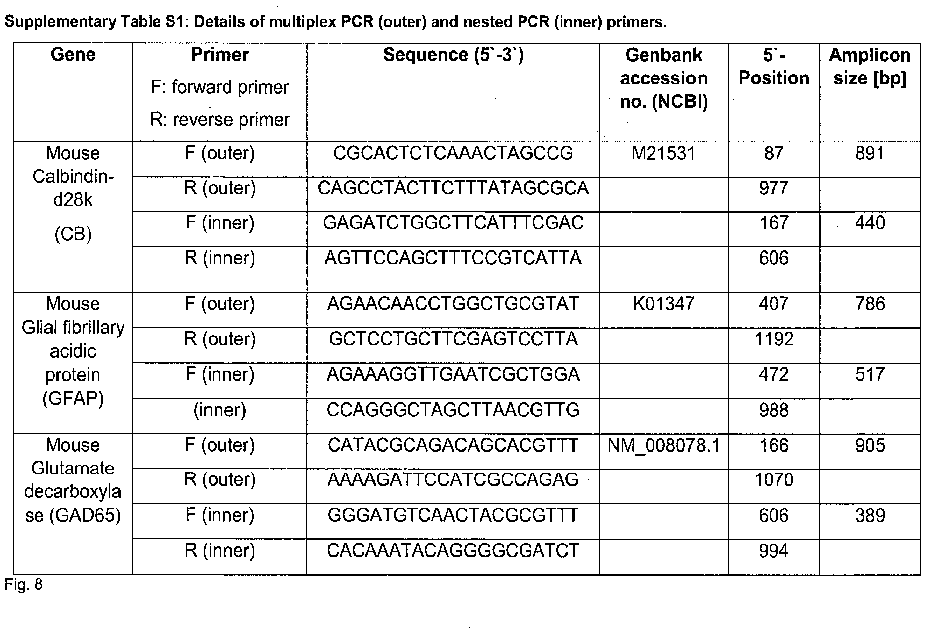

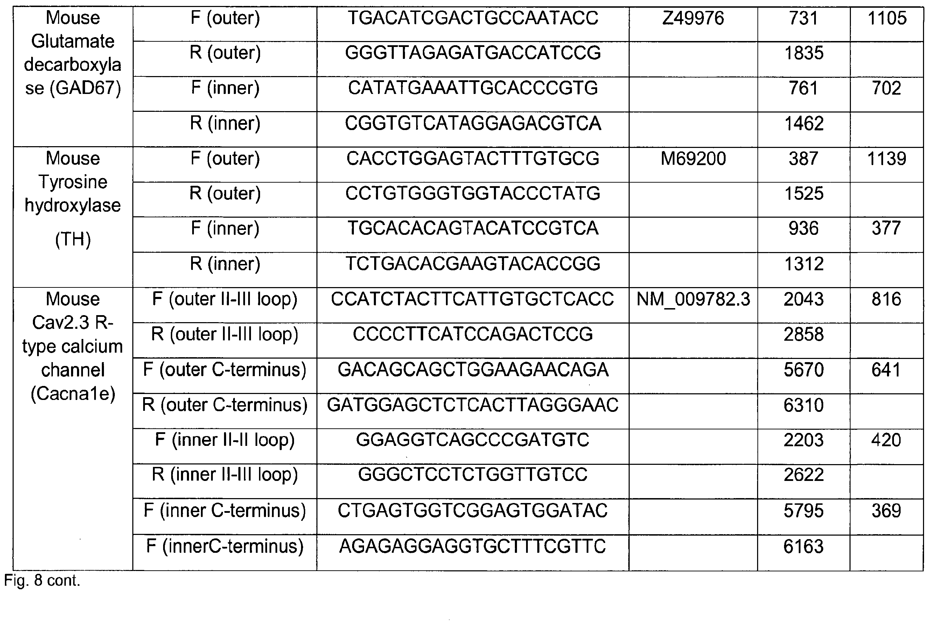

**Figure 8** shows **Supplementary Table S1** representing details of multiplex PCR (outer) and nested PCR (inner) primers.

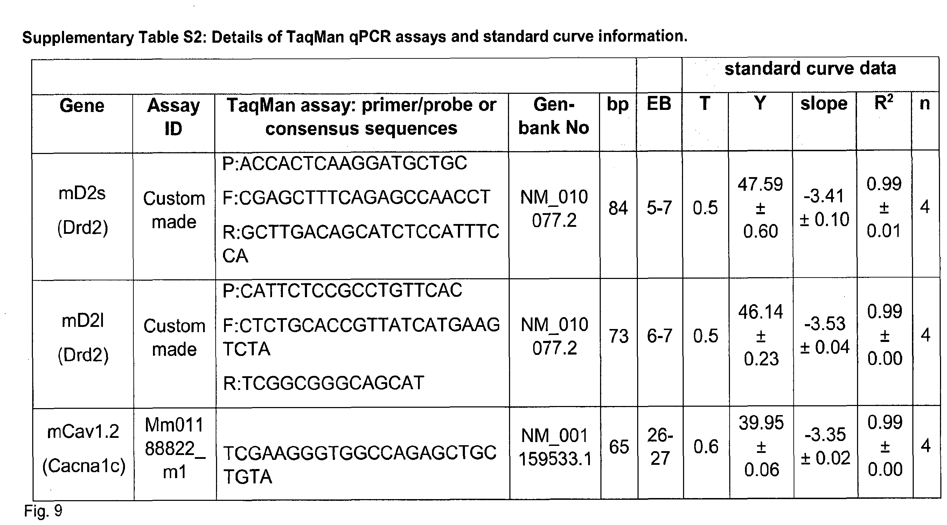

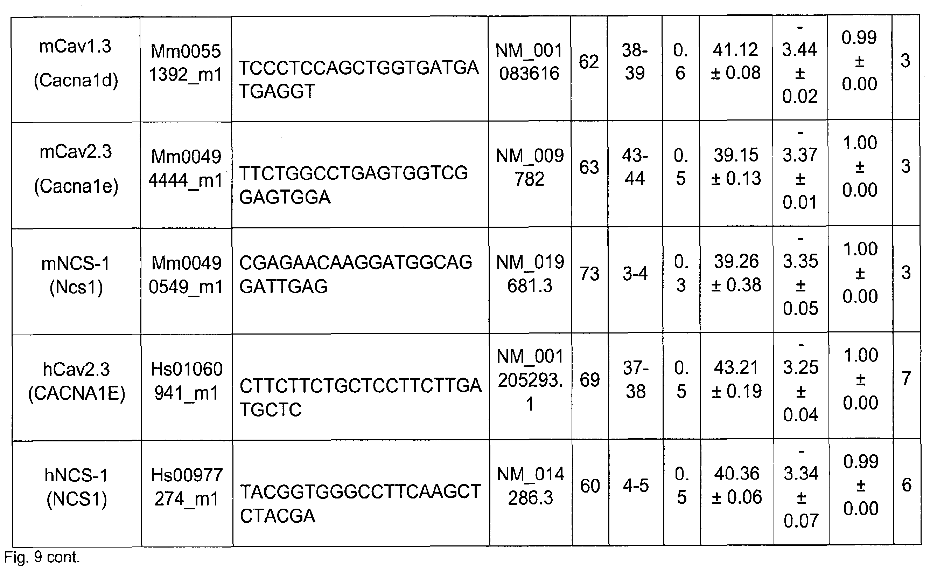

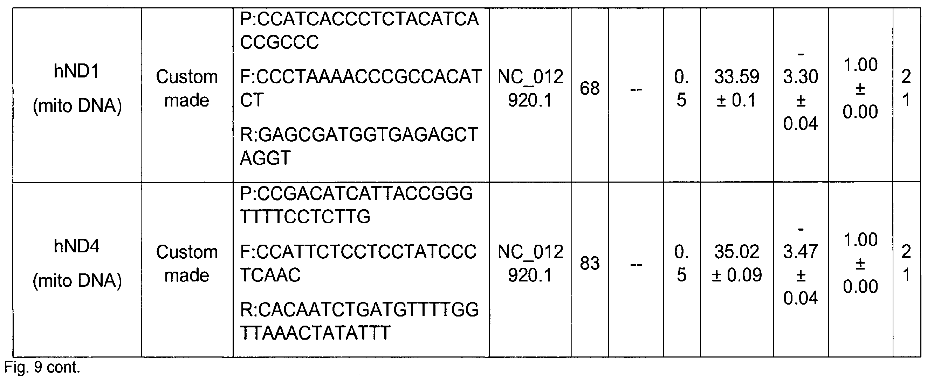

**Figure 9** shows **Supplementary Table S2** representing details of TaqMan qPCR assays and standard curve information. All probes are 5'-FAM (6-carboxyfluorescein) and 3'-NFQ (non-fluorescent quencher) labelled. P: probe, F: forward primer, R: reverse primer; m: mouse, h: human; bp: amplicon size; EB: exon-boundary spanned; T: threshold for analysis; Y: Y-intercept of standard curve. Y, slope and $R^2$ given as mean $\pm$ SEM.

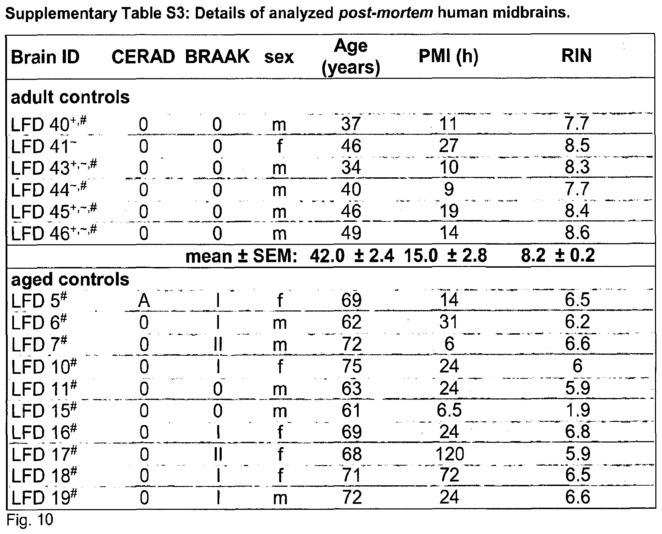

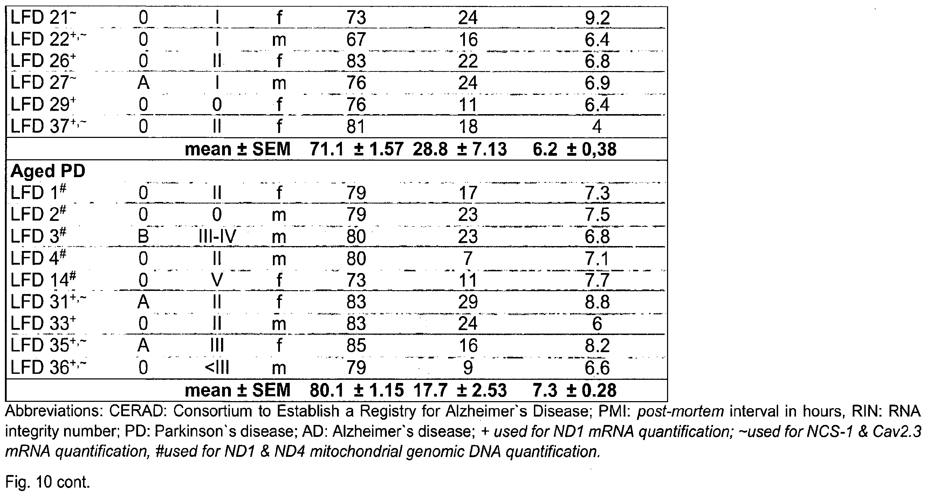

**Figure 10** shows **Supplementary Table S3** representing details of analyzed *post mortem* human midbrains. Abbreviations: CERAD: Consortium to Establish a Registry for Alzheimer's Disease; PMI: post mortem interval in hours, RIN: RNA integrity number; PD: Parkinson's disease; AD: Alzheimer's disease; + *used for ND1 mRNA quantification; ~used for NCS-1 & Cav2.3mRNA quantification, #used for ND1 & ND4 mitochondrial genomic DNA quantification.*

**Figure 11** shows **Supplementary Table S4** representing *in vitro* activity of SN DA neurons at last minute in dopamine. Data & statistics for graphs shown in **Fig. 1b-g** and **Fig. 5a-j** given as mean, $\pm$ SD, $\pm$SEM, median and 95% confidence interval (CI). Parameters were obtained from perforated/cell-attached recordings, if not marked by[&] for whole-cell recordings. n represents number of analyzed SN DA neurons. Significances according to Mann-Whitney U tests (MWU) (significant values in bold). *Data set partly from (Dragicevic et al., 2014, PMID: 24934288).*

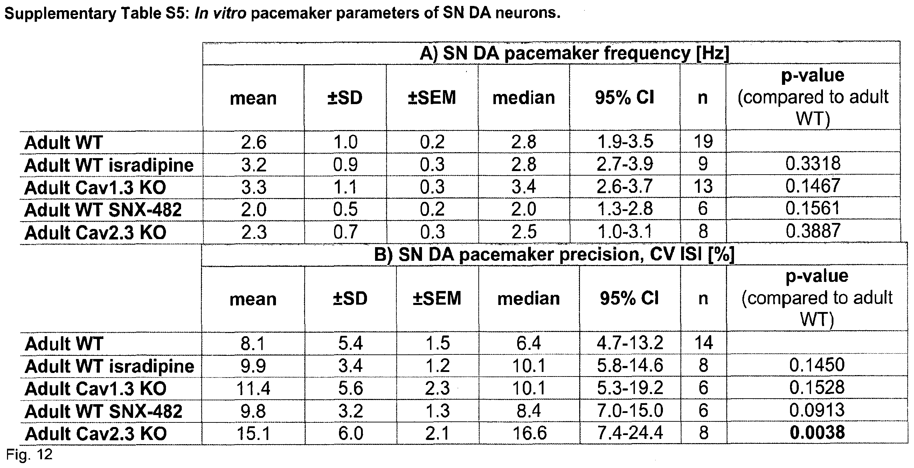

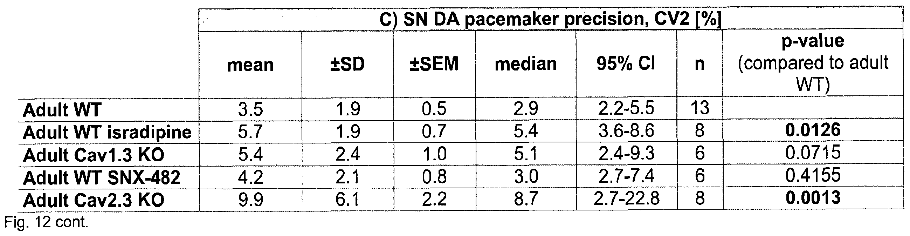

**Figure 12** shows **Supplementary Table S5** representing *in vitro* pacemaker parameters of SN DA neurons. Data given as mean, $\pm$SD, $\pm$SEM, median and 95% confidence interval (CI) for basal pacemaker frequency and pacemaker precision (given as coefficient of variation of the interspike interval, CV ISI, as well as CV2 values). n represents number of analyzed SN DA neurons. Significances according to MWU-test (significant values in bold).

**Figure 13** shows **Supplementary Table S6 A/B** representing Calcium imaging in SN and VTA DA neurons. Data & statistics for graphs shown in **Fig. 2a-c (A) and 2d (B)** given as mean, $\pm$SD, $\pm$SEM, median and 95% confidence interval (CI). n represents number of analyzed DA neurons. Significances according to *unpaired (A) and #paired (B)MWU (significant values in bold).

**Figure 14** shows **Supplementary Table S7 A/B/C** representing single cell qPCR data of mouse & human SN DA neurons.RT-qPCR data & statistics of genes as indicated for graphs shown in Fig 1h (A), **Fig. 4a** (B) and **Fig. 4c** (C) given as mean, $\pm$ SD, $\pm$SEM, median and 95% confidence interval (CI). n represents number of analyzed SN DA samples. Significances according to *MWU- or #Kruskal-Wallis-tests (significant values in bold). If Kruskal-Wallis test showed significant differences, individual p values of Dunn's multiple comparison test are given. For mRNA ND1: adult vs. aged, p = **<0.0001;** adult vs. PD, p = **0.0009;** aged vs. PD, p = >0.9999. For hND4/hND1: blood vs. adult, p = **0.0172;** blood vs. aged, p = 0.2999; blood vs. PD, p = **0.0314.**

**Figure 15** shows **Supplementary Table S8 A/B/C/D** representing Stereology and densiometry data.
Data & statistics for graphs shown in **Fig. 4b** (A), **Fig. 3 (B,C)** and Fig. 6 (D) given as mean, $\pm$ SD, $\pm$SEM, median and 95% confidence interval (CI). n represents number of analyzed mice (except for D). Significances according to *MWU- or #Kruskal-Wallis test with "uncorrected Dunn's test (significant values in bold). +T-test was only used for relative remaining fibers and neurons without bootstrapping analysis (see **Supplementary Table S8C).** Relative loss of TH-signals (%) in the dorsal (DS) and ventral striatum (VS) and relative loss of SN DA and VTA DA neurons were calculated in addition to normalization to the respective mean values of saline treated group (see **Supplementary Table S8C)** as bootstrapping approach (see **Supplementary Table S8D** and methods for details).

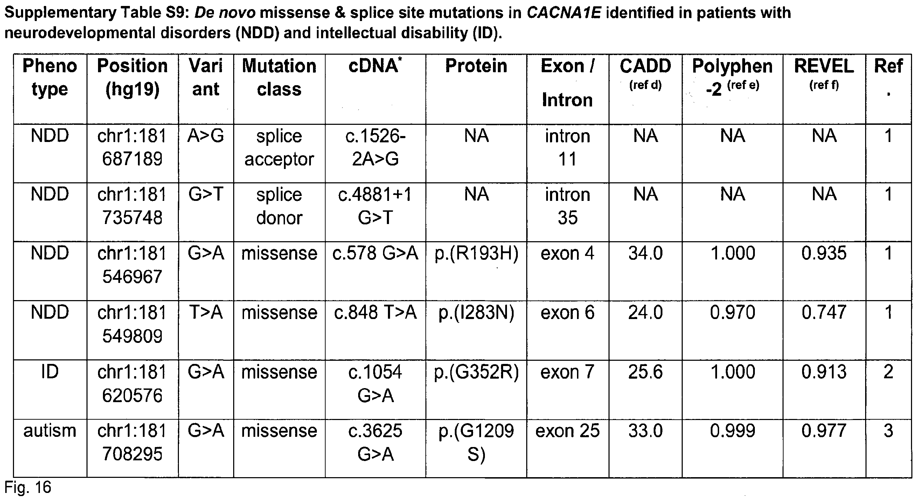

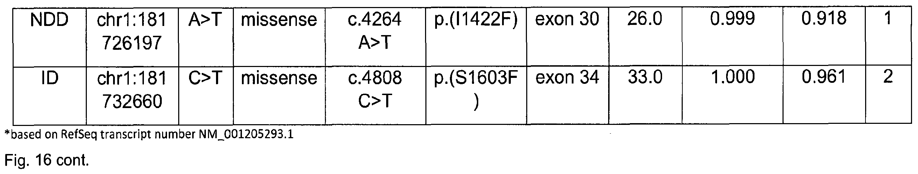

**Figure 16** shows **Supplementary Table S9** representing *de novo* missense & splice site mutations in CACNA1E identified in patients with neurodevelopmental disorders (NDD) and intellectual disability (ID).Two mutations alter the invariant splice sites of intron 11 and intron 35 predicted to severely interfere with splicing and probably leading to exon skipping. This is predicted to result in a consecutive frame-shift and premature stop codon in the first case and in an in-frame deletion of 22 amino acids (including 18 highly conserved residues of transmembrane domain IVS5) in the second case, so that both splice site alterations most probably represent LoF alleles. The six de novo mutations are missense alterations located in different parts of the Cav2.3 protein.

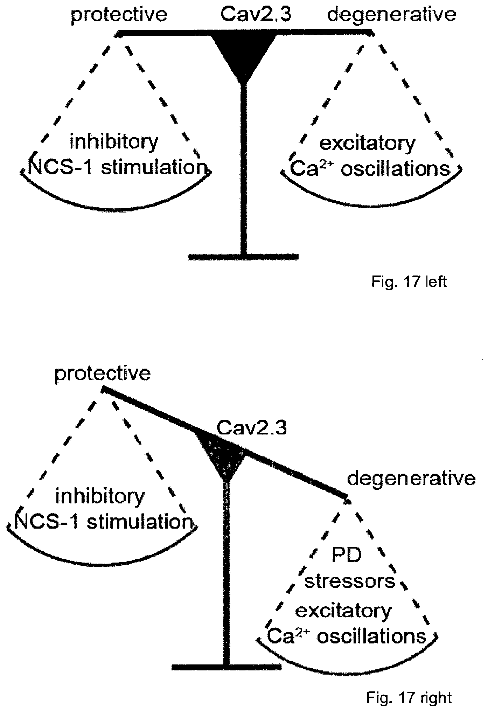

**Figure 17** illustrates as a conclusion of the invention the bidirectional functional roles of Cav2.3 in SN DA neurons that get out of balance due to additional neurodegerative triggers (PD-Stressors like aging, PARK-genes, environmental factors).

## Detailed Description of the Invention

According to the invention, it has been shown by combining brain-slice patch-clamp recordings with $Ca^{2+}$-imaging, pharmacological and genetic tools, as well as by cell-specific molecular and cell biological analysis of mouse and human SN DA neurons, that a type of VGCCs, the so-called Cav2.3 R-type $Ca^{2+}$ channels, *persistently stimulate* NCS-1 binding to D2-ARs in mature SN DA neurons from adult mice, and thereby constantly sensitize their inhibitory D2-AR dopamine autoreceptor responses **(Fig. 1, Fig.5).** In the presence of the Cav2.3 channel blocker SNX-482 and in a Cav2.3 knockout (KO) mouse, most adult SN DA neurons now displayed desensitized D2-AR responses, normally only found in those of younger mice **(Fig. 1e-g).** Buffering of internal Ca2+ (0.1 mM EGTA), using a peptide that blocks NCS-1 binding to D2-AR, and knockout of NCS-1 had similar effects on mature D2-AR responses than Cav2.3 inhibition or loss **(Fig. 1c,**g, **Fig. 5b,**f). In contrast, the LTCC blocker isradipine (300nM) P/Q-type (1 $\mu$M $\square$-conotoxin MVIIC) and T-type (10 $\mu$M Z941) VGCCs did not substantially alter sensitized dopamine-autoinhibition of SN DA neurons **(Fig. 1d,**g, **Fig.5e,**g). This confirms that the functional coupling of Cav2.3, NCS-1 and D2-AR signaling is crucial for maintaining constantly sensitized inhibitory responses to dopamine in mature SN DA neurons. Consistent with a prominent role of Cav2.3 channels in SN DA neurons, inventors cell-specific quantitative RT-qPCR results **(Fig. 1h, Fig 4d, Fig 5j-m)** show that Cav2.3 (more precisely the Cav2.3e splice variant) is the major VGCC isoform in SN DA neurons from adult mice; Cav2.3e mRNA levels (as well as those of D2-AR, short and long splice variants) increase with postnatal maturation, while those of Cav1.3 and Cav1.2 LTCCs decrease. This is in line with a respective age-dependent reduction of LTCC currents and the contribution of Cav1.3 for D2 AR sensitization in juvenile but not in adult SN DA neurons (*Branch et al, 2016 PMID: 27053209*). Sensitized D2-AR responses inhibit pacemaker activity (and associated metabolic stress) to a greater extent, and their Cav2.3 dependency points to a protective inhibitory feedback-loop, as Cav2.3 activity could also contribute to activity-dependent Ca2+ entry and hence to stressful activity, Ca2+ load, and a high metabolic burden in adult SN DA neurons.

To directly show that Cav2.3 significantly contributes to voltage-dependent somatic $Ca^{2+}$-dynamics during pacemaker activity (*Guzman et al., 2010, PMID: 21068725),* brain-slice electrophysiology were combined with ratiometric $Ca^{2+}$-imaging **(Fig. 2).** Indeed, *somatic* activity-related $Ca^{2+}$ oscillations in SN DA neurons were to a large extent mediated by Cav2.3. Somatic activity-dependent $Ca^{2+}$ oscillations were dramatically reduced in the cell-body / soma of SN DA neurons from adult Cav2.3 KO mice during spontaneous activity **(Fig. 2a-c),** while they were not significantly affected in the cell body of WT SN DA neurons in the presence of the LTCC-inhibitor isradipine(Ortner *et al., 2017, PMID: 28592699*). Moreover, in SN DA neurons from WT mice, in the presence of the Cav2.3-inhibitor SNX-482, action potential-induced somatic $Ca^{2+}$ transients were also significantly reduced **(Fig. 2d),** while more resistant mesolimbic VTA DA neurons from WT displayed almost no somatic $Ca^{2+}$ oscillations **(Fig. 2c,f. and Supplemetary table S6 / Figure 13).**

These data indicate bidirectional functions of Cav2.3 in SN DA neurons: (i) activity-dependent Ca2+ influx, potentially causing elevated metabolic stress as demonstrated for L-type channels (*Guzman et al., 2010, PMID: 21068725*), and (ii) feedback stimulation of inhibitory NCS-1/D2-AR activity, reducing activity-related metabolic stress, and likely protecting SN DA neurons from Ca2+ overload and degeneration *(Duda et al., 2016, PMID: 26865375).* This hypothesis predicts that disturbance of this delicate balance by reducing NCS-1-expression should increase the vulnerability of SN DA neurons to PD-stressors, while reduced Cav2.3-expression should have the opposite effect.

To confirm his hypothesis, the inventor applied a chronic neurotoxin PD-model (MPTP/probenecid) to NCS-1 KO, Cav2.3 KO, and WT mice, and compared pattern and degree of induced DA neuron loss in striatum and midbrain by tyrosine hydroxylase (TH) staining and stereology (Fig. 3 & E3). In line with a neuroprotective NCS-1 effect, the toxin-induced loss of SN DA neurons and their axonal projections in the dorsal striatum was about twice as high in the NCS-1 KO compared to WT, while no such differences in the VTA or the ventral striatum between WT and KO after MPTP were detected. **(Fig. 3a,**b, **Fig.6a**,b). In contrast, and coherent with a causal degenerative effect, SN DA neurons from Cav2.3 KO were fully rescued in this PD-model: no loss of SN DA neurons in Cav2.3 KO were detected, while WT animals displayed a selective SN DA loss of -50% **(Fig. 3c,**d, **Fig.6c**,d). TH-signals in the striatum confirmed a selective rescue of SN DA neuron-bodies and their axonal projections, as the remaining signal in the dorsal striatum was almost double as high in the Cav2.3 KO compared to WT.

These data suggest an emerging functional homeostatic antagonistic interplay of NCS-1 and Cav2.3. In line, Cav2.3 mRNA levels are significantly lower in young and aged SN DA neurons from NCS-1 KO mice **(Fig. 4a),** and NCS-1 KO mice displayed about 30% fewer SN DA neurons (with no additional loss upon aging like in WT), while numbers of VTA DA neurons were not different from WT **(Fig. 4b, Fig.7b, Tab. S8A).** In contrast, human SN DA neurons from healthy adult (~42 years) or aged individuals (-71 years), and from aged PD patients (-80 years) displayed similar NCS-1/Cav2.3 mRNA ratios **(Fig. 4c left, Fig 7c left).** However, indicative of elevated mitochondrial stress levels, human SN DA neurons from controls and PD patients displayed higher degrees of mitochondrial DNA deletions (defined by genomic DNA ratios of the mitochondrially coded NADH dehydrogenase subunits ND4/ND1, **(Fig. 4c right, Fig. 7c right),** similar to previous studies(Bender *et al., 2006, PMID: 16604074).* Also, mRNA levels of ND1 were elevated in human SN DA neurons from aged controls and PD patients, while genomic ND1 levels were unaltered **(Fig. 4c middle, Fig. 7c middle),** arguing for an upregulation of ND1 enzyme rather than for more mitochondria in these neurons.

Together, these data indicate that in response to increasing mitochondrial deletions and metabolic stress during age and PD, human SN DA neurons upregulate mitochondrial ND1 expression, seemingly to compensate for mitochondrial dysfunction, but they are unable to alter NCS-1/Cav2.3 expression ratios as another protective compensatory mechanism.

In Summary **(Fig 4d),** the inventor shows that Cav2.3 channels are active during pacemaker activity and substantially contribute to oscillatory elevated free $Ca^{2+}$ levels in the soma of mouse SN DA neurons that display a particularly high vulnerability to PD-stressors and preferentially degenerate in PD. The inventor demonstrates that Cav2.3 channel activity continuously stimulates inhibitory NCS-1/D2-AR signaling in mature SN DA neurons and that loss of NCS-1 activity further increases high vulnerability of SN DA neurons to degeneration. Activity-dependent $Ca^{2+}$ oscillations in SN DA neurons are causally linked to their metabolic stress levels and as a consequence to their increased susceptibility to PD stressors. However, only in distal dendrites but not in the cell-bodies of SN DA neurons these oscillations are sensitive to the L-type VGCC blocker isradipine (*Guzman etal., 2010, PMID: 21068725; Ortner et al., in press, PMID*), while low-threshold depolarizations in proximal dendrites are sensitive to T-type VGCC-inhibitors (*Evans et al., 2017, PMID: 28264982).*

Independent evidence for a vital role of Cav2.3 for the development and function of the human brain is provided by genomics. Whole exome sequencing of more than 60,700 individuals has found no loss-of-function (LoF) genetic variants; although, more than 80 such variants are expected when compared to the exome-wide level of genetic variation. Intolerance of LoF variation is enriched in genes linked to neurodevelopmental disorders (NDD). Large-scale sequencing studies have identified eight different CACNA1E (coding for Cav2.3) de novo missense or splice site mutations in patients with NDD, intellectual disability or autism. These mutations are located nearby or within the highly conserved transmembrane domains or the pore region of Cav2.3, and are predicted to be deleterious **(Fig. 7d & Tab. S9 / Fig. 16).** It will be interesting to see if CACNA1E variants are also associated with an altered susceptibility to PD.

The inventor shows that in the cell-bodies of SN DA neurons $Ca^{2+}$ oscillations are mediated prominently by Cav2.3 VGCCs, and evidence has been provided from mouse models und human *post mortem* brains for an antagonizing functional interplay of Cav2.3 channels and NCS-1 in SN DA neurons **(Fig 17):** the more NCS-1 activity the more stressful Cav2.3 activity (and to a lesser extent Cav1.3 VGCC activity) SN DA neurons might tolerate. Due to their VGCCs stimulated pacemaker-activity, and related $Ca^{2+}$-stimulation of ATP-generation and metabolic stress, SN DA neurons are energetically *"living on the edge"*(*Bolam and Pissadaki, 2012, PMID: 23008164; Duda etal., 2016, PMID: 26865375),* and additional PD-stressors (like PARK mutations, environmental factors or simply aging, all causing as an unifying event metabolic stress) further add to their delicate balance and could *"tip them over the edge"*, **Fig4d** *(Bolam and Pissadaki, 2012, PMID: 23008164; Duda et al., 2016, PMID: 26865375; Surmeier et al., 2017, PMID: 28104909).* This scenario could explain the high vulnerability of SN DA neurons and other neurons with inherent higher metabolic stress levels to PD-stressors, as suggested before (*Duda et al., 2016, PMID: 26865375; Surmeier et al., 2017, PMID: 28104909).* However, in contrast to current thinking, the experiments according to the invention strongly suggest that this $Ca^{2+}$ related stress in SN DA neurons is caused in large parts by Cav2.3 R-type VGCCs, rather than by L-type VGCCs.

Consequently, the inventor's findings are of particular importance with respect to therapeutic PD-concepts that focus on inhibition of Cav1.3 or Cav1.2 LTCCs as neuroprotective strategy, and the current phase III clinical trial testing. The data according to the invention would predict and could explain a possible inconclusive outcome of that trial, similar to the respective PD-mouse-model studies (*Ilijic et al., 2011, PMID: 21515375;Ortner et al., 2017, PMID: 2859269929).* Plasma-levels that are reached with the maximal tolerable dosis of 10 mg isradipine/day (*Parkinson Study, 2013, PMID: 24123224*) might not be sufficient to inhibit Cav1.3 (and Cav1.2) channels in SN DA neurons *2017, PMID: 28592699*), and inventors previous studies would predict that Cav1.3 inhibition might be protective only under conditions that lead to transient high dopamine levels, like dopamine-replacement therapy (e.g. with L-DOPA or rasagilin, a therapeutically relevant blocker of the monoamine-oxidase B) (*Dragicevic et al., 2014, PMID: 24934288; Poetschke et al., 2015, PMID: 26381090; Oertel and Schulz, 2016, PMID: 27577098).*

Most importantly, inventor's findings identify Cav2.3 VGCCs as attractive molecular targets to reduce the activity-dependent $Ca^{2+}$ load in SN DA neurons as neuroprotective strategy in PD-therapy. In line with the findings according to the invention, downregulation of Cav2.3 in response to a PD-stressor (human PARK1) in mouse cholinergic neurons of the dorsal motor nucleus of the vagus nerve (DMV) has been (*Goldberg et al., 2012, PMID: 22941107* DMV neurons display a similar stressful $Ca^{2+}$ based pacemaker than SN DA neurons, and they also display the typical protein-aggregate inclusions (Lewy bodies) present in PD, but for unknown reasons they are much less vulnerable to degeneration in PD than SN DA neurons(*Lasser-Katz etal., 2017, PMID: 28053029)* - but downregulation of Cav2.3 in response to PD stressors may offer an explanation.

At present no Cav2.3-selective brain permeable small molecule is available (*Schneider et al., 2013, PMID: 24276260; Zamponi et al., 2015, PMID: 26362469*) to test if pharmacological inhibition of these channels would exert a similar strong neuroprotective effect as we have observed in our PD mouse model for constitutive Ca2.3 deficiency. Based on the findings according to the invention, the discovery of such compounds should be pursued. Based on this invention, Crispr/Cas9 base gene-therapy approaches that reduce or knock down the expression of the Cav2.3 gene (i.e. CACNA1E that codes for all Cav2.3 splice-variants offer an alternative complementary, gene-therapy-based novel approach for neuroprotective therapy for PD and related neurodegenerative disorders. The inventor designed and successfully tested suitable Crispr/Cas9 constructs, and a protocol for its viral delivery into SN DA neurons of adult WT mice (Fig. 7e), as a proof-of-principle for a Crisp/Cas9 based neuroprotective gene-therapy approach that reduces Cav2.3 channel expression in highly vulnerable neurons, in particular SN DA neurons for PD.

As differential vulnerability of different neuronal population to neurodegenerative stressor is a common hallmark of all neurodegenerative diseases, and elevated metabolic stress and altered calcium homeostasis are not only interdependent factors, but seem also provide a crucial converging general downstream event in neurodegenerative disease and age-dependent neuron loss, this invention offers with Cav2.3 channels attractive targets for neuroprotective therapeutical intervention not only for PD and SN DA neurons but also for other neurodegenerative diseases with related pathomechanisms.

## EXAMPLES

The present invention is further illustrated by the following examples.

## Example 1: Animals and MPTP (1-Methyl-4-phenyl-1,2,3,6-tetrahydropyridin) treatment

Juvenile (-PN13), adult (-PN90) and aged (~PN360 and -PN550) male C57BL/6 and NCS-1 KO / wildtype (WT) mice (Ng, E. et al. Neuronal calcium sensor-1 deletion in the mouse decreases motivation and dopamine release in the nucleus accumbens. Behav Brain Res 301, 213-225, doi:10.1016/j.bbr.2015.12.037 (2016*)*) were bred in the animal facility of Ulm University. Cav2.3 KO mice and controls (Pereverzev, A. et al. Disturbances in glucose-tolerance, insulin-release, and stress-induced hyperglycemic upon disruption of the Ca(v)2.3 (alpha 1EJ subunit of voltage-gated Ca(2+) channels. Mol Endocrinol 16, 884-895, doi:10.1210/mend.16.4.0801 (2002*)*) were bread at the University of Cologne. MPTP injections and perfusions were carried out at the University of Göttingen. All animal procedures were approved by the German Regierungspräsidium Tübingen (Ref: 35/9185.81-3; TV-No. 921, 1043 and 1291, Reg. Nr. o.147) or the German Landesamt für Natur, Umwelt und Verbraucherschutz Nordrhein-Westfalen (Ref: 84-02.04.2016.A505), and carried out in accordance with the respective guidelines (for MPTP in particular see *Jackson-Lewis, Przedborski, 2007, PMID: 17401348)*. Mice were injected 10 times with MPTP hydrochloride (20 mg/kg in saline, subcutaneously, Sigma) and probenecid (250 mg/kg in PBS buffer, intraperitoneally, Thermo Fisher) for 5 weeks every 3.5 days. MPTP passes the blood-brain-barrier, and its active metabolite MPP+ is an inhibitor of complex I of the mitochondrial respiratory chain. MPP+ induces metabolic stress and selective loss of SN DA neurons but rather not of VTA DA neurons, and Parkinsonism into mice and men. Probenecid retards renal excretion of MPP+ and thus biological variations *(*Meredith, G. E. & Rademacher, D. J. MPTP mouse models of Parkinson's disease: an update. J Parkinsons Dis 1, 19-33, doi:10.3233/JPD-2011-11023 (2011*))*. Control mice were injected with saline and probenecid only. One week after the last injections mice were sacrificed, and perfused with PFA for immunohistochemistry.

## Example 2: Human brain tissue

Snap frozen *post-mortem* human midbrain blocks were obtained on dry ice from the German Brain Bank (BrainNet). All to available information on the respective donors and tissues are summarized in **Supplementary Table S3 (Fig. 10).** All experiments on human material were approved by the ethic commission of Ulm University (247/10-UBB/bal). Consent was obtained from all subjects by the German Brian Bank.

## Example 3: *In vivo* retrograde tracing

Retrograde tracing was performed essentially as described in Krabbe, S. et al. Increased dopamine D2 receptor activity in the striatum alters the firing pattern of dopamine neurons in the ventral tegmental area. Proc Natl Acad Sci U S A 112, E1498-1506, doi:10.1073/pnas.1500450112 (2015*)*. For retrograde labeling of nigrostriatal SN DA and mesolimbic VTA DA neurons of adult mice, red fluorescent latex retrobeads (Lumafluor) were injected unilaterally into the dorsal striatum (caudate putamen, CPu. Coordinates: Bregma (y-axis) 0.98 & -0.1 mm, lateral (x-axis) 1.9 & 2.70 mm, ventral (z-axis) -3.2 mm, 2x 60 nl) or bilaterally into the ventral striatum (Nucleus accumbens, NAc core and medial shell.
Coordinates: Bregma (y-axis) 1.6 mm, lateral (x-axis) 0.8 mm, ventral (z-axis,) -4.0 mm, 60 nl) of anaesthetized mice. Retrobead-injection was performed under stereotactic control (Kopf Instruments) with a NanoFil syringe attached to a micropump (UMP3 with SYS-Micro4 Controller; World Precision Instruments) at a rate of 50 nl/min for SN tracing and 30 nl/min for VTA tracing. Animals were sacrificed 7 days (CPu) or 14 days (NAc) after retrobead injection for calcium imaging (VTA DA) or UV-LMD and RT-qPCR analysis (SN DA neurons). Injection sites were verified according to the mouse brain atlas *(*Paxinos, G. & Franklin, K. The Mouse Brain in Stereotaxic Coordinates (Academic. New York). (2007*))* as described in *Krabbe S. et al.* (ref. as above).

## Example 4: *In vitro* electrophysiological recordings from juvenile and adult mouse brain slices

Coronal mouse brain slice preparations and *in vitro* electrophysiological whole cell, cell-attached, and perforated-patch clamp recordings from SN DA neurons on 200-250 $\mu$m mouse midbrain slices were carried out essentially as described (see Dragicevic, E. et al. Cav1.3 channels control D2-autoreceptor responses via NCS-1 in substantia nigra dopamine neurons. Brain 137, 2287-2302, doi:10.1093/brain/awu131 (2014*)*; Poetschke, C. et al., Compensatory T-type Ca2+ channel activity alters D2-autoreceptor responses of Substantia nigra dopamine neurons from Cavl.3 L-type Co2+ channel KO mice. Sci Rep 5, 13688, doi:10.1038/srep13688 (2015*))*. Briefly, SN DA neurons were identified according to their anatomical location, morphology, and typical electrophysiological fingerprint. Activity patterns were recorded in the presence of 10 $\mu$M DNQX (6,7-dinitroquinoxaline-2,3-dione, Tocris) and 10 $\mu$M gabazine (SR95531 hydrobromide, Tocris) for inhibition of fast synaptic transmission, in cell-attached or perforated patch (100 $\mu$g/ml gramicidine, Sigma) to maintain intracellular signaling (or whole-cell for 0.1 mM EGTA, Sigma). For dopamine auto-inhibition, after recording of a minimum of 5 min stable baseline activity, 100 $\mu$M dopamine hydrochloride (Sigma) in aCSF was applied via bath-perfusion for 15 min, followed by 20 min washout. Mean frequency of each minute was normalized to the respective baseline frequency for each cell. For pharmacology, slices were preincubated with 10 $\mu$M DNIP, a D2/NCS-1 interaction preventing peptide (*Dragicevic, E. et al.*see ref. above; Saab, B. J. et al. NCS-1 in the dentate gyrus promotes exploration, synaptic plasticity, and rapid acquisition of spatial memory. Neuron 63, 643-656, doi:10.1016/j.neuron.2009.08.014 (2009*))*, synthesized by Genscript, 860 Centennial Ave. Piscataway, NJ 08854, USA (http://www.genscript.com/index.html)., or 300 nM isradipine (Tocris) to block L-type Ca$^{2+}$ channels , or 10 $\mu$M Z941 (provided by Prof. T.P. Snutch, University of Brutish Columbia, 301 - 2185 East Mall, Vancouver, BC Canada V6T1Z4) to block T-type Ca$^{2+}$ channels (Tringham, E. et al. T-type calcium channel blockers that attenuate thalamic burst firing and suppress absence seizures. Sci Transl Med 4, 121ra119, doi:10.1126/scitranslmed.3003120 (2012*))*, or 1 $\mu$M $\omega$-conotoxin MVIIC (Tocris) to block N-, P/Q-type Ca$^{2+}$ channels, or 100 nM SNX-482 (Tocris) to block R-type Ca$^{2+}$ channels, bath applied

(in aCSF) for at least 30 minutes before and during recordings. Cells were repatched for whole cell recordings of basal biophysical properties for verification of SN DA identity (i.e. sag component/slow Ih-current, A-type current, broad action potentials).

**Example 5: Combined calcium Imaging and electrophysiology from adult mouse brain slices**

Experiments were performed in the same way as described previously (*Hess, M. E. et al., reference as above);* Konner, A. C. et al. Role for insulin signaling in catecholaminergic neurons in control of energy homeostasis. Cell metabolism 13, 720-728, doi:10.1016/j.cmet.2011.03.021 (2011*); Ortner et al., 2017 J. neurosci, in press).*SN DA neurons were identified as described above, and post hoc by immunohistochemistry for TH. Mesolimbic VTA DA neurons were identified by retrograde tracing, electrophysiological fingerprints, and post hoc by immunohistochemistry for TH. Only electrotypical Nucleus Accumbens core / medial shell VTA DA neurons43 were analyzed for this study. Perforated-patch clamp recordings were performed with pipette solution containing 1% biocytin (Sigma) and (in mM): 128 K-gluconate, 10 KCI, 10 HEPES, 2 MgCl2, and adjusted to pH 7.3 with KOH. Pipettes were tip filled with internal solution and backfilled with amphotericin B (-200 $\mu$g/ml in DMSO, freshly prepared; Sigma) containing solution. For Ca2+ imaging, 250 $\mu$M sulfinpyrazone (S9509, Sigma) was added to the aCSF. The imaging setup consisted of an Imago/SensiCam CCD camera with a 640x480 chip (Till Photonics) and a Polychromator IV (Till Photonics) coupled via an optical fiber into the upright microscope. Fura-2 was excited at 340 nm, 360 nm or 380 nm (410 nm dichroic mirror; DCLP410, Chroma). Emitted fluorescence was detected through a 440 nm long-pass filter. Data were acquired as 80x60 frames using 8x8 on-chip binning. Images were recorded in analog-to-digital units (ADUs) and stored and analyzed as 12-bit grayscale images. Before establishing the perforated-patch clamp recordings fura-2 was loaded into the neurons by electroporation (1 V with 1 ms pulse duration at 65 Hz for 10 - 15 s). The loading pipette contained intracellular saline and 3.6 mM fura-2 (pentapotassium salt, F1200, Life Technologies). Loading was monitored at 360 nm excitation to reach comparable loading states (128 $\pm$ 9 ADU, n = 45). To measure Ca2+ dynamics, pairs of frames excited with 340 nm and 380 nm were taken at 25 Hz (pacemaker activity) or 10 Hz (current induced action potentials, APs). The mean ADU value within a region of interest (ROI) from the soma was used. The ROI was adjusted for each cell. For 'background subtraction' for the whole-time series an adjacent, second ROI was chosen. Data were analyzed as normalized fura-2 F340/F380 ratios. Mean amplitudes of 20 oscillations were calculated for each analyzed neuron. Since AP-associated Ca2+ dynamics are strongly frequency dependent, the AP frequency was adjusted for Ca2+ imaging in all recorded neurons to a similar value of ~1.5 Hz. As SN DA neurons from (+/+) and C57BL/6 mice showed no significant difference, data were pooled. For pharmacology, 100 nM SNX-482 (Tocris) was bath applied (in aCSF) for at least 30 mins before and during recordings.. As SNX-482 besides Cav2.3 also inhibits voltage-sensitive A-type Kv4.3 K+ channels with high nanomolar affinity (Kimm, T. & Bean, B. P. Inhibition of A-type potassium current by the peptide toxin SNX-482. J Neurosci 34, 9182-9189 doi:10.1523/JNEUROSCI.0339-14.2014 (2014*)),* and as Kv4.3 channels are prominently expressed in SN DA neurons, and crucial for their pacemaker activity and vulnerability (Kimm, T. & Bean, B. P. Inhibition of A-type potassium current by the peptide toxin SNX-482. J Neurosci 34, 9182-9189, doi:10.1523/JNEUROSCI.0339-14.2014 (2014*)),* the inventor performed SNX-482 Ca2+ imaging experiments in the presence of the A-type channel blockers 4-aminopyridine (4 mM, Sigma). For SNX-482 experiments, neurons were hyperpolarized to ~ -70 mV and depolarizing currents (50 - 70 ms, 50 - 80 pA) were injected to induce two action potentials. Biocytin-streptavidin labeling combined with TH immunohistochemistry for confirmation of DA phenotype was performed as described (Hess, M. E. et al. The fat mass and obesity associated gene (Fto) regulates activity of the dopaminergic midbrain circuitry. Not Neurosci 16, 1042-1048, doi:10.1038/nn.3449 (2013*)).*

**Example 6: Cryo-sectioning, UV laser microdissection and RT qPCR analysis**

Coronal (mice) and horizontal (human) 12 $\mu$m cryosections were cut and mounted on PEN-membrane slides (Mirodissect) essentially as previously described (see *Krabbe S. et al.* (ref. as above); see also Schlaudraff, F. et al. Orchestrated increase of dopamine and PARK mRNAs but not miR-133b in dopamine neurons in Parkinson's disease. Neurobiol Aging 35, 2302-2315, doi:10.10161j.neurobiologing.2014.03.016 (2014*)).* UV-Laser-microdissection (UV-LMD) of SN DA neurons from cresyl-violet-stained midbrain sections from juvenile mice and human tissue, or from retrogradely traced adult mouse midbrain sections, was carried out using an LMD7000 system (Leica Microsystems). UV-LMD, cell lysis, cDNA synthesis, precipitation, multiplex nested PCR (for marker-gene analysis of murine samples) and quantitative real-time PCR (qPCR) of UV-LMD samples were performed as described (see *Krabbe S. et al.* (ref. as above); *Schlaudraff, F. et al.* (ref. as above); Duda, J., Fouler, M., Grundemann, J. & Liss, B. Cell-specific RNA quantification in human SN DA neurons from heterogeneous post mortem midbrain samples by UV-laser microdissection and RT-qPCR. Methods Mol Biol (in press*)).* For human samples, only neuromelanin positive neurons were collected, and only tyrosine hydroxylase (TH) positive UV-LMD samples were further analyzed. For mouse-derived samples, only samples expressing a correct marker gene profile were used for qPCR analysis: positive for TH, and negative for calbindin d28k (CB), glial fibrillary acidic protein (GFAP), and I-glutamate decarboxylase ($GAD_{65/67}$). Details of all primers and qPCR assays (TaqMan), and respective standard curve parameters (derived from mouse or human cDNA from respective midbrain tissues) used for RT-qPCR analysis are provided in **Supplementary Table S1 & S2 (Fig. 8, Fig.9).**

For absolute quantification of Cav1.2, Cav1.3, Cav2.3, and NCS-1 cDNA molecule numbers, defined amounts of DNA molecules were utilized for generating standard curves, essentially as described before (Liss, B. et al. Tuning pacemaker frequency of individual dopaminergic neurons by Kv4.3L and KChip3.1 transcription. EMBO J 20, 5715-5724, doi:10.1093/emboj/20.20.5715 (2001); Ortner, N. J. et al. Lower affinity of isradipinefor L-type Ca2+ channels during Substantia nigra dopamine neuron-like activity: implications for neuroprotection in Parkinson's disease. J Neurosci (in revision)). For amplification of cDNA fragments covering the TaqMan® assay locations, the following primers were used: mCav1.2 forward: TCACCACTCTGCTGCAGTTC, reverse: GACGAAACCCACGAAGATGT (amplicon size: 392 bp); mCav1.3 forward: TCGGGACTGGTCTATTCTGG, reverse: TACTTCCCCACCAGTCCTTG (480 bp); mCav2.3 forward: TTGGATCTGCTTGTGCCCATG, mCav2.3 reverse: GGAATTTGAACGCTTATCCCGAA (amplicon size: 543 bp or 414 bp depending on splice variant expression); mNCS-1 forward: CCGAGCATGGGGAAATCCAAC, mNCS-1 reverse: TCATGGCAAAGATCCGGTCCAC (472 bp). After gel electrophoresis, PCR products were purified (QIAquick Gel Extraction Kit), and quantified (with a Qubit 3.0 fluorometer). DNA standards containing 1.000.000 molecules down to 1 molecule (in 10-fold diluted steps) were used to generate absolute cDNA standard curves.

The relative cDNA amount in respect to a midbrain tissue-derived cDNA standard curve (for human assay and murine D2s and D2l assay) and the number of cDNA molecules (for murine Cav1.2, Cav1.3, Cav2.3 and NCS-1 data) per cell were calculated according to *Duda, J. et al.* reference as above; Grundemann, J., Schlaudraff, F. & Liss, B. UV-laser microdissection and mRNA expression analysis of individual neurons from postmortem Parkinson's disease brains. Methods Mol Biol 755, 363-374, doi:10.10071978-1-61779-163-5_30 (2011):

$$cDNA\ amount/molecules\ per\ cell = \frac{S^{[(Ct-Y_{intercept})/slope]}}{No_{cells} \cdot cDNA\ fraction}$$

With S = serial dilution factor of the standard curve (i.e. 10), $No_{cells}$ = number of harvested SN DA neurons per sample (i.e. 10 for mouse and 20 for human cDNA samples or 1 for human genomic DNA samples, respectively), cDNA fraction = fraction of the UV LMD cDNA reaction used as template in the individual qPCR reactions (i.e. 5/17 for all mouse genes, 1/9 for human Cav2.3, 2/33 for human NCS-1 and human ND1 cDNA and 1/12 for human genomic ND1/ND4) and th Y-intercept of the absolute standards, or relative standards (). Mouse SN DA neuron expression levels were normalized to equal 1, for juveniles in comparisons to adults, and to WTs for KO comparisons. Human SN DA neuron expression levels were normalized to 1 for adults in mRNA and gDNA comparisons, and to that of undegraded blood levels for ND4/ND1 ratios.

Mitochondrial genomic DNA was isolated from human SN DA neurons after UV-LMD with the QiaAmp DNA Micro Kit (Qiagen) as previously described (Muhling, T., Duda, J., Weishaupt, J. H., Ludolph, A. C. & Liss, B. Elevated mRNA-levels of distinct mitochondrial and plasma membrane Co(2+) transporters in individual hypoglossal motor neurons of endstage SOD1 transgenic mice. Front Cell Neurosci 8, 353, doi:10.3389/fncel.2014.00353 (2014)). DNA was eluted in 30 μl molecular biology grade water and stored at 4°C until qPCR. For mitochondrial DNA integrity analysis the human ND4 gene copy number was determined, which is deleted in 96% of all deletion events, as well as the human ND1 gene copy number which is almost never deleted in humans. The respective relative ND4/ND1 ratio (between 1 and 0) indicates the percentage of fully intact mitochondrial genomes. (Bender, A. et al. High levels of mitochondrial DNA deletions in substantia nigra neurons in aging and Parkinson disease. Nat Genet 38, 515-517, doi:10.1038/ng1769 (2006); He, L. et al. Detection and quantification of mitochondrial DNA deletions in individual cells by real-time PCR. Nucleic Acids Res 30, e68 (2002); Krishnan, K. J., Bender, A., Taylor, R. W. & Turnbull, D. M. A multiplex real-time PCR method to detect and quantify mitochondrial DNA deletions in individual cells. Anal Biochem 370, 127-129, doi:10.1016/j.ab.2007.06.024 (2007)). ND4/ND1 ratios derived from freshly isolated DNA from human blood samples were used as relative standard for fully intact mitochondrial DNA.

For Cav2.3 splice variant analysis, two cDNA fragments (mCav2.3 II-III loop and mCav2.3 C-terminus) covering the three respective splice sites were amplified in a duplex PCR, followed by two individual nested PCR reactions. PCR conditions: 15 min, 95°C; 35 cycles: 30 s 94°C; 1 min 61°C; 3 min 72°C; 7 min,72°C for duplex PCR. 3 min 94°C; 35 cycles: 30 s 94°C; 1 min 61°C; 1 min 72°C; 7 min,72°C for nested PCRs. Primer sequences are given in Table S1. Outer duplex primer pairs were chosen using Oligo7.60 software (possible PCR amplicon sizes mCav2.3 II-III loop: 816 bp, 795 bp and 759 bp; mCav2.3 C-terminus: 770 bp and 641 bp). For the two individual nested PCRs, primer sequences from23 were used (possible PCR amplicon sizes mCav2.3 II-III loop: 420 bp, 399 bp and 363 bp; mCav2.3 C-terminus: 498 bp and 369 bp). Nested PCR products were separated in a 4% agarose gel (MetaPhor agarose, Biozym), and expressed splice variants (mouse whole brain tissue as positive control, and in individual SN DA neurons) were identified according to the amplicon sizes of the nested PCR products:

|  | II-III loop nested PCR fragment | C-terminus nested PCR fragment |
|---|---|---|
| Cav2.3a | 363 bp | 369 bp |
| Cav2.3b | 399 bp | 369 bp |
| Cav2.3c | 420 bp | 369 bp |
| Cav2.3d | 420 bp | 498 bp |
| Cav2.3e | 363 bp | 498 bp |
| Cav2.3f | 399 bp | 498 bp |

| Mouse Cav2.3 R-type calcium channel (Cacna1e) | F (outer II-III loop) | CCATCTACTTCATTGTGCTCACC | NM_009782.3 | 2043 | 816 |
|---|---|---|---|---|---|
|  | R (outer II-III loop) | CCCCTTCATCCAGACTCCG |  | 2858 |  |
|  | F (outer C-terminus) | GACAGCAGCTGGAAGAACAGA |  | 5670 | 641 |
|  | R (outer C-terminus) | GATGGAGCTCTCACTTAGGGAAC |  | 6310 |  |
|  | F (inner II-II loop) | GGAGGTCAGCCCGATGTC |  | 2203 | 420 |
|  | R (inner II-III loop) | GGGCTCCTCTGGTTGTCC |  | 2622 |  |
|  | F (inner C-terminus) | CTGAGTGGTCGGAGTGGATAC |  | 5795 | 369 |
|  | F (inner C-terminus) | AGAGAGGAGGTGCTTTCGTTC |  | 6163 |  |

Source of the splice variants as used: Schneider, Dubue, Hescheler: How "Pharmacoresistant" is Cav2.3, the Major Component of Voltage-Gated R-type Ca2+ Channels? Pharmaceuticals (Basel). 2013;6(6):759-76. doi: 10.3390/ph6060759.

**Example 7: Immunohistochemistry**

Immunohistochemistry was essentially performed as described in Liss, B. et al. K-ATP channels promote the differential degeneration of dopaminergic midbrain neurons. Nat Neurosci 8, 1742-1751, doi:10.1038/nn1570 (2005). After post-fixation of the brains in PFA at 4°C, they were stored in 0.05% $NaN_3$ in PBS at 4 °C. Before cutting, brains were incubated for 1 h in cutting solution (10% sucrose and 0.05% $NaN_3$ in PBS) at 4°C. 30 $\mu$m coronal midbrain sections for stereological analysis and 100 $\mu$m coronal striatal sections for striatal fiber analysis were cut with a vibratome (VT 1000S, Leica). Free floating slices were washed three times in PBS for 10 min each with shaking (300 rpm, microplate shaker, VWR) and treated for 2 h with a blocking solution (10% normal goat serum, 0.2% BSA and 0.5% Triton X-100 in PBS). After washing the slices with PBS, rabbit anti-TH (1:5000, Merck Millipore) was applied in a carrier solution (1% goat serum, 0.2% BSA and 0.5% Triton X-100 in PBS) and incubated overnight at room temperature while shaking (300 rpm, microplate shaker, VWR). Slices were washed three times in 0.2% Triton X-100 in PBS for 10 min and incubated with the secondary antibody (goat anti-rabbit biotinylated, 1:1000, Vector Laboratories) for 2 h at room temperature while shaking (300 rpm, microplate shaker, VWR) in the carrier solution as described above. TH staining was visualized using a DAB kit (Vector Laboratories). The slices were mounted on glas-slides, dehydrated, and coverslipped for further stereological and optical density analysis.

## Example 8: Optical densiometry and stereological analysis

Optical densiometry and stereological analysis essentially as described in *Liss, B. et al.* (ref. as above). Regions of interests (dorsal striatum, ventral striatum, SN and VTA) were identified according to established anatomical landmarks (Paxinos mouse brain atlas [Paxinos, G. & Franklin, K. The Mouse Brain in Stereotaxic Coordinates (Academic. New York). (2007*)*]), and data were adjusted for systematic differences in TH-signal intensities in samples that were not processed in parallel. Relative optical densities of striatal TH-derived DAB-immunoreactivity signal was determined using digital imaging software (imaged). Images were acquired with the LMD Software 8.1.0.6156 (Leica). Striatal TH-DAB signal was quantified in 18 dorsal and 10 ventral serial striatal sections, covering the whole caudorostral axis (bregma -0.3 to 1.7 for dorsal striatum and bregma 0.6 to 1.7 for ventral striatum). The respective signals from cortices were subtracted as background.

Total numbers of TH$^+$ neurons in SN and VTA were determined with high resolution stereology, using an unbiased optical fractionator method (Stereo Investigator software; MBF Bioscience). 40 serial TH-stained sections were analyzed, covering the whole caudorostral axis (bregma - 3.8 to -2.7) of the murine midbrain (each section for SN, every second section for VTA). Estimated total number of TH$^+$ neurons (N) was calculated for each animal according to equation (1):

$$(1) \quad N = \sum Q^- \cdot \frac{t}{h \cdot asf \cdot ssf}$$

with $\Sigma Q^-$ = number of counted neurons, t = mean section thickness, h = counting frame height (i.e. 11 $\mu$m), asf = area sampling fraction (i.e. 0.44), and ssf = section sampling fraction (i.e. 1 for SN and 2 for VTA). Sampling grid dimensions were 75 $\times$ 75 $\mu$m (x,y-axes) and counting frame size were 50 $\times$ 50 $\mu$m (x,y-axes). Reliability of the estimation was evaluated by the Gundersen coefficient (CE, m = 1) according to equation (2):

$$(2) \quad CE = \frac{\sqrt{s^2 + VAR_{SRS}}}{s^2}$$

$$(3) \quad VAR_{SRS} = \frac{3(A - s^2) - 4B + C}{240}$$

for m=1 with $A = \sum_{i=1}^{n}(Q_i^-)^2$; $B = \sum_{i=1}^{n-1} Q_i^- Q_{i+1}^-$; $C = \sum_{i=1}^{n-2} Q_i^- Q_{i+2}^-$ with s$^2$ = variance due to noise and VAR$_{SRS}$ = variance due to systematic random sampling, according to equation (3) for m = 1.

CE values were all $\leq$ 0.05 for all analyzed animals. Caudorostral axes were generated by plotting the mean relative optical densities, or the mean absolute counted number of neurons for each analyzed section for each animal.

## Example 9: Multiple Sequence Alignement and large-scale exome sequencing data analysis

Multiple protein sequence alignment was performed by Clustal Omega () using default parameters. The following human RefSeq sequences were used for the alignment: CACNA1E/Cav2.3 (NP_001192222), CACNA1A/Cav2.1 (NP_075461), CACNA1B/Cav2.2 (NP_000709), CACNA1S/Cav1.1 (NP_000060), CACNA1C/Cav1.2 (NP_955630), CACNA1D/Cav1.3 (NP_000711), and CACNA1F/Cav1.4 (NP_005174). The "probability of being intolerant to loss-of-function (LoF) mutation"-score (pLI-score) (*Lek et al., 2016: PMID: 27535533)* was assessed at the ExAC data base (http://exac.broadinstitute.org/). None of the described eight de novo mutations in CACNA1E/Cav2.3 in patients with NDD, ID or autism (given in Table S9 and Fig 7d) is present in over 80,000 control alleles, and all of them are predicted to be deleterious by the independent prediction programs CADD (*Kircher et al., 2014: PMID: 24487276),* Polyphen-2 (*Adzhubei et al., 2010, PMID: 20354512*) and REVEL (*Ionnidis et al., 2016, PMID: 27666373).* A full list of Exome Aggregation Consortium contributing groups can be found at http://exac.broadinstitute.org/about.

## Example 10: Crispr/Cas9 mediated knock-down of Cav2.3 channels via viral delivery to SN DA neurons.

We synthesized adeno-associated virus 2 (AAV2)-constructs containing two independent expression cassettes: C-terminally AU1-tagged Staphylococcus aureus CRISPR associated protein 9 (SaCas9) (*Ran et al., 2015, PMID:*

*25830891*) driven by the neuron-specific human synapsin 1 gene (hSyn)-promoter, as well as the guide RNA sequence (gRNA, T2 or T3) followed by the SaCas9-specific scaffold sequence and driven by the U6-promoter. The construct was designed for knock-down via non-homologous end-joining (NHEJ). Targeted sequences, situated in exon 1 of Cacna1e, were:

CCTTGTCGGTTCCTGCTCTGGT**CCGAGT** (T2), and
CCGCCGGCCCCGAGGCCGGGAC**GGGGGT** (T3), whereas the underlined sequences in bold demarcate the Protospacer Adjacent Motifs (PAM) and the non-underlined sequences are reverse complementary to the target Cacna1e and correspond to the gRNA. Constructs were synthesized and amplified by GenScript (NJ, USA) and packaged into AAV2 capsids by Vigene Biosciences (MD, USA). Titers were 2.7 (T2) or 4.2 (T3) * 1013 IU/ml, respectively, and suspensions were injected either undiluted or diluted to an approximate titer of $5 \times 1012$ IU/ml in sterile saline. For adult mouse SN, at anteroposterior (AP) -3.2 and mediolateral (ML) 1.0 relative to bregma, 900nl each were injected at 4.2 and 4.0 mm from pia. For combined hippocampal / neocortical transfections (as controls), at AP -2.0 and ML 1.5 relative to bregma, 500nl each were injected at 1.3 and 0.8 mm from pia (similar procedure as in Example 3). Animals were sacrificed 3-5 weeks after surgery, and expression of Cas9 was confirmed by immunohistochemical staining (as in Example 7) against AU1 (MMS-130R, Covance, diluted 1:1000). The successful genome edit is validated using the Surveyor Mutation Detection Kit (IDT).

**Example 11: Data analysis**

Data analysis and graphical illustrations were performed using FitMaster software (HEKA Electronics), Graph-Pad Prism 7 (GraphPad Software, Inc.), Adobe Illustrator CC2015.3 (Adobe Systems Software Ireland Limited), Igor Pro 6 (Wavemetrics Inc.), Spike2 (CED), Neuroexplorer (Nex Technologies), SDS 2.4 (Applied Biosystems), Stereo Investigator (MBF Bioscience), and ImageJ (NIH) softwares.

For normalization of MPTP data, as there are no individual controls for the individual MPTP treated mice, % of remaining TH-positive fibers / neurons in the MPTP treated groups were calculated in respect to the mean of the corresponding saline treated group (particularly important for NCS-1 KO, as KO controls had lower numbers of SN DA neurons than WT, compare **Fig. 2e)** and given in **Fig. 3** and **Supplementary Table S8C, Fig. 15.** In an alternative bootstrapping approach, each individual MPTP value was compared to each individual saline value of the respective groups for calculation of % of remaining TH-signal intensity and neuron numbers (see **Fig.6,** and **Supplementary Table S8D, Fig. 15**).

Sample sizes for all mouse-derived data were chosen in agreement with the Biometrics department, and as approved by the German "Regierungspräsidium". Sample size for human *post mortem* data was limited by the respective tissue that the inventor could obtain from the German Brainbank. The experimentor was blinded only for the stereological analysis. No particular procedure was applied for randomization / allocating WT and KO mice to the respective experimental groups. Data were derived from at least N = 3 individual mice or N = 5 individual human samples.

Data were tested for outliers via the ROUT function of GraphPad Prism 7. Tests for statistically significant differences were performed in GraphPad Prism 7 software. Most variances were similar between groups that were compared. Parametric statistical test were only chosen if data were normally distributed. Normal distribution was tested with D'Agostino-Pearson omnibus normality test. As n-values were low in general, more conservative non-parametric tests were performed nevertheless in any case, even if data were normally distributed. For comparisons of multiple groups, non-parametric Kruskal Wallis with post hoc Dunn's test or if required. Frequency plots over time were statistically tested via repeated measures two-way ANOVA. If not otherwise stated, unpaired tests were used.

Statistical significances are indicated as $p < 0.05$ (*), $p < 0.01$ (**), $p < 0.001$ (***) and $p < 0.0001$ (****), mostly conservative non-parametric test were applied; individual test are given in the **Supplementary Tables S4-S8 (Fig. 11-15).** In bargraphs, data are given as mean $\pm$ SD or SEM (as indicated). Horizontal lines in boxplots indicate the median of the data, + sign indicated the mean of the data, boxes indicate the $25^{th}$ and $75^{th}$ percentile and whiskers the $10^{th}$ and $90^{th}$ percentile of the data.

Unless defined otherwise, all technical and scientific terms used herein have the same meaning as is commonly understood by one of skill in the art to which this invention belongs.

**Claims**

1. Cav2.3 R-type calcium channel antagonist for use in the treatment of Parkinson's disease, wherein the antagonist is a CRISPR/Cas9 construct, and wherein said antagonist partially or fully inhibits the functional expression of the calcium voltage-gated channel subunit alpha 1E (CACNA1E) gene.

**2.** Cav2.3 R-type calcium channel antagonist for the use according to claim 1, wherein the antagonist is used in combination with dopamine mimetics selected from the group consisting of L-Dopa, dopamine-receptor agonists, and monoamine oxidase inhibitors (MAO inhibitors).

**Patentansprüche**

**1.** Calciumkanalantagonist vom Typ Cav2.3 R zur Verwendung bei der Behandlung von Parkinson-Krankheit, wobei der Antagonist ein CRISPR/Cas9-Konstrukt ist und wobei der Antagonist die funktionelle Expression des Gens der Alpha-1E-Untereinheit spannungsabhängiger Calciumkanäle (CACNA1E) teilweise oder vollständig hemmt.

**2.** Calciumkanalantagonist vom Typ Cav2.3 R zur Verwendung nach Anspruch 1, wobei der Antagonist in Kombination mit Dopaminmimetika verwendet wird, die aus der Gruppe ausgewählt sind, die aus L-Dopa, Dopaminrezeptor-Agonisten und Monoaminoxidase-Hemmern (MAO-Hemmern) besteht.

**Revendications**

**1.** Antagoniste des canaux calciques de type Cav2.3 R destiné à être utilisé dans le traitement de la maladie de Parkinson, dans lequel l'antagoniste est un produit de recombinaison CRISPR/Cas9, et dans lequel ledit antagoniste inhibe partiellement ou complètement l'expression fonctionnelle du gène de la sous-unité alpha 1E des canaux calciques voltage-dépendants (CACNA1E).

**2.** Antagoniste des canaux calciques de type Cav2.3 R destiné à être utilisé selon la revendication 1, dans lequel l'antagoniste est utilisé en combinaison avec des mimétiques de la dopamine sélectionnés dans le groupe consistant en la L-Dopa, des agonistes des récepteurs de la dopamine, et des inhibiteurs de la monoamine oxydase (inhibiteurs de la MAO).

Fig. 1a

Fig. 1b

Fig. 1c

Fig. 1d

Fig. 1e

Fig. 1f

Fig. 1g left

Fig. 1g right

Fig. 1h left

Fig. 1h right

Fig. 2a

Fig. 2b

WT VTA DA

Fig. 2c

WT SN DA
Cav2.3 KO SN DA
WT VTA DA

Fig. 2d

Fig. 2e

Fig. 3a left

Fig. 3a middle

Fig. 3a right

Fig. 3b left

SN

Fig. 3b middle

VTA

SN

Fig. 3b right

Fig. 3c left

Fig. 3c middle

Fig. 3c right

Fig. 3d left

SN

VTA

Fig. 3d middle

SN

[mm]

Fig. 3d right

□ WT
▣ NCS-1 KO

## Cav2.3

Fig. 4a

□ WT

500 µm

▣ NCS-1 KO

Fig. 4b left

Fig. 4b right

NCS-1/Cav2.3

Fig. 4c left

## ND1 mRNA

## ND1 genom DNA

Fig. 4c middle

## genom ND4/ND1

Fig. 4c right

I    II    III    IV

S1 S2 S3 S4 S5 S6    S1 S2 S3 S4 S5 S6    S1 S2 S3 S4 S5 S6    S1 S2 S3 S4 S5 S6

N

C

● R193H  ◉ G1209S
◉ I283N   ● I1422F
◉ G352R   ● S1603F

★ exon 19 missing
☆ 7 aa in exon 20 missing
✦ exon 45 missing

Cav2.3a  ★ ☆
Cav2.3b  ☆ ✦
Cav2.3c  ✦
Cav2.3d
Cav2.3e  ★
Cav2.3f  ☆

Fig. 4d

EP 3 638 283 B1

EP 3 638 283 B1

Fig. 5a

Fig. 5b

Fig. 5c

Fig. 5d

Fig. 5e

Fig. 5f

Fig. 5g

Fig. 5h

Fig. 5i

Fig. 5j

■ juvenile WT
□ adult WT

Fig. 5k left

Fig. 5k right

Fig. 5l upper

## Cav2.3 splice variants

SN DA neuron
Cav2.3e

whole brain
Cav2.3e-f

Fig. 5l lower

Fig. 5m upper

Fig. 5m middle

Fig. 5m lower

Fig. 6a left

Fig. 6a right

Fig. 6b left

Fig. 6b right

Fig. 6c left

Fig. 6c right

Fig. 6d left

Fig. 6d right

Fig. 7a

Fig. 7b left

Fig. 7b right

Fig. 7c left

ND1 mRNA

ND1 genom DNA

Fig. 7c middle

genom ND4/ND1

Fig. 7c right

Fig. 7a upper

**IS4**

| R193H | . . . . . . . . . H . . . . . . . . . . . . | 205 |
| CACNA1E | DLRTLRAVRVLRPLKLVSGIP | 205 |
| CACNA1A | . . . . . . . . . . . . . . . . . . . . | 210 |
| CACNA1B | . . . . . . . . . . . . . . . . . . . . | 207 |
| CACNA1S | .VKA...F......R....V. | 180 |
| CACNA1C | .VKA...F......R....V. | 258 |
| CACNA1D | .VKA...F......R....V. | 255 |
| CACNA1F | .VKA...F......R....V. | 221 |

**IIIS2**

| G1209S | . . . . . . . . . S . . . . . . . . . . | 1219 |
| CACNA1E | EMVIKMIDQGLILQDGSYFR | 1219 |
| CACNA1A | ........L..V.HQ.A... | 1313 |
| CACNA1B | ........L..L.HP.A... | 1216 |
| CACNA1S | .I.L..TTY.AF.HK..FC. | 865 |
| CACNA1C | .IIL..TAY.AF.HK..FC. | 986 |
| CACNA1D | .ILL..TTF.AF.HK.AFC. | 972 |
| CACNA1F | .ILL..TVF.AF.HR..FC. | 937 |

Fig. 7d lower left

```
                                      ┌────────────┐
                              ────────│    pore     │
                                      └────────────┘
I283N           ......N..............         297

CACNA1E         ECK-DWIGPNDGITQFDNILF         297
CACNA1A         K.QPY.E...N..........         306
CACNA1B         ..REY.P...F...N......         302
CACNA1S         ..RGG.P...H...H...FG.         280
CACNA1C         V..PG.D..KH...N...FA.         351
CACNA1D         ..RSG.V...G...N...FA.         352
CACNA1F         ..RGR.P...G...N...FF.         318


                          ┌────────────┐
                          │    IIIS6    │────────
                          └────────────┘
I1422F          ..........F..........

CACNA1E         FFFV....ALIIITFQEQGD         1431
CACNA1A         ....................         1522
CACNA1B         ....................         1424
CACNA1S         ..MM....GFV.V......E         1071
CACNA1C         ..MM....GFV.V......E         1192
CACNA1D         ..MM....GFV.V......E         1178
CACNA1F         ..MM....GFV....RA..E         1143
```

Fig. 7d lower middle

```
                        ┌──────────────┐
                        │     IS6      │──────────────
                        └──────────────┘
G352R            .........R..........          362

CACNA1E          LNLVLGVLSGEFAKERERVE          362
CACNA1A          ....................          372
CACNA1B          ....................          368
CACNA1S          ............T....KAK          345
CACNA1C          ............S....KAK          414
CACNA1D          ............S....KAK          415
CACNA1F          ............S....KAK          383


                                 ┌──────────────┐
                    ─────────────│     IVS5     │
                                 └──────────────┘
S1603F           ........F...........

CACNA1E          ILLWTFVQSFKALPYVCLLIA         1615
CACNA1A          ....................          1705
CACNA1B          ....................          1607
CACNA1S          T.....IK..Q.....A...V         1275
CACNA1C          T.....IK..Q.....A...V         1416
CACNA1D          T.....IK..Q.....A....         1378
CACNA1F          T.....IK..Q.....A....         1335
```

Fig. 7d lower right

Supplementary Table S1: Details of multiplex PCR (outer) and nested PCR (inner) primers.

| Gene | Primer<br><br>F: forward primer<br><br>R: reverse primer | Sequence (5`-3`) | Genbank accession no. (NCBI) | 5`-Position | Amplicon size [bp] |
|---|---|---|---|---|---|
| Mouse Calbindin-d28k<br><br>(CB) | F (outer) | CGCACTCTCAAACTAGCCG | M21531 | 87 | 891 |
| | R (outer) | CAGCCTACTTCTTTATAGCGCA | | 977 | |
| | F (inner) | GAGATCTGGCTTCATTTCGAC | | 167 | 440 |
| | R (inner) | AGTTCCAGCTTTCCGTCATTA | | 606 | |
| Mouse Glial fibrillary acidic protein (GFAP) | F (outer) | AGAACAACCTGGCTGCGTAT | K01347 | 407 | 786 |
| | R (outer) | GCTCCTGCTTCGAGTCCTTA | | 1192 | |
| | F (inner) | AGAAAGGTTGAATCGCTGGA | | 472 | 517 |
| | (inner) | CCAGGGCTAGCTTAACGTTG | | 988 | |
| Mouse Glutamate decarboxylase (GAD65) | F (outer) | CATACGCAGACAGCACGTTT | NM_008078.1 | 166 | 905 |
| | R (outer) | AAAAGATTCCATCGCCAGAG | | 1070 | |
| | F (inner) | GGGATGTCAACTACGCGTTT | | 606 | 389 |
| | R (inner) | CACAAATACAGGGGCGATCT | | 994 | |

Fig. 8

| | | | | | |
|---|---|---|---|---|---|
| Mouse Glutamate decarboxyla se (GAD67) | F (outer) | TGACATCGACTGCCAATACC | Z49976 | 731 | 1105 |
| | R (outer) | GGGTTAGAGATGACCATCCG | | 1835 | |
| | F (inner) | CATATGAAATTGCACCCGTG | | 761 | 702 |
| | R (inner) | CGGTGTCATAGGAGACGTCA | | 1462 | |
| Mouse Tyrosine hydroxylase (TH) | F (outer) | CACCTGGAGTACTTTGTGCG | M69200 | 387 | 1139 |
| | R (outer) | CCTGTGGGTGGTACCCTATG | | 1525 | |
| | F (inner) | TGCACACAGTACATCCGTCA | | 936 | 377 |
| | R (inner) | TCTGACACGAAGTACACCGG | | 1312 | |
| Mouse Cav2.3 R-type calcium channel (Cacna1e) | F (outer II-III loop) | CCATCTACTTCATTGTGCTCACC | NM_009782.3 | 2043 | 816 |
| | R (outer II-III loop) | CCCCTTCATCCAGACTCCG | | 2858 | |
| | F (outer C-terminus) | GACAGCAGCTGGAAGAACAGA | | 5670 | 641 |
| | R (outer C-terminus) | GATGGAGCTCTCACTTAGGGAAC | | 6310 | |
| | F (inner II-II loop) | GGAGGTCAGCCCGATGTC | | 2203 | 420 |
| | R (inner II-III loop) | GGGCTCCTCTGGTTGTCC | | 2622 | |
| | F (inner C-terminus) | CTGAGTGGTCGGAGTGGATAC | | 5795 | 369 |
| | F (innerC-terminus) | AGAGAGGAGGTGCTTTCGTTC | | 6163 | |

Fig. 8 cont.

**Supplementary Table S2: Details of TaqMan qPCR assays and standard curve information.**

| Gene | Assay ID | TaqMan assay: primer/probe or consensus sequences | Gen-bank No | bp | EB | | standard curve data | | | |
|---|---|---|---|---|---|---|---|---|---|---|
| | | | | | | T | Y | slope | $R^2$ | n |
| mD2s (Drd2) | Custom made | P:ACCACTCAAGGATGCTGC<br>F:CGAGCTTTCAGAGCCAACCT<br>R:GCTTGACAGCATCTCCATTTCCA | NM_010077.2 | 84 | 5-7 | 0.5 | 47.59 ± 0.60 | -3.41 ± 0.10 | 0.99 ± 0.01 | 4 |
| mD2l (Drd2) | Custom made | P:CATTCTCCGCCTGTTCAC<br>F:CTCTGCACCGTTATCATGAAGTCTA<br>R:TCGGCGGGCAGCAT | NM_010077.2 | 73 | 6-7 | 0.5 | 46.14 ± 0.23 | -3.53 ± 0.04 | 0.99 ± 0.00 | 4 |
| mCav1.2 (Cacna1c) | Mm01188822_m1 | TCGAAGGGTGGCCAGAGCTGCTGTA | NM_001159533.1 | 65 | 26-27 | 0.6 | 39.95 ± 0.06 | -3.35 ± 0.02 | 0.99 ± 0.00 | 4 |

Fig. 9

EP 3 638 283 B1

| | | | | | | | | | |
|---|---|---|---|---|---|---|---|---|---|
| mCav1.3 (Cacna1d) | Mm0055 1392_m1 | TCCCTCCAGCTGGTGATGA TGAGGT | NM_001 083616 | 62 | 38-39 | 0.6 | 41.12 ± 0.08 | -3.44 ± 0.02 | 0.99 ± 0.00 | 3 |
| mCav2.3 (Cacna1e) | Mm0049 4444_m1 | TTCTGGCCTGAGTGGTCG GAGTGGA | NM_009 782 | 63 | 43-44 | 0.5 | 39.15 ± 0.13 | -3.37 ± 0.01 | 1.00 ± 0.00 | 3 |
| mNCS-1 (Ncs1) | Mm0049 0549_m1 | CGAGAACAAGGATGGCAG GATTGAG | NM_019 681.3 | 73 | 3-4 | 0.3 | 39.26 ± 0.38 | -3.35 ± 0.05 | 1.00 ± 0.00 | 3 |
| hCav2.3 (CACNA1E) | Hs01060 941_m1 | CTTCTTCTGCTCCTTCTTGA TGCTC | NM_001 205293. 1 | 69 | 37-38 | 0.5 | 43.21 ± 0.19 | -3.25 ± 0.04 | 1.00 ± 0.00 | 7 |
| hNCS-1 (NCS1) | Hs00977 274_m1 | TACGGTGGGCCTTCAAGCT CTACGA | NM_014 286.3 | 60 | 4-5 | 0.5 | 40.36 ± 0.06 | -3.34 ± 0.07 | 0.99 ± 0.00 | 6 |

Fig. 9 cont.

| hND1 (mito DNA) | Custom made | P:CCATCACCCTCTACATCA CCGCCC<br><br>F:CCCTAAAACCCGCCACAT CT<br><br>R:GAGCGATGGTGAGAGCT AGGT | NC_012 920.1 | 68 | -- | 0.5 | 33.59 ± 0.1 | -3.30 ± 0.04 | 1.00 ± 0.00 | 2 | 1 |
| hND4 (mito DNA) | Custom made | P:CCGACATCATTACCGGG TTTTCCTCTTG<br><br>F:CCATTCTCCTCCTATCCC TCAAC<br><br>R:CACAATCTGATGTTTTGG TTAAACTATATTT | NC_012 920.1 | 83 | -- | 0.5 | 35.02 ± 0.09 | -3.47 ± 0.04 | 1.00 ± 0.00 | 2 | 1 |

Fig. 9 cont.

**Supplementary Table S3: Details of analyzed *post-mortem* human midbrains.**

| Brain ID | CERAD | BRAAK | sex | Age (years) | PMI (h) | RIN |
|---|---|---|---|---|---|---|
| **adult controls** | | | | | | |
| LFD 40[+,#] | 0 | 0 | m | 37 | 11 | 7.7 |
| LFD 41[-] | 0 | 0 | f | 46 | 27 | 8.5 |
| LFD 43[+,-,#] | 0 | 0 | m | 34 | 10 | 8.3 |
| LFD 44[-,#] | 0 | 0 | m | 40 | 9 | 7.7 |
| LFD 45[+,-,#] | 0 | 0 | m | 46 | 19 | 8.4 |
| LFD 46[+,-,#] | 0 | 0 | m | 49 | 14 | 8.6 |
| | | mean ± SEM: | | 42.0 ± 2.4 | 15.0 ± 2.8 | 8.2 ± 0.2 |
| **aged controls** | | | | | | |
| LFD 5[#] | A | I | f | 69 | 14 | 6.5 |
| LFD 6[#] | 0 | I | m | 62 | 31 | 6.2 |
| LFD 7[#] | 0 | II | m | 72 | 6 | 6.6 |
| LFD 10[#] | 0 | I | f | 75 | 24 | 6 |
| LFD 11[#] | 0 | 0 | m | 63 | 24 | 5.9 |
| LFD 15[#] | 0 | 0 | m | 61 | 6.5 | 1.9 |
| LFD 16[#] | 0 | I | f | 69 | 24 | 6.8 |
| LFD 17[#] | 0 | II | f | 68 | 120 | 5.9 |
| LFD 18[#] | 0 | I | f | 71 | 72 | 6.5 |
| LFD 19[#] | 0 | I | m | 72 | 24 | 6.6 |

Fig. 10

| | | | | | | |
|---|---|---|---|---|---|---|
| LFD 21⁻ | 0 | I | f | 73 | 24 | 9.2 |
| LFD 22⁺,⁻ | 0 | I | m | 67 | 16 | 6.4 |
| LFD 26⁺ | 0 | II | f | 83 | 22 | 6.8 |
| LFD 27⁻ | A | I | m | 76 | 24 | 6.9 |
| LFD 29⁺ | 0 | 0 | f | 76 | 11 | 6.4 |
| LFD 37⁺,⁻ | 0 | II | f | 81 | 18 | 4 |
| | **mean ± SEM** | | | 71.1 ± 1.57 | 28.8 ± 7.13 | 6.2 ± 0,38 |
| **Aged PD** | | | | | | |
| LFD 1# | 0 | II | f | 79 | 17 | 7.3 |
| LFD 2# | 0 | 0 | m | 79 | 23 | 7.5 |
| LFD 3# | B | III-IV | m | 80 | 23 | 6.8 |
| LFD 4# | 0 | II | m | 80 | 7 | 7.1 |
| LFD 14# | 0 | V | f | 73 | 11 | 7.7 |
| LFD 31⁺,⁻ | A | II | f | 83 | 29 | 8.8 |
| LFD 33⁺ | 0 | II | m | 83 | 24 | 6 |
| LFD 35⁺,⁻ | A | III | f | 85 | 16 | 8.2 |
| LFD 36⁺,⁻ | 0 | <III | m | 79 | 9 | 6.6 |
| | **mean ± SEM** | | | 80.1 ± 1.15 | 17.7 ± 2.53 | 7.3 ± 0.28 |

Abbreviations: CERAD: Consortium to Establish a Registry for Alzheimer's Disease; PMI: *post-mortem* interval in hours, RIN: RNA integrity number; PD: Parkinson's disease; AD: Alzheimer's disease; + *used for ND1 mRNA quantification; ~used for NCS-1 & Cav2.3 mRNA quantification, #used for ND1 & ND4 mitochondrial genomic DNA quantification.*

Fig. 10 cont.

EP 3 638 283 B1

Supplementary Table S4: *In vitro* activity of SN DA neurons at last minute in dopamine.

| | SN DA activity at 15min in dopamine [%] | | | | | | |
|---|---|---|---|---|---|---|---|
| | mean | ±SD | ±SEM | median | 95% CI | n | p-value (compared to adult WT) |
| Adult WT* | 0.0 | 0.1 | 0.0 | 0.0 | 0.0-0.0 | 14 | |
| Juvenile WT* | 40.0 | 31.8 | 7.5 | 44.7 | 0.0-66.4 | 18 | <0.0001 |
| Adult WT EGTA& | 22.8 | 30.8 | 11.6 | 0.0 | 0.0-72.9 | 7 | 0.0263 |
| Adult WT DNIP | 56.8 | 36.2 | 20.9 | 55.2 | 21.4-93.8 | 3 | 0.0015 |
| Adult NCS-1 KO | 31.3 | 38.0 | 12.7 | 0.0 | 0.0-80.3 | 9 | 0.0142 |
| Adult WT isradipine | 1.5 | 4.6 | 1.5 | 0.0 | 0.0-0.0 | 9 | 0.7510 |
| Adult WT Z941 | 2.8 | 6.6 | 2.2 | 0.0 | 0.0-5.3 | 9 | 0.3478 |
| Adult WT ω–ctx MVIIC | 15.0 | 24.9 | 10.2 | 3.1 | 0.0-63.1 | 6 | 0.0175 |
| Adult WT SNX-482 | 33.7 | 28.8 | 11.8 | 34.9 | 0.0-73.2 | 6 | 0.0004 |
| Adult Cav2.3 KO | 26.2 | 33.9 | 12.8 | 7.4 | 0.0-74.7 | 7 | 0.0010 |

Fig. 11

EP 3 638 283 B1

Supplementary Table S5: *In vitro* pacemaker parameters of SN DA neurons.

| | A) SN DA pacemaker frequency [Hz] | | | | | | |
|---|---|---|---|---|---|---|---|
| | mean | ±SD | ±SEM | median | 95% CI | n | p-value (compared to adult WT) |
| **Adult WT** | 2.6 | 1.0 | 0.2 | 2.8 | 1.9-3.5 | 19 | |
| **Adult WT isradipine** | 3.2 | 0.9 | 0.3 | 2.8 | 2.7-3.9 | 9 | 0.3318 |
| **Adult Cav1.3 KO** | 3.3 | 1.1 | 0.3 | 3.4 | 2.6-3.7 | 13 | 0.1467 |
| **Adult WT SNX-482** | 2.0 | 0.5 | 0.2 | 2.0 | 1.3-2.8 | 6 | 0.1561 |
| **Adult Cav2.3 KO** | 2.3 | 0.7 | 0.3 | 2.5 | 1.0-3.1 | 8 | 0.3887 |
| | B) SN DA pacemaker precision, CV ISI [%] | | | | | | |
| | mean | ±SD | ±SEM | median | 95% CI | n | p-value (compared to adult WT) |
| **Adult WT** | 8.1 | 5.4 | 1.5 | 6.4 | 4.7-13.2 | 14 | |
| **Adult WT isradipine** | 9.9 | 3.4 | 1.2 | 10.1 | 5.8-14.6 | 8 | 0.1450 |
| **Adult Cav1.3 KO** | 11.4 | 5.6 | 2.3 | 10.1 | 5.3-19.2 | 6 | 0.1528 |
| **Adult WT SNX-482** | 9.8 | 3.2 | 1.3 | 8.4 | 7.0-15.0 | 6 | 0.0913 |
| **Adult Cav2.3 KO** | 15.1 | 6.0 | 2.1 | 16.6 | 7.4-24.4 | 8 | **0.0038** |

Fig. 12

| | C) SN DA pacemaker precision, CV2 [%] | | | | | | |
|---|---|---|---|---|---|---|---|
| | mean | ±SD | ±SEM | median | 95% CI | n | p-value (compared to adult WT) |
| **Adult WT** | 3.5 | 1.9 | 0.5 | 2.9 | 2.2-5.5 | 13 | |
| **Adult WT isradipine** | 5.7 | 1.9 | 0.7 | 5.4 | 3.6-8.6 | 8 | **0.0126** |
| **Adult Cav1.3 KO** | 5.4 | 2.4 | 1.0 | 5.1 | 2.4-9.3 | 6 | 0.0715 |
| **Adult WT SNX-482** | 4.2 | 2.1 | 0.8 | 3.0 | 2.7-7.4 | 6 | 0.4155 |
| **Adult Cav2.3 KO** | 9.9 | 6.1 | 2.2 | 8.7 | 2.7-22.8 | 8 | **0.0013** |

Fig. 12 cont.

**Supplementary Table S6 A/B: Ca$^{2+}$ imaging in SN and VTA DA neurons.**

| A) Spontaneous activity-dependent Ca$^{2+}$ transients | | | | | | | | | | | |
|---|---|---|---|---|---|---|---|---|---|---|---|
| max. amplitude (norm. Fura-2 ratio) | | | | | | area under the curve | | | | | |
| mean | ±SD | ±SEM | median | 95% CI | n | mean | ±SD | ±SEM | median | 95% CI | n |
| **SN DA WT** | 1.10 | 0.03 | 0.01 | 1.09 | 1.08-1.12 | 8 | 399.5 | 101.8 | 36.0 | 376.6 | 314.4-484.6 | 8 |
| **SN DA Cav2.3 KO** | 1.06 | 0.02 | 0.01 | 1.05 | 1.04-1.08 | 9 | 233.9 | 62.8 | 20.9 | 210.9 | 185.6-282.2 | 9 |
| **VTA DA WT** | 1.02 | 0.01 | 0.004 | 1.03 | 1.02-1.04 | 7 | 137.4 | 48.2 | 18.2 | 120.0 | 92.9-182.0 | 7 |

*p-values for max. amplitude: SN DA WT vs. Cav2.3 KO: 0.0016; VTA DA WT vs. SN DA WT: <0.0001; VTA DA WT vs. SN DA Cav2.3 KO: 0.0094

*p-values for area under the curve: SN DA WT vs. Cav2.3 KO: 0.0004; VTA DA WT vs. SN DA WT: <0.0001; VTA DA WT vs. SN DA Cav2.3 KO: 0.0185

| B) Action-potential induced Ca$^{2+}$ transients | | | | | | | | | | | | |
|---|---|---|---|---|---|---|---|---|---|---|---|---|
| WT control | | | | | | WT SNX-482 | | | | | | |
| mean | ±SD | ±SEM | median | 95% CI | n | mean | ±SD | ±SEM | median | 95% CI | n | p-value# |
| **SN DA neurons** | 1.08 | 0.03 | 0.01 | 1.08 | 1.05-1.12 | 6 | 1.07 | 0.03 | 0.01 | 1.07 | 1.03-1.10 | 6 | |
| **Remaining Ca$^{2+}$ signal [%]** | | | | | | | 76.3 | 16.6.0 | 6.8 | 77 | 58.8-93.7 | 6 | **0.0173** |

p-value for WT control vs. WT SNX-482: **0.031**, p-value for Remaining Ca$^{2+}$ signal: **0.0173**

Fig. 13

Supplementary Table S7 A/B/C: Single cell qPCR data of mouse and human SN DA neurons.

| analyzed mRNA | A) juvenile vs. adult WT mice | | | | | | | | | | | | |
|---|---|---|---|---|---|---|---|---|---|---|---|---|---|
| | juvenile | | | | | | adult | | | | | | |
| | mean | ±SD | ±SEM | median | 95% CI | n | mean | ±SD | ±SEM | median | 95% CI | n | p-value* |
| mCav2.3 | 10.3 | 3.3 | 0.9 | 10.7 | 4.6-15.5 | 13 | 21.4 | 13.9 | 2.7 | 15.6 | 5.8-53.9 | 27 | **0.0081** |
| mCav1.2 | 8.4 | 3.4 | 0.7 | 7.6 | 3.0-17.4 | 26 | 6.1 | 3.4 | 0.7 | 5.9 | 1.4-13.9 | 25 | **0.0171** |
| mCav1.3 | 7.1 | 4.0 | 0.8 | 6.5 | 1.3-19.6 | 25 | 4.3 | 3.0 | 0.6 | 3.5 | 0.7-13.7 | 25 | **0.0018** |
| mNCS-1 | 34.8 | 15.0 | 4.2 | 32.2 | 14.1-63.0 | 13 | 30.2 | 16.7 | 4.5 | 23.7 | 5.7-64.1 | 14 | 0.4020 |
| mNCS1/ mCav2.3 | 3.4 | 1.2 | 0.3 | 3.6 | 2.4-4.5 | 13 | 1.5 | 0.6 | 0.2 | 1.2 | 1.0-2.2 | 14 | **<0.0001** |
| mD2s | 1.0 | 0.8 | 0.3 | 0.7 | 0.2-1.9 | 10 | 1.7 | 0.5 | 0.2 | 1.5 | 1.2-2.2 | 10 | **0.0288** |
| mD2l | 1.0 | 0.8 | 0.3 | 0.7 | 0.2-2.1 | 10 | 2.0 | 0.6 | 0.2 | 1.8 | 1.4-2.5 | 10 | **0.0232** |
| mD2l/ mD2s | 1.0 | 0.3 | 0.1 | 1.2 | 0.7-1.3 | 10 | 1.3 | 0.4 | 0.1 | 1.3 | 0.8-1.7 | 10 | 0.1655 |

Fig. 14

EP 3 638 283 B1

| B) Cav2.3 mRNA in WT vs. NCS-1 KO mice | | | | | | | | | | | | |
|---|---|---|---|---|---|---|---|---|---|---|---|---|
| | **WT** | | | | | | **NCS-1 KO** | | | | | |
| | mean | ±SD | ±SEM | median | 95% CI | n | mean | ±SD | ±SEM | median | 95% CI | n | p-value* |
| Juvenile (PN 13) | 26.8 | 9.0 | 2.4 | 25.8 | 13.1-37.6 | 14 | 13.6 | 7.2 | 1.6 | 13.3 | 4.1-27.4 | 20 | 0.0002 |
| Aged (PN 550) | 25.1 | 9.6 | 2.5 | 23.8 | 7.8-41.6 | 15 | 14.7 | 4.4 | 1.2 | 15.9 | 8.2-24.6 | 14 | 0.0007 |

| C) human SN DA neurons | | | | | | | | | | | | |
|---|---|---|---|---|---|---|---|---|---|---|---|---|
| | **Blood standard** | | | | | | **Adult control** | | | | | |
| | mean | ±SD | ±SEM | median | 95% CI | n/N | mean | ±SD | ±SEM | median | 95% CI | n/N |
| hNCS-1/ hCav2.3 | | | | | | | 1.0 | 0.7 | 0.1 | 0.7 | 0.5-1.2 | 39/5 |
| mRNA ND1 | | | | | | | 1.0 | 0.5 | 0.1 | 0.9 | 0.6-1.4 | 23/4 |
| gDNA hND1 | | | | | | | 1.0 | 0.5 | 0.1 | 0.9 | 0.7-1.1 | 81/6 |
| hND4/hND1 | 1.0 | 0.3 | 0.1 | 0.9 | 0.9-1.2 | 21/1 | 0.8 | 0.1 | 0.0 | 0.9 | 0.8-0.9 | 83/6 |

Fig. 14 cont.

EP 3 638 283 B1

| | C) human SN DA neurons | | | | | | | | | | | | |
|---|---|---|---|---|---|---|---|---|---|---|---|---|---|
| | Aged control | | | | | | PD | | | | | | |
| | mean | ±SD | ±SEM | median | 95% CI | n/N | mean | ±SD | ±SEM | median | 95% CI | n/N | p-value[#] |
| hNCS-1/ hCav2.3 | 1.0 | 0.7 | 0.2 | 0.9 | 0.5-1.4 | 19/4 | 1.1 | 0.6 | 0.2 | 0.9 | 0.5-1.8 | 14/3 | 0.6949 |
| mRNA ND1 | 2.5 | 1.4 | 0.3 | 2.3 | 1.3-2.9 | 27/4 | 2.0 | 0.8 | 0.2 | 2.0 | 1.3-2.7 | 19/4 | **<0.0001** |
| gDNA hND1 | 1.0 | 0.7 | 0.1 | 0.8 | 0.6-0.9 | 148/10 | 0.9 | 0.4 | 0.1 | 0.9 | 0.7-1.0 | 73/5 | 0.1764 |
| hND4/hND1 | 0.9 | 0.3 | 0.0 | 0.9 | 0.9-1.0 | 150/10 | 0.8 | 0.3 | 0.0 | 0.8 | 0.7-0.9 | 75/5 | **0.0112** |

Fig. 14 cont.

Supplementary Table S8 A/B/C/D: Stereology and densitometry data.

| | A) SN DA neurons | | | | | | | | | | | | |
|---|---|---|---|---|---|---|---|---|---|---|---|---|---|
| | WT | | | | | | NCS-1 KO | | | | | | p-value: 0.0013# |
| | mean | ±SD | ±SEM | median | 95% CI | n | mean | ±SD | ±SEM | median | 95% CI | n | |
| juvenile | 5707 | 459 | 265 | 5647 | 5282-6193 | 3 | 3991 | 226 | 130 | 4001 | 3760-4211 | 3 | 0.0063~ |
| adult | 5498 | 76 | 44 | 5535 | 5411-5548 | 3 | 4094 | 118 | 68 | 4111 | 3968-4202 | 3 | 0.0165~ |
| 1 year | 5219 | 276 | 123 | 5179 | 4989-5689 | 5 | 4124 | 280 | 114 | 4170 | 3653-4475 | 6 | 0.0270~ |
| 1.5 years | 4949 | 152 | 88 | 4990 | 4781-5077 | 3 | 3809 | 228 | 132 | 3682 | 3673-4073 | 3 | 0.0493~ |

| | VTA DA neurons | | | | | | | | | | | | |
|---|---|---|---|---|---|---|---|---|---|---|---|---|---|
| | WT | | | | | | NCS-1 KO | | | | | | p-value: 0.6752# |
| | mean | ±SD | ±SEM | median | 95% CI | n | mean | ±SD | ±SEM | median | 95% CI | n | |
| juvenile | 5683 | 79 | 46 | 5703 | 5596-5751 | 3 | 5464 | 173 | 100 | 5373 | 5355-5664 | 3 | 0.1985~ |
| adult | 5570 | 226 | 131 | 5667 | 5311-5731 | 3 | 5458 | 95 | 55 | 5501 | 5349-5523 | 3 | 0.3284~ |

Fig. 15

| | | | | | | | | | | | | |
|---|---|---|---|---|---|---|---|---|---|---|---|---|
| **1 year** | 5609 | 201 | 90 | 5637 | 5279-5822 | 5 | 5673 | 233 | 116 | 5703 | 5371-5916 | 4 | 0.5668~ |
| **1.5 years** | 5571 | 336 | 194 | 5585 | 5228-5900 | 3 | 5541 | 197 | 114 | 5604 | 5320-5698 | 3 | 0.9590~ |

~p-value of uncorrected Dunn´s test as each comparison stands alone.

| | **B) Dorsal Striatum (DS)** | | | | | | | | | | | | |
|---|---|---|---|---|---|---|---|---|---|---|---|---|---|
| | **control** | | | | | | **MPTP** | | | | | | |
| | **mean** | **±SD** | **±SEM** | **median** | **95% CI** | **n** | **mean** | **±SD** | **±SEM** | **median** | **95% CI** | **n** | **p-value*** |
| **NCS-1 WT** | 32.8 | 5.1 | 1.4 | 32.1 | 28.1-36.4 | 14 | 13.8 | 6.9 | 1.9 | 15.2 | 5.8-20.6 | 13 | **<0.0001** |
| **NCS-1 KO** | 31.9 | 6.1 | 1.9 | 29.9 | 25.9-39.4 | 10 | 7.4 | 4.6 | 1.4 | 6.0 | 3.5-15.3 | 11 | **<0.0001** |
| **Cav2.3 WT** | 32.9 | 2.7 | 0.9 | 32.8 | 30.2-36.0 | 9 | 14.7 | 7.0 | 1.9 | 16.8 | 8.1-21.3 | 13 | **<0.0001** |
| **Cav2.3 KO** | 36.1 | 5.6 | 1.9 | 33.7 | 31.2-42.3 | 9 | 23.4 | 7.2 | 2.1 | 22.8 | 19.8-30.0 | 12 | **0.0005** |

Fig. 15 cont.

| | Ventral Striatum (VS) | | | | | | | | | | | | |
|---|---|---|---|---|---|---|---|---|---|---|---|---|---|
| | control | | | | | | MPTP | | | | | | |
| | mean | ±SD | ±SEM | median | 95% CI | n | mean | ±SD | ±SEM | median | 95% CI | n | p-value* |
| NCS-1 WT | 32.7 | 6.4 | 1.7 | 31.7 | 25.8-39.4 | 14 | 26.8 | 5.1 | 1.4 | 28.0 | 22.4-33.0 | 13 | 0.0193 |
| NCS-1 KO | 36.2 | 9.1 | 2.9 | 33.8 | 27.9-45.4 | 10 | 27.7 | 6.7 | 2.0 | 27.2 | 20.6-36.2 | 11 | 0.0357 |
| Cav2.3 WT | 33.4 | 4.9 | 1.6 | 32.5 | 28.0-39.5 | 9 | 24.9 | 3.7 | 1.0 | 25.0 | 21.5-28.1 | 13 | 0.0004 |
| Cav2.3 KO | 40.5 | 8.1 | 2.7 | 37.1 | 33.2-51.6 | 9 | 31.2 | 5.6 | 1.6 | 29.1 | 26.4-37.3 | 12 | 0.0227 |

| | Substantia nigra (SN) | | | | | | | | | | | | |
|---|---|---|---|---|---|---|---|---|---|---|---|---|---|
| | control | | | | | | MPTP | | | | | | |
| | mean | ±SD | ±SEM | median | 95% CI | n | mean | ±SD | ±SEM | median | 95% CI | n | p-value* |
| NCS-1 WT | 4351 | 780 | 208 | 4224 | 3171-5887 | 14 | 2070 | 688 | 191 | 1839 | 1159-3576 | 13 | <0.0001 |
| NCS-1 KO | 3519 | 289 | 91 | 3454 | 3239-4090 | 10 | 1257 | 364 | 110 | 1332 | 657-1644 | 11 | <0.0001 |
| Cav2.3 WT | 3959 | 643 | 215 | 3826 | 3637-4622 | 9 | 2535 | 890 | 247 | 2906 | 1566-3542 | 13 | 0.0002 |
| Cav2.3 KO | 4170 | 520 | 174 | 4264 | 3475-4833 | 9 | 3990 | 454 | 144 | 3938 | 3618-4286 | 10 | 0.4002 |

Fig. 15 cont.

EP 3 638 283 B1

| | Ventral tegmental area (VTA) | | | | | | | | | | | | |
|---|---|---|---|---|---|---|---|---|---|---|---|---|---|
| | control | | | | | | MPTP | | | | | | |
| | mean | ±SD | ±SEM | median | 95% CI | n | mean | ±SD | ±SEM | median | 95% CI | n | p-value* |
| NCS-1 WT | 6499 | 1222 | 327 | 6414 | 4387-9188 | 14 | 5045 | 1250 | 347 | 4763 | 2948-6808 | 13 | **0.0091** |
| NCS-1 KO | 6217 | 911 | 288 | 6057 | 5156-8364 | 10 | 4076 | 1009 | 304 | 4333 | 1755-4871 | 11 | **<0.0001** |
| Cav2.3 WT | 6806 | 825 | 275 | 6731 | 5934-7910 | 9 | 6057 | 1209 | 335 | 5600 | 5238-7683 | 13 | 0.0708 |
| Cav2.3 KO | 7480 | 931 | 310 | 7547 | 6679-8285 | 9 | 6689 | 660 | 209 | 6642 | 6071-7167 | 10 | 0.0535 |

Fig. 15 cont.

| | C) Remaining fibers [%] | | | | | | | | | | | |
|---|---|---|---|---|---|---|---|---|---|---|---|---|
| | WT MPTP | | | | | | KO MPTP | | | | | |
| | mean | ±SD | ±SEM | median | 95% CI | n | mean | ±SD | ±SEM | median | 95% CI | n | p-value[+] |
| NCS-1 KO: DS | 42.2 | 21.3 | 5.9 | 46.4 | 17.6-63.1 | 13 | 23.7 | 15.5 | 4.7 | 18.0 | 10.4-50.1 | 11 | **0.0253** |
| NCS-1 KO: VS | 81.2 | 11.3 | 3.1 | 77.7 | 71.3-93.7 | 13 | 76.8 | 19.7 | 6.0 | 76.6 | 55.7-102.2 | 11 | 0.4987 |
| Cav2.3 KO: DS | 44.7 | 21.4 | 5.9 | 50.8 | 24.5-64.2 | 13 | 66.0 | 19.4 | 5.6 | 65.4 | 57.0-83.9 | 12 | **0.0159** |
| Cav2.3 KO: VS | 74.5 | 12.3 | 3.4 | 76.7 | 62.7-86.1 | 13 | 78.2 | 13.5 | 3.9 | 75.8 | 67.3-90.4 | 12 | 0.4817 |

| | Remaining neurons [%] | | | | | | | | | | | |
|---|---|---|---|---|---|---|---|---|---|---|---|---|
| | WT MPTP | | | | | | KO MPTP | | | | | |
| | mean | ±SD | ±SEM | median | 95% CI | n | mean | ±SD | ±SEM | median | 95% CI | n | p-value[+] |
| NCS-1 KO: SN | 47.6 | 15.8 | 4.4 | 42.2 | 26.6-82.2 | 13 | 35.7 | 10.0 | 3.0 | 36.6 | 19.3-48.3 | 11 | **0.0431** |
| NCS-1 KO: VTA | 77.8 | 19.9 | 5.5 | 72.2 | 46.3-106.9 | 13 | 65.6 | 16.2 | 4.9 | 70.0 | 28.3-78.7 | 11 | 0.1169 |
| Cav2.3 KO: SN | 64.1 | 23.0 | 6.4 | 74.6 | 40.2-86.7 | 13 | 95.4 | 11.0 | 3.5 | 93.8 | 86.2-109.3 | 10 | **0.0007** |
| Cav2.3 KO: VTA | 95.8 | 19.1 | 5.3 | 88.6 | 82.8-121.5 | 13 | 96.7 | 9.5 | 3.0 | 96.1 | 87.8-103.7 | 10 | 0.8880 |

Fig. 15 cont.

| | **D) Remaining fibers [%] (bootstrapping approach)** | | | | | | | | | | | | |
|---|---|---|---|---|---|---|---|---|---|---|---|---|---|
| | **WT MPTP** | | | | | | **KO MPTP** | | | | | | |
| | mea n | ±SD | ±SE M | media n | 95% CI | n | mea n | ±SD | ±SE M | media n | 95% CI | n | p- value* |
| **NCS-1 KO: DS** | 43.0 | 21.7 | 1.6 | 43.3 | 33.7- 51.8 | 182 | 24.0 | 15.1 | 1.4 | 19.6 | 17.8- 21.3 | 110 | **<0.000 1** |
| **NCS-1 KO: VS** | 84.9 | 22.7 | 1.7 | 80.8 | 76.6- 87.7 | 182 | 80.5 | 26.2 | 2.5 | 76.1 | 70.5- 83.4 | 110 | 0.0730 |
| **Cav2.3 KO: DS** | 45.0 | 21.0 | 1.9 | 48.0 | 45.6- 51.9 | 117 | 66.2 | 21.8 | 2.1 | 66.4 | 61.6- 70.3 | 108 | **<0.000 1** |
| **Cav2.3 KO: VS** | 76.0 | 15.2 | 1.4 | 73.4 | 70.7- 77.9 | 117 | 79.5 | 19.4 | 1.9 | 77.6 | 74.8- 80.4 | 108 | 0.2005 |
| | **Remaining neurons [%] (bootstrapping approach)** | | | | | | | | | | | | |
| | **WT MPTP** | | | | | | **KO MPTP** | | | | | | |
| | mea n | ±SD | ±SE M | media n | 95% CI | n | mea n | ±SD | ±SE M | media n | 95% CI | n | p- value* |
| **NCS-1 KO: SN** | 49.0 | 17.9 | 1.3 | 45.0 | 19.7- 112.8 | 182 | 35.9 | 10.2 | 1.0 | 37.5 | 16.1- 50.7 | 110 | **<0.000 1** |
| **NCS-1 KO: VTA** | 80.1 | 24.1 | 1.8 | 76.5 | 32.1- 155.2 | 182 | 66.7 | 18.0 | 1.7 | 70.8 | 21.0- 94.5 | 110 | **<0.000 1** |
| **Cav2.3 KO: SN** | 65.8 | 25.6 | 2.4 | 67.8 | 57.7- 75.9 | 117 | 97.1 | 16.0 | 1.7 | 95.3 | 90.3- 99.7 | 90 | **<0.000 1** |
| **Cav2.3 KO: VTA** | 90.1 | 20.2 | 1.9 | 88.3 | 83.2- 92.3 | 117 | 90.7 | 13.7 | 1.4 | 89.1 | 86.5- 93.2 | 90 | 0.5003 |

Fig. 15 cont.

EP 3 638 283 B1

Supplementary Table S9: *De novo* missense & splice site mutations in *CACNA1E* identified in patients with neurodevelopmental disorders (NDD) and intellectual disability (ID).

| Pheno type | Position (hg19) | Vari ant | Mutation class | cDNA* | Protein | Exon / Intron | CADD (ref d) | Polyphen -2 (ref e) | REVEL (ref f) | Ref . |
|---|---|---|---|---|---|---|---|---|---|---|
| NDD | chr1:181 687189 | A>G | splice acceptor | c.1526-2A>G | NA | intron 11 | NA | NA | NA | 1 |
| NDD | chr1:181 735748 | G>T | splice donor | c.4881+1 G>T | NA | intron 35 | NA | NA | NA | 1 |
| NDD | chr1:181 546967 | G>A | missense | c.578 G>A | p.(R193H) | exon 4 | 34.0 | 1.000 | 0.935 | 1 |
| NDD | chr1:181 549809 | T>A | missense | c.848 T>A | p.(I283N) | exon 6 | 24.0 | 0.970 | 0.747 | 1 |
| ID | chr1:181 620576 | G>A | missense | c.1054 G>A | p.(G352R) | exon 7 | 25.6 | 1.000 | 0.913 | 2 |
| autism | chr1:181 708295 | G>A | missense | c.3625 G>A | p.(G1209 S) | exon 25 | 33.0 | 0.999 | 0.977 | 3 |

Fig. 16

| NDD | chr1:181726197 | A>T | missense | c.4264 A>T | p.(I1422F) | exon 30 | 26.0 | 0.999 | 0.918 | 1 |
| ID | chr1:181732660 | C>T | missense | c.4808 C>T | p.(S1603F) | exon 34 | 33.0 | 1.000 | 0.961 | 2 |

*based on RefSeq transcript number NM_001205293.1

Fig. 16 cont.

Fig. 17 left

Fig. 17 right

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2012321594 A1, HONG KYONSOO
- US 2014038927 A1, COHEN DANIEL
- WO 9415607 A

### Non-patent literature cited in the description

- **RODOLPHE HAJJ.** Combination of acamprosate and baclofen as a promising therapeutic approach for Parkinson's disease. *SCIENTIFIC REPORTS,* 06 November 2015, vol. 5 (1
- **GEZA BERECKI.** Differential Ca v 2.1 and Ca v 2.3 channel inhibition by baclofen and [alpha]-conotoxin Vc1.1 via GABA B receptor activation. *JOURNAL OF GENERAL PHYSIOLOGY,* 31 March 2014, vol. 143 (4), ISSN 0022-1295, 465-479
- **M. J. CANO-CEBRIAN.** Local acamprosate modulates dopamine release in the rat nucleus accumbens through NMDA receptors: an in vivo microdialysis study. *NAUNYN-SCHMIEDEBERG'S ARCHIVES OF PHARMACOLOI,* 01 February 2003, vol. 367 (2), ISSN 0028-1298, 119-125
- **KOMEILI GHOLAMREZA.** Effects of Isradipine on Induced-Parkinson's Disease in Rats. *Zahedan Journal of Research in Medical Sciences,* 26 April 2016
- Amiloride is neuroprotective in an MPTP model of Parkinson's disease. **ARIAS R L.** NEUROBIOLOGY OF DISEASE. ELSEVIER, 01 September 2008, vol. 31, 334-341
- **GOMEZ-MANCILLA BALTAZAR.** Effect of ethosuximide on rest tremor in the MPTP monkey model: ETHOSUXIMIDE AND REST TREMOR. *MOVEMENT DISORDERS,* 01 January 1992, vol. 7 (2), ISSN 0885-3185, 137-141
- **SCHNEIDER TONI.** How Pharmacoresistant is Cav2.3, the Major Component of Voltage-Gated R-type Ca2+ Channels?. *Pharmaceuticals,* 01 January 2013, vol. 6 (6), 759-776, https://www.ncbi.nlm.nih.gov/pmc/articles/PMC3816731/pdf/pharmaceuticals-06-00759.pdf
- **HAINSWORTH ATTICUS H.** Actions of sipatrigine, 202W92 and lamotrigine on R-type and T-type Ca2+ channel currents. *European Journal of Pharmacology,* 01 April 2003, vol. 467 (1-3), ISSN 0014-2999, 77-80

- The novel anticonvulsant lamotrigine prevents dopamine depletion in C57 black mice in the MPTP animal model of Parkinson's disease. **JONES-HUMBLE 5 A.** LIFE SCIENCE. PERGAMON PRESS, 01 January 1994, vol. 54, 245-252
- **MCMANUS OB.** HTS assays for developing the molecular pharmacology of ion channels. *Curr Opin Pharmacol.,* 2014, vol. 15, 91-96
- **HAI-BO YU ; MIN LI ; WEI-PING WANG ; XIAO-LIANG WANG.** High throughput screening technologies for ion Channels. *Acta Pharmacologica Sinica,* 2016, vol. 37, 34-43
- *Assay Guidance Manual,* 31 March 2017, https://www.ncbi.nlm.nih.gov/books/NBK100915
- **NG, E. et al.** Neuronal calcium sensor-1 deletion in the mouse decreases motivation and dopamine release in the nucleus accumbens. *Behav Brain Res,* 2016, vol. 301, 213-225
- **PEREVERZEV, A. et al.** Disturbances in glucose-tolerance, insulin-release, and stress-induced hyperglycemic upon disruption of the Ca(v)2.3 (alpha 1EJ subunit of voltage-gated Ca(2+) channels. *Mol Endocrinol,* 2002, vol. 16, 884-895
- **MEREDITH, G. E. ; RADEMACHER, D. J.** MPTP mouse models of Parkinson's disease: an update. *J Parkinsons Dis,* 2011, vol. 1, 19-33
- **KRABBE, S. et al.** Increased dopamine D2 receptor activity in the striatum alters the firing pattern of dopamine neurons in the ventral tegmental area. *Proc Natl Acad Sci U S A,* 2015, vol. 112, E1498-1506
- **PAXINOS, G. ; FRANKLIN, K.** *The Mouse Brain in Stereotaxic Coordinates,* 2007
- **DRAGICEVIC, E. et al.** Cav1.3 channels control D2-autoreceptor responses via NCS-1 in substantia nigra dopamine neurons. *Brain,* 2014, vol. 137, 2287-2302
- **POETSCHKE, C. et al.** Compensatory T-type Ca2+ channel activity alters D2-autoreceptor responses of Substantia nigra dopamine neurons from Cavl.3 L-type Co2+ channel KO mice. *Sci Rep,* 2015, vol. 5, 13688

- **SAAB, B. J. et al.** NCS-1 in the dentate gyrus promotes exploration, synaptic plasticity, and rapid acquisition of spatial memory. *Neuron,* 2009, vol. 63, 643-656
- **TRINGHAM, E. et al.** T-type calcium channel blockers that attenuate thalamic burst firing and suppress absence seizures. *Sci Transl Med,* 2012, vol. 4, 121-119
- **KONNER, A. C. et al.** Role for insulin signaling in catecholaminergic neurons in control of energy homeostasis. *Cell metabolism,* 2011, vol. 13, 720-728
- **ORTNER et al.** *J. neurosci,* 2017
- **KIMM, T. ; BEAN, B. P.** Inhibition of A-type potassium current by the peptide toxin SNX-482. *J Neurosci,* 2014, vol. 34, 9182-9189
- **HESS, M. E. et al.** The fat mass and obesity associated gene (Fto) regulates activity of the dopaminergic midbrain circuitry. *Not Neurosci,* 2013, vol. 16, 1042-1048
- **SCHLAUDRAFF, F. et al.** Orchestrated increase of dopamine and PARK mRNAs but not miR-133b in dopamine neurons in Parkinson's disease. *Neurobiol Aging,* 2014, vol. 35, 2302-2315
- **DUDA, J. ; FOULER, M. ; GRUNDEMANN, J. ; LISS, B.** Cell-specific RNA quantification in human SN DA neurons from heterogeneous post mortem midbrain samples by UV-laser microdissection and RT-qPCR. *Methods Mol Biol*
- **LISS, B. et al.** Tuning pacemaker frequency of individual dopaminergic neurons by Kv4.3L and KChip3.1 transcription. *EMBO J,* 2001, vol. 20, 5715-5724
- **ORTNER, N. J. et al.** Lower affinity of isradipinefor L-type Ca2+ channels during Substantia nigra dopamine neuron-like activity: implications for neuroprotection in Parkinson's disease. *J Neurosci*
- **GRUNDEMANN, J. ; SCHLAUDRAFF, F. ; LISS, B.** UV-laser microdissection and mRNA expression analysis of individual neurons from postmortem Parkinson's disease brains. *Methods Mol Biol,* 2011, vol. 755, 363-374
- **MUHLING, T. ; DUDA, J. ; WEISHAUPT, J. H. ; LUDOLPH, A. C. ; LISS, B.** Elevated mRNA-levels of distinct mitochondrial and plasma membrane Co(2+) transporters in individual hypoglossal motor neurons of endstage SOD1 transgenic mice. *Front Cell Neurosci,* 2014, vol. 8, 353
- **BENDER, A. et al.** High levels of mitochondrial DNA deletions in substantia nigra neurons in aging and Parkinson disease. *Nat Genet,* 2006, vol. 38, 515-517
- **HE, L. et al.** Detection and quantification of mitochondrial DNA deletions in individual cells by real-time PCR. *Nucleic Acids Res,* 2002, vol. 30, e68
- **KRISHNAN, K. J. ; BENDER, A. ; TAYLOR, R. W. ; TURNBULL, D. M.** A multiplex real-time PCR method to detect and quantify mitochondrial DNA deletions in individual cells. *Anal Biochem,* 2007, vol. 370, 127-129
- **SCHNEIDER ; DUBUE ; HESCHELER.** How "Pharmacoresistant" is Cav2.3, the Major Component of Voltage-Gated R-type Ca2+ Channels?. *Pharmaceuticals (Basel),* 2013, vol. 6 (6), 759-76
- **LISS, B. et al.** K-ATP channels promote the differential degeneration of dopaminergic midbrain neurons. *Nat Neurosci,* 2005, vol. 8, 1742-1751